(19) Europäisches Patentamt
European Patent Office
Office européen des brevets

(11) EP 4 759 811 A1

(12) EUROPEAN PATENT APPLICATION

(43) Date of publication:
**17.06.2026 Bulletin 2026/25**

(21) Application number: **25223031.3**

(22) Date of filing: **12.12.2025**

(51) International Patent Classification (IPC):
***C07D 471/06** (2006.01)* ***C07D 495/16** (2006.01)*
***C07D 517/16** (2006.01)* ***H10K 50/00** (2023.01)*

(52) Cooperative Patent Classification (CPC):
**C07D 471/06; C07D 495/16; C07D 517/16; H10K 50/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **13.12.2024 KR 20240186475**

(71) Applicant: **Samsung Electronics Co., Ltd.**
**Suwon-si, Gyeonggi-do 16677 (KR)**

(72) Inventors:
- **Shibuya, Hiromasa**
  **16678 Suwon-si, Gyeonggi-do (KR)**
- **Yi, Jeoung In**
  **16678 Suwon-si, Gyeonggi-do (KR)**
- **Yun, Sungyoung**
  **16678 Suwon-si, Gyeonggi-do (KR)**
- **Son, Youngmok**
  **16678 Suwon-si, Gyeonggi-do (KR)**
- **Park, Sang Ho**
  **16678 Suwon-si, Gyeonggi-do (KR)**
- **Jung, Jiyoung**
  **16678 Suwon-si, Gyeonggi-do (KR)**
- **Park, Kyung Bae**
  **16678 Suwon-si, Gyeonggi-do (KR)**
- **Kim, Hyeong-Ju**
  **16678 Suwon-si, Gyeonggi-do (KR)**

(74) Representative: **Grünecker Patent- und Rechtsanwälte**
**PartG mbB**
**Leopoldstraße 4**
**80802 München (DE)**

(54) **COMPOUND AND NONLINEAR OPTICALLY ACTIVE ORGANIC MATERIAL, OPTICAL MODULATOR AND OPTICAL DEVICE INCLUDING SAME**

(57) Disclosed is a compound represented by Chemical Formula 1.

[Chemical Formula 1]

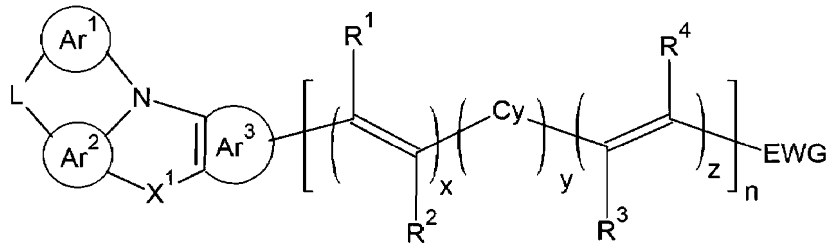

In Chemical Formula 1, the definition of each substituent is as described in the detailed description.

EP 4 759 811 A1

FIG. 1

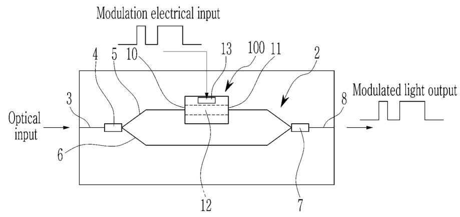
Modulation electrical input
Optical input
Modulated light output
3
4
5
10
13
100
11
2
8
6
12
7

## Description

## BACKGROUND

### 1. Field

The present inventive concepts relate to compounds and nonlinear optically active organic materials, optical modulators, and optical devices including the same.

### 2. Description of the Related Art

Recently, the rapid exchange of information through the internet on computers has led to the globalization of information and communication between countries around the world. Ultra-high-speed information communication and networks including same may utilize optical communication that uses light traveling through optical fibers at a much faster rate than electrons, while vibrating at a relatively fast rate, for example about $10^{14}$ Hz. Optical communication, which involves encoding a large amount of information into light and transmitting the information quickly, has been utilized for modern information communication.

An optical information processing system may include a light emitting device that generates light, a photodetector that detects light, and an optical signal processing device that processes optical signals. The optical signal processing device, for example, may include an optical switch and an optical modulator.

## SUMMARY

Example embodiments provide a compound that is thermally stable, has a high electro-optic coefficient ($r_{33}$), and may be manufactured into a film through a simple process, making it suitable for use in optical communication devices for example including optical modulators.

The invention is what is claimed in the independent claims. Preferred embodiments are specified in the dependent claims.

Example embodiments provide a nonlinear optically active organic material including the compound.

Example embodiments provide an optical modulator including the compound.

Example embodiments provide an optical device including the compound.

Example embodiments provide an electronic device including the compound or the optical modulator.

An optical modulator that includes the compound, and any device including same (e.g., an optical device), may enable an optical transmission configured to transmit information to support high-speed video communication applications such as video conferencing and high-definition television, for example based on supporting a modulation speed of over about 10 Gbps, for example based on utilizing a combination of a continuous-wave laser and an external light modulator. Such an optical modulator and any device including same (e.g., an optical device), may provide higher-speed communication than optical transmission systems configured to transmit information based on direct modulation of a semiconductor laser, which may enable a modulation speed of up to about 2.5 Gbps. The optical modulator that includes the compound, and any device including same (e.g., an optical device), may exhibit superior processability and reduced manufacturing cost to meet demand for such high-speed communication applications, thereby overcoming limitations on meeting demand for such information devices that might otherwise exist due to manufacturing costs of external light modulators configured to support such high-speed communication applications, for example due to difficulties in the manufacturing process of external light modulators such as external light modulators that may use inorganic crystals such as $LiNbO_3$.

According to some example embodiments, a compound may be represented by Chemical Formula 1.

[Chemical Formula 1]

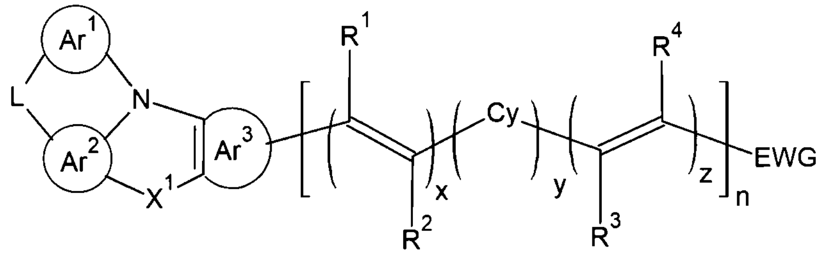

In Chemical Formula 1,

$Ar^1$, $Ar^2$, and $Ar^3$ may each independently be a substituted or unsubstituted C6 to C30 arene group, a substituted or unsubstituted C3 to C30 heteroarene group, or any combination thereof,

$X^1$ may be a single bond, -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-(CR^fR^9)_{n1}-$, $-CR^{ff}R^{gg}-$, $-C(R^m)=C(R^n)-$, or $-C(R^p)=N-$ (wherein $R^{a1}$, $R^{a2}$, $R^b$, $R^c$, $R^d$, $R^e$, $R^f$, $R^g$, $R^m$, $R^n$, and $R^p$ may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and $R^{bb}$, $R^{cc}$, $R^{dd}$, $R^{ee}$, $R^{ff}$, and $R^{gg}$ are each independently $(CH_2)_x$ or a heteroatom of O, N, S, Se, or Te, wherein x in $(CH_2)_x$ is a positive integer, and wherein a pair of $R^{bb}$ and $R^{cc}$, a pair of $R^{dd}$ and $R^{ee}$, or a pair of $R^{ff}$ and $R^{gg}$ are linked to each other to form a first ring structure, and in $-(CR^fR^g)_{n1}-$, n1 is 1 or 2),

L may be a single bond, -O-, -S-, -Se-, -Te-, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-(CR^fR^9)_{n1}-$, $-CR^{ff}R^{gg}-$, $-C(R^m)=C(R^n)-$, $-C(R^{mm})=C(R^{nn})-$, or $-C(R^p)=N-$ (wherein $R^{a1}$, $R^{a2}$, $R^b$, $R^c$, $R^d$, $R^e$, $R^f$, $R^g$, $R^m$, $R^n$, and $R^p$ may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and $R^{bb}$, $R^{cc}$, $R^{dd}$, $R^{ee}$, $R^{ff}$, $R^{gg}$, $R^{mm}$, and $R^{nn}$ are each independently $(CH_2)_y$ or a heteroatom of O, N, S, Se, or Te, wherein y in $(CH_2)_y$ is a positive integer, and wherein a pair of $R^{bb}$ and $R^{cc}$, a pair of $R^{dd}$ and $R^{ee}$, a pair of $R^{ff}$ and $R^{gg}$, or a pair of $R^{mm}$ and $R^{nn}$ are linked to each other to form a second ring structure, and in $-(CR^fR^9)_{n1}-$, n1 is 1 or 2),

when L is $-NR^{a1}-$, $-BR^{a2}-$, $-SiR^bR^c-$, $-GeR^dR^e-$, $-(CR^fR^9)_{n1}-$, $-C(R^m)=C(R^n)-$, or $-C(R^p)=N-$, L is optionally linked to $Ar^1$ or $Ar^2$ to form a third ring structure,

Cy may be a substituted or unsubstituted C6 to C10 arylene group, a substituted or unsubstituted C3 to C10 heteroarylene group, a substituted or unsubstituted C3 to C10 cycloalkylene group, a substituted or unsubstituted C3 to C10 cycloalkenylene group, a substituted or unsubstituted C3 to C10 heterocycloalkylene group, a substituted or unsubstituted C3 to C10 heterocycloalkenylene group, or any combination thereof,

EWG may be $CR^aR^b$ (wherein $R^a$ and $R^b$ are each independently a cyano group or a cyano-containing group); a substituted or unsubstituted C6 to C30 hydrocarbon ring group having at least one functional group selected from C=O, C=S, C=Se, C=Te, and $CR^aR^b$ (wherein $R^a$ and $R^b$ are each independently a cyano group or a cyano-containing group); a substituted or unsubstituted C2 to C30 heterocyclic group having at least one functional group selected from C=O, C=S, C=Se, C=Te, and $CR^aR^b$ (wherein $R^a$ and $R^b$ are each independently a cyano group or a cyano-containing group), or any combination thereof,

$R^1$ to $R^4$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted C2 to C30 acyl group, a halogen, a cyano group (-CN), a cyano-containing group, a nitro group, a pentafluorosulfanyl group ($-SF_5$), a hydroxyl group, an amine group, a hydrazine group, a hydrazone group, a carboxyl group or a salt thereof, a sulfonic acid group or a salt thereof, a phosphoric acid group or a salt thereof, $-SiR^aR^bR^c$ (wherein $R^a$, $R^b$, and $R^c$ are each independently hydrogen or a substituted or unsubstituted C1 to C10 alkyl group), or any combination thereof,

x may be an integer from 0 to 5,

y may be an integer from 1 to 5,

z may be an integer from 0 to 5, and

n may be an integer from 1 to 5.

The compound represented by Chemical Formula 1 may be a compound represented by Chemical Formula 2.

[Chemical Formula 2]

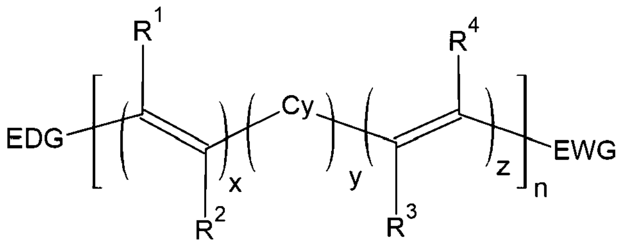

In Chemical Formula 2, EDG may be an electron donor moiety represented by Chemical Formula 2A:

[Chemical Formula 2A]

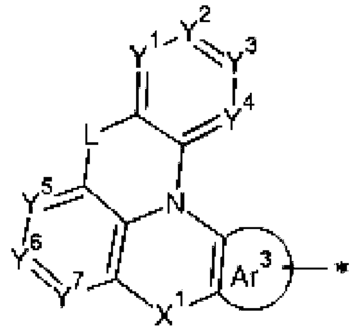

in Chemical Formula 2 and Chemical Formula 2A,

$X^1$, L, Cy, $Ar^3$, $R^1$ to $R^4$, EWG, x, y, z, and n are the same as in Chemical Formula 1,
$Y^1$ to $Y^7$ may each independently be N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$s may be linked to each other to form a substituted or unsubstituted C6 to C30 arene group, a substituted or unsubstituted C3 to C30 heteroarene group, or a condensed ring thereof, and
* indicates a linking point with Chemical Formula 2.

In Chemical Formula 2A, $Y^4$ may be N or $CR^k$ (wherein $R^k$ is a halogen, a cyano group, a C1 to C10 haloalkyl group, a C1 to C10 cyanoalkyl group, or an amine group), or
$Y^7$ may be N or $CR^k$ (wherein $R^k$ may be a halogen, a cyano group, a C1 to C10 haloalkyl group, a C1 to C10 cyanoalkyl group, or an amine group), and $X^1$ may be -O-, -S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, $-C(R^m)=C(R^n)-$, or -C(RP)=N- (wherein $R^{a1}$, $R^{a2}$, $R^b$, $R^c$, $R^d$, $R^e$, $R^f$, $R^g$, $R^m$, $R^n$, and $R^p$ may each independently be a halogen, a C1 to C20 haloalkyl group, or a C1 to C20 cyanoalkyl group, and wherein n1 in $-(CR^fR^g)_{n1}-$ is 1 or 2).

In Chemical Formula 2A, when adjacent two of $Y^1$ to $Y^7$ are $CR^k$, the adjacent two $CR^k$s may be linked to each other to form a fused ring.

In this case, EDG represented by Chemical Formula 2A may be represented by Chemical Formula 2AA.

[Chemical Formula 2AA]

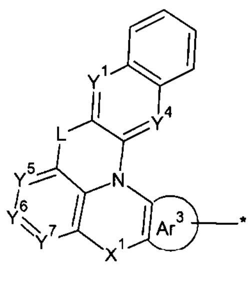
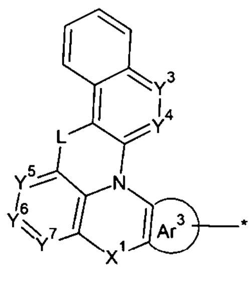
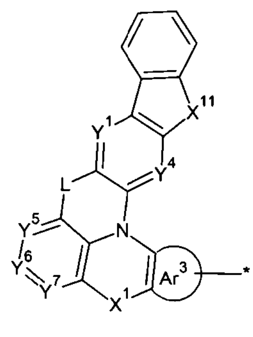
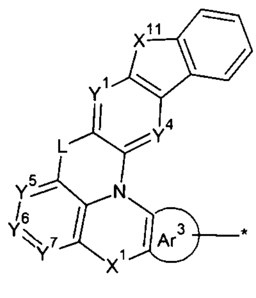

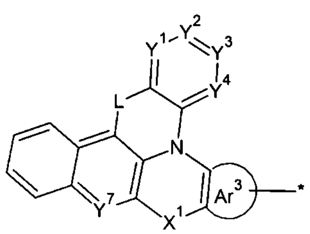
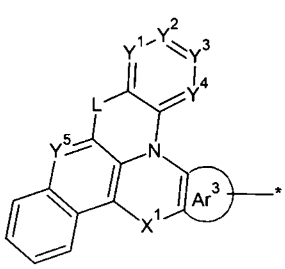

In Chemical Formula 2AA,

$X^1$, L, $Y^1$ to $Y^7$, and $Ar^3$ are the same as in Chemical Formula 2A,
$X^{11}$ is the same as $X^1$ in Chemical Formula 1, and
* indicates a linking point with an adjacent $(C(R^1)=C(R^2))_x$ or $(Cy)_y$ group of Chemical Formula 2.

In Chemical Formula 1, the first ring structure and the second ring structure may each independently be a spiro structure or a fused ring structure.
The spiro structure may include one of a plurality of moieties represented by Group 1.

[Group 1]

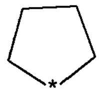
(1)

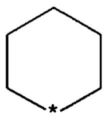
(2)

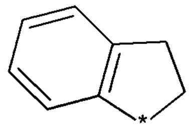
(3)

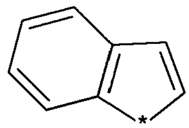
(4)

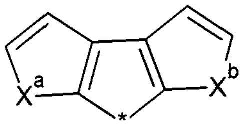
(5)

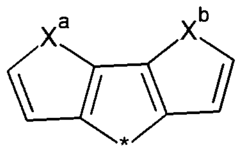
(6)

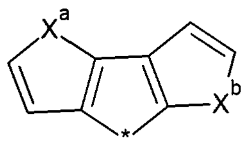
(7)

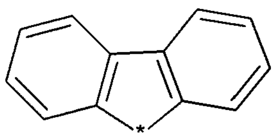
(8)

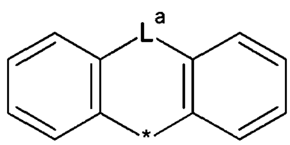
(9)

In Group 1,

$X^a$ and $X^b$ may each independently be -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, or $-GeR^{dd}R^{ee}-$ (wherein $R^{a1}$, $R^{a2}$, $R^b$, $R^c$, $R^d$, and $R^e$ are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and $R^{bb}$, $R^{cc}$, $R^{dd}$, and $R^{ee}$ are each independently $(CH_2)_z$, or a heteroatom of O, N, S, Se, or Te, wherein z in $(CH_2)_z$ is a positive integer, and wherein a pair of $R^{bb}$ and $R^{cc}$ or a pair of $R^{dd}$ and $R^{ee}$ are linked to each other to form a third ring structure),
$L^a$ may be -O-, -S-, -Se-, -Te-, $-NR^{a1}-$, $-BR^{a2}-$, $-SiR^bR^c-$, $-GeR^dR^e-$, $-(CR^fR^g)_{n1}-$, $-C(R^p)=N-$, or a single bond (wherein $R^{a1}$, $R^{a2}$, $R^b$, $R^c$, $R^d$, $R^e$, $R^f$, $R^g$, and $R^p$ may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and wherein n1 in $-(CR^fR^g)_{n1}-$ is 1 or 2), and
one or more hydrogens of each ring may be optionally replaced with at least one substituent selected from deuterium, halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In Group 1, CH present in the aromatic rings of moieties (3), (4), (5), (6) and (7) may be replaced with N.
In Chemical Formula 1, $Ar^3$ may be any one of a plurality of moieties represented by Group 2.

[Group 2]

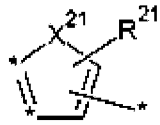
(1)
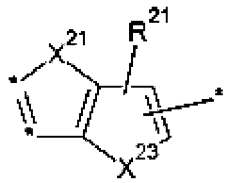
(2)
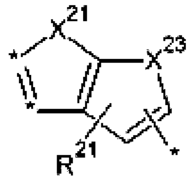
(3)

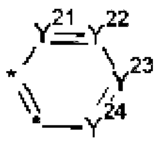
(4)
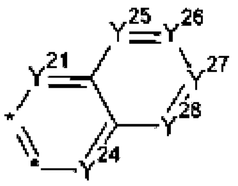
(5)
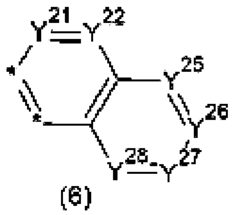
(6)

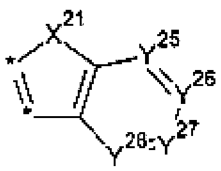
(7)
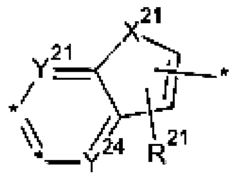
(8)
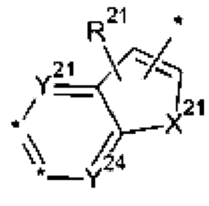
(9)

In Group 2,

$X^{21}$ and $X^{23}$ may each independently be a single bond, -O-, -S-, -Se-, -Te-, - S(=O)-, $-S(=O)_2-$, $-NR^{a1}-$, $-BR^{a2}-$, $-SiR^bR^c-$, $-SiR^{bb}R^{cc}-$, $-GeR^dR^e-$ , $-GeR^{dd}R^{ee}-$, $-CR^fR^g-$, or $-CR^{ff}R^{gg}-$ (wherein $R^{a1}$, $R^{a2}$, $R^b$, $R^c$, $R^d$, $R^e$, $R^f$, and $R^g$ may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and $R^{bb}$, $R^{cc}$, $R^{dd}$, $R^{ee}$, $R^{ff}$, and $R^{gg}$ are each independently $(CH_2)_w$ or a heteroatom of O, N, S, Se, or Te, wherein w in $(CH_2)_w$ is a positive integer, and wherein a pair of $R^{bb}$ and $R^{cc}$, a pair of $R^{dd}$ and $R^{ee}$, or a pair of $R^{ff}$ and $R^{gg}$ are linked to each other to form a third ring structure),

$R^{21}$ may be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted C2 to C30 acyl group, a halogen, a cyano group (-CN), a cyano-containing group, a nitro group, a pentafluorosulfanyl group ($-SF_5$), a hydroxyl group, an amine group, a hydrazine group, a hydrazone group, a carboxyl group or a salt thereof, a sulfonic acid group or a salt thereof, a phosphoric acid group or a salt thereof, $-SiR^aR^bR^c$ (wherein $R^a$, $R^b$, and $R^c$ are each independently hydrogen or a substituted or unsubstituted C1 to C10 alkyl group), or any combination thereof,

$Y^{21}$ to $Y^{28}$ may each independently be N or $CR^k$, wherein $R^k$ may be hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$s may be linked to each other to form a substituted or unsubstituted C6 to C30 arene group, a substituted or unsubstituted C3 to C30 heteroarene group, or a condensed ring thereof, provided that any one of $Y^{21}$ to $Y^{24}$ in moiety (4), any one of $Y^{25}$ to $Y^{28}$ in moiety (5), any one of $Y^{25}$ to $Y^{28}$ in moiety (6), or any one of $Y^{25}$ to $Y^{28}$ in moiety (7) is a single bond linking group, more precisely, a linking point with an adjacent $(C(R^1)=C(R^2))_x$ or $(Cy)_y$ group of Chemical Formula 1,

*=* is a moiety fused to an $X^1$-containing ring of Chemical Formula 1, and

-* is a linking point with an adjacent $(C(R^1)=C(R^2))_x$ or $(Cy)_y$ group of Chemical Formula 1.

In Chemical Formula 1, Cy may each independently be any one of a plurality of moieties represented by Group 3. When there are two or more Cy's, each Cy may be the same or different and may be independently selected from Group 3.

[Group 3]

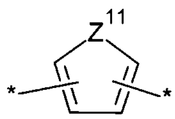

(1)

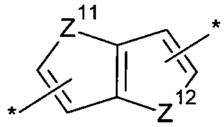

(2)

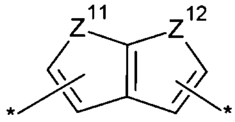

(3)

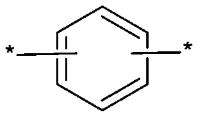

(4)

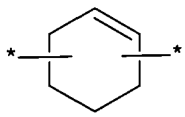

(5)

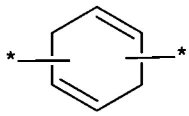

(6)

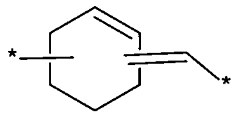

(7)

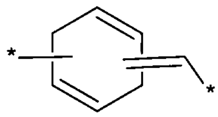

(8)

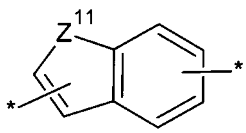

(9)

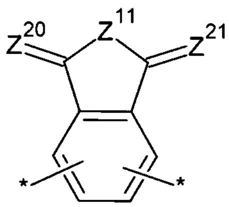

(10)

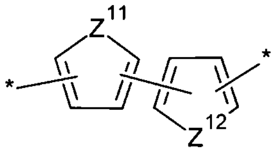

(11)

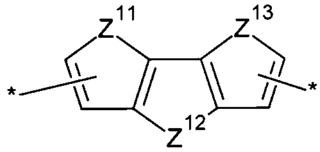

(12)

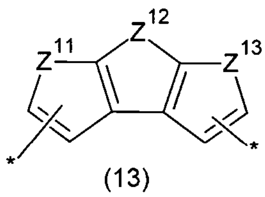

(13)

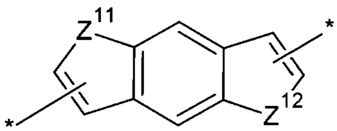

(14)

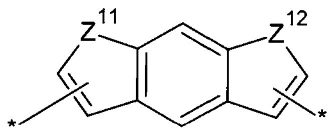

(15)

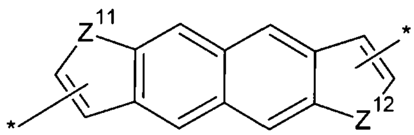

(16)

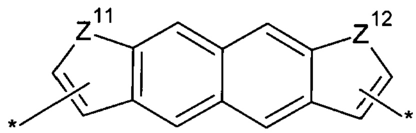

(17)

In Group 3,

$Z^{11}$, $Z^{12}$, and $Z^{13}$ may each independently be O, S, Se, Te, S(=O), $S(=O)_2$, $NR^a$, or $SiR^bR^c$ (wherein $R^a$, $R^b$, and $R^c$ may each independently be hydrogen, a C1 to C10 alkyl group, a C1 to C10 haloalkyl group, $-SiH_3$, a C1 to C10 alkylsilyl group, $-NH_2$, a C1 to C10 alkylamine group, a C6 to C12 aryl group, a C3 to C12 heteroaryl group, a halogen, a cyano group, or any combination thereof),

$Z^{20}$ and $Z^{21}$ may each independently be O, S, Se, or Te,

$R^b$ and $R^c$ may each independently be present or may be linked to form a third ring structure, and

* is a linking point with Chemical Formula 1.

In Chemical Formula 1, EWG may be a ring group represented by Chemical Formula 3.

[Chemical Formula 3]

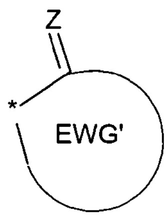

In Chemical Formula 3,

EWG' may be a substituted or unsubstituted C6 to C30 aryl group, a substituted or unsubstituted C3 to C30 heteroaryl group, or a substituted or unsubstituted C3 to C30 heterocycloalkenyl group,
Z may be O, S, Se, Te, or $CR^aR^b$, wherein $R^a$ and $R^b$ are each independently a cyano group or a cyano-containing group, and
* is a linking point with Chemical Formula 1.

In Chemical Formula 1, EWG may be a ring group represented by any one of Chemical Formula 3A to Chemical Formula 3G.

[Chemical Formula 3A]

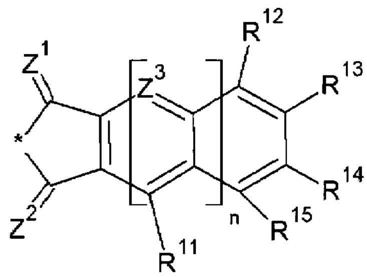

In Chemical Formula 3A,

$Z^1$ may be O, S, Se, Te, or $CR^aR^b$, wherein $R^a$ and $R^b$ may each independently be a cyano group or a cyano-containing group,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group,
$Z^3$ may be N and $CR^c$ (wherein $R^c$ is hydrogen, deuterium, or a substituted or unsubstituted C1 to C10 alkyl group),
$R^{11}$, $R^{12}$, $R^{13}$, $R^{14}$, and $R^{15}$ may be the same or different and may each independently be hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof, wherein $R^{12}$ and $R^{13}$ and $R^{14}$ and $R^{15}$ may each independently be present or be linked to each other to form a fused aromatic ring,
n may be 0 or 1, and
* indicates a linking point with Chemical Formula 1,

[Chemical Formula 3B]

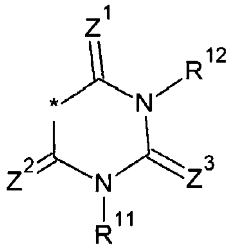

wherein, in Chemical Formula 3B,
$Z^1$ may be O, S, Se, Te, or $CR^aR^b$, wherein $R^a$ and $R^b$ may each independently be a cyano group or a cyano-containing group,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group,
$Z^3$ may be O, S, Se, Te, or $C(R^a)(CN)$ (wherein $R^a$ may be hydrogen, a cyano group (-CN), or a C1 to C10 alkyl group),
$R^{11}$ and $R^{12}$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* indicates a linking point with Chemical Formula 1,

[Chemical Formula 3C]

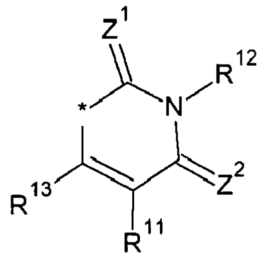

wherein, in Chemical Formula 3C,
$Z^1$ may be O, S, Se, Te, or $CR^aR^b$, wherein $R^a$ and $R^b$ may each independently be a cyano group or a cyano-containing group,
$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group,
$R^{11}$, $R^{12}$ and $R^{13}$ may be the same or different and may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* indicates a linking point with Chemical Formula 1,

[Chemical Formula 3D]

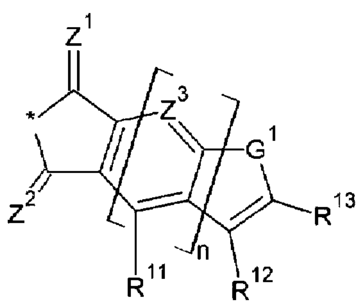

wherein, in Chemical Formula 3D,

$Z^1$ may be O, S, Se, Te, or $CR^aR^b$, wherein $R^a$ and $R^b$ may each independently be a cyano group or a cyano-containing group,

$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group,

$Z^3$ may be N or $CR^c$ (wherein $R^c$ may be hydrogen or a substituted or unsubstituted C1 to C10 alkyl group),

$G^1$ may be O, S, Se, Te, $SiR^xR^y$, or $GeR^zR^w$, wherein $R^x$, $R^y$, $R^z$, and $R^w$ may be the same or different and may each independently be hydrogen, deuterium, a halogen, a substituted or unsubstituted C1 to C20 alkyl group, or a substituted or unsubstituted C6 to C20 aryl group,

$R^{11}$, $R^{12}$, and $R^{13}$ may be the same or different and may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof, wherein $R^{12}$ and $R^{13}$ may each independently be present or be linked to each other to form a fused aromatic ring,

n may 0 or 1, and

* indicates a linking point with Chemical Formula 1,

[Chemical Formula 3E]

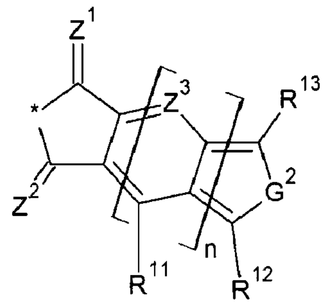

wherein, in Chemical Formula 3E,

$Z^1$ may be O, S, Se, Te, or $CR^aR^b$, wherein $R^a$ and $R^b$ may each independently be a cyano group or a cyano-containing group,

$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group,

$Z^3$ may be N or $CR^c$ (wherein $R^c$ may be hydrogen or a substituted or unsubstituted C1 to C10 alkyl group),

$G^2$ may be O, S, Se, Te, $SiR^xR^y$, or $GeR^zR^w$, wherein $R^x$, $R^y$, $R^z$, and $R^w$ may be the same or different and may each independently be hydrogen, deuterium, a halogen, a substituted or unsubstituted C1 to C20 alkyl group, or a substituted or unsubstituted C6 to C20 aryl group,

$R^{11}$, $R^{12}$, and $R^{13}$ may be the same or different and may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof,

n may be 0 or 1, and

* indicates a linking point with Chemical Formula 1,

[Chemical Formula 3F]

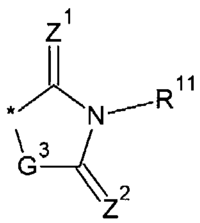

wherein, in Chemical Formula 3F,

$Z^1$ may be O, S, Se, Te, or $CR^aR^b$, wherein $R^a$ and $R^b$ may each independently be a cyano group or a cyano-containing group,

$Z^2$ may be O, S, Se, Te, or $CR^aR^b$, wherein $R^a$ and $R^b$ may each independently be hydrogen, a substituted or unsubstituted C1 to C10 alkyl group, a cyano group, or a cyano-containing group,

$R^{11}$ may be hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof, and

$G^3$ may be O, S, Se, Te, $SiR^xR^y$, or $GeR^zR^w$, wherein $R^x$, $R^y$, $R^z$, and $R^w$ may be the same or different and are each independently hydrogen, deuterium, a halogen, a substituted or unsubstituted C1 to C20 alkyl group, or a substituted or unsubstituted C6 to C20 aryl group, and

* indicates a linking point with Chemical Formula 1,

[Chemical Formula 3G]

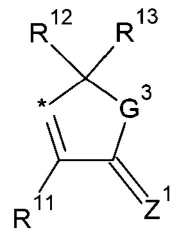

wherein, in Chemical Formula 3G,

$Z^1$ may be O, S, Se, Te, or $CR^aR^b$, wherein $R^a$ and $R^b$ are each independently a cyano group or a cyano-containing group,

$R^{11}$ to $R^{13}$ may be hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof, and

$G^3$ may be O, S, Se, Te, $SiR^xR^y$, or $GeR^zR^w$, wherein $R^x$, $R^y$, $R^z$, and $R^w$ may be the same or different and may each independently be hydrogen, deuterium, a halogen, a substituted or unsubstituted C1 to C20 alkyl group, or a substituted or unsubstituted C6 to C20 aryl group, and

* indicates a linking point with Chemical Formula 1.

The compound may have a hyperpolarizability of less than or equal to about - 170.

The compound may have a dipole moment of greater than or equal to about 15 Debye.

The compound may have a glass transition temperature of less than or equal to about 150 °C.

According to some example embodiments, a nonlinear optically active organic material may include the compound represented by Chemical Formula 1.

According to some example embodiments, an optical modulator may include the compound represented by Chemical Formula 1 and/or a nonlinear optically active organic material that includes the compound represented by Chemical Formula 1.

According to some example embodiments, an optical modulator may include a first electrode and a second electrode facing each other; and an active layer between the first electrode and the second electrode, wherein the active layer includes the compound represented by Chemical Formula 1.

The optical modulator may further include a charge blocking layer between the first electrode and the active layer.

The active layer may further include a matrix polymer, a photoconductive polymer, a photosensitizer, or any combination thereof.

According to some example embodiments, an optical device may include an input section, a branch section, a waveguide, a combining section and an output section. The waveguide may include an optical modulator. The optical modulator may include a first electrode and a second electrode facing each other, and an active layer between the first electrode and the second electrode, wherein the active layer includes the compound represented by Chemical Formula 1.

The compound is thermally stable and has a high electro-optic coefficient ($r_{33}$), and may be manufactured into a film through a simple process, and thus the compound may be suitably used in optical communication devices (also referred to herein interchangeably as "optical devices") for example including optical modulators and may enable optical devices for example including such optical modulators that enable high-speed communication via optical transmission with reduced manufacturing costs based on including the compound.

## BRIEF DESCRIPTION OF THE DRAWINGS

FIG. 1 is a schematic view showing a use state of an optical modulator in an optical communication device such as a Mach-Zehnder interferometer according to some example embodiments.

FIG. 2 is a cross-sectional view of an optical modulator according to some example embodiments.

FIG. 3 is a schematic view of an electronic device according to some example embodiments.

## DETAILED DESCRIPTION

Hereinafter, some example embodiments are described in detail so that those of ordinary skill in the art can easily implement them. However, a structure that is actually applied may be implemented in various different forms, and is not limited to the example embodiments described herein.

In the drawings, the thickness of layers, films, panels, regions, etc., are exaggerated for clarity. Like reference numerals designate like elements throughout the specification. It will be understood that when an element such as a layer, film, region, or substrate is referred to as being "on" another element, it can be directly on the other element or intervening elements may also be present. In contrast, when an element is referred to as being "directly on" another element, there are no intervening elements present.

In the drawings, parts having no relationship with the description are omitted for clarity of the embodiments, and the same or similar constituent elements are indicated by the same reference numeral throughout the specification.

As used herein, "at least one of A, B, or C," "one of A, B, C, or any combination thereof" and "one of A, B, C, and any combination thereof" refer to each constituent element, and any combination thereof (e.g., A; B; C; A and B; A and C; B and C; or A, B and C).

As used herein, when specific definition is not otherwise provided, "substituted" refers to replacement of a hydrogen of a compound or a functional group by a halogen atom (F, Br, Cl, or I), a hydroxy group, a nitro group, a cyano group, an azido group, an amidino group, an amine group (-NR'R", wherein R' and R" are the same or different, and a hydrogen atom, a C1 to C20 alkyl group, a silyl group, C1 to C20 alkylsilyl group, C6 to C30 aryl group, (C1 to C20 alkyl)(di-C6 to C10 aryl group)silyloxy(C1 to C20) alkyl group, or C7 to C30 alkylarylsilyl group), a hydrazino group, a hydrazono group, an acylgroup, a carbamyl group, a thiol group, an ester group, a carboxyl group or a salt thereof, a sulfonic acid group or a salt thereof, a phosphoric acid group or a salt thereof, a C1 to C20 alkyl group, a C1 to C20 alkoxy group, a C2 to C20 alkenyl group, a C2 to C20 alkynyl group, a C6 to C30 aryl group, a C7 to C30 arylalkyl group, a C2 to C20 heteroaryl group, a C3 to C20 heteroarylalkyl group, a C3 to C30 (e.g., C3 to C20 or C3 to C10) cycloalkyl group, a C3 to C15 cycloalkenyl group, a C6 to C15 cycloalkynyl group, a C2 to C20 heterocycloalkyl group, or any combination thereof.

The "arene group" refers to a hydrocarbon ring group having an aromatic ring, and includes monocyclic and multicyclic hydrocarbon ring groups, and additional rings of the multicyclic hydrocarbon ring group may be an aromatic ring or a non-aromatic ring. The arene group may be a C6 to C30 arene group, a C6 to C20 arene group, or a C6 to C10 arene group.

"Heteroarene group" refers to an arene group including 1 to 3 heteroatoms selected from N, O, S, P, and Si in the ring. The heteroarene group may be a C3 to C30 heteroarene group, a C3 to C20 heteroarene group, or a C3 to C10 heteroarene group.

As used herein, "hydrocarbon ring group" may be a C3 to C30 hydrocarbon ring group. The hydrocarbon ring group may be an aromatic hydrocarbon ring group (e.g., C6 to C30 arene group, C6 to C20 arene group, or C6 to C10 arene group or C6 to C30 aryl group, C6 to C20 aryl group, or C6 to C10 aryl group), an alicyclic hydrocarbon ring group (e.g., C3 to C30 cycloalkyl group, C5 to C30 cycloalkyl group, C3 to C20 cycloalkyl group or C3 to C10 cycloalkyl group), or a fused ring group thereof. For example, the fused ring group may refer to a fused ring of an aromatic ring (arene ring) and a non-aromatic ring (alicyclic ring), for example a fused ring in which at least one aromatic ring (arene ring) such as a C6 to C30 arene group, a C6 to C20 arene group, or a C6 to C10 arene group or a C6 to C30 aryl group, a C6 to C20 aryl group, or a C6 to C10 aryl group and at least one non-aromatic ring (alicyclic ring) such as a C3 to C30 cycloalkyl group, a C3 to C20 cycloalkyl group, or a C3 to C10 cycloalkyl group are fused to each other.

As used herein, the "heterocyclic group" may be a C2 to C30 heterocyclic group. The heterocyclic group may be a cyclic group in which at least one, for example 1 to 3 carbons of an aromatic hydrocarbon ring group (e.g., a C6 to C30 arene group, a C6 to C20 arene group, or a C6 to C10 arene group or a C6 to C30 aryl group, a C6 to C20 aryl group, or a C6 to C10 aryl group), an alicyclic hydrocarbon ring group (e.g., a C3 to C30 cycloalkyl group, a C3 to C20 cycloalkyl group, or a C3 to C10 cycloalkyl group), and a fused ring group thereof are replaced by a heteroatom selected from N, O, S, P, and Si. In addition or alternatively, at least one carbon atom of the heterocyclic group may be replaced by a carbonyl group (C=O) or a thiocarbonyl group (C=S).

As used herein, when specific definition is not otherwise provided, "'hetero" refers to one including 1 to 3

heteroatoms selected from N, O, S, P, and S.

As used herein, "alkyl group" refers to a monovalent linear or branched saturated hydrocarbon group, for example a methyl group, an ethyl group, a propyl group, an isopropyl group, an n-butyl group, an isobutyl group, a t-butyl group, a pentyl group, a hexyl group, and the like.

As used herein, "cycloalkyl group" refers to a monovalent saturated hydrocarbon ring group in which the atoms of the cycle are carbon, for example a cyclopropyl group, a cyclobutyl group, a cyclopentyl group, or a cyclohexyl group.

As used herein, "heterocycloalkyl group" refers to a group wherein one or more carbon atoms in the cycloalkyl group are each independently replaced by a heteroatom selected from N, O, S, P, and Si, a carbonyl group (C=O), and/or a thiocarbonyl group (C=S). As used herein, "heterocycloalkylene group" refers to a divalent group having the same structure as the heterocycloalkyl group.

As used herein, "aryl group" refers to a substituent all ring-forming atoms of which have p-orbitals which form conjugation, and may be a monocyclic, polycyclic or fused ring polycyclic (e.g., rings sharing adjacent pairs of carbon atoms) functional group.

As used herein, when a definition is not otherwise provided, "cyano-containing group" refers to a monovalent group such as a C1 to C30 (e.g., C1 to C20 or C1 to C10) alkyl group, a C2 to C30 (e.g., C2 to C20 or C2 to C10) alkenyl group, or a C2 to C30 (e.g., C2 to C20 or C2 to C10) alkynyl group where at least one hydrogen is substituted with a cyano group. The cyano-containing group also refers to a divalent group such as $=CR^{x'}-(CR^{x}R^{y})_p-CR^{y'}(CN)_2$ wherein $R^x$, $R^y$, $R^{x'}$, and $R^{y'}$ are each independently hydrogen or a C1 to C10 alkyl group and p is an integer of 0 to 10 (or 1 to 10). Specific examples of the cyano-containing group may be a dicyanomethyl group, a dicyanovinyl group, a cyanoethynyl group, and the like. As used herein, the cyano-containing group does not include a functional group including a cyano group (-CN) alone.

As used herein, when a definition is not otherwise provided, "aromatic ring" refers to a C6 to C10 cyclic group (e.g., a C6 to C10 aryl group) providing a conjugated structure or a C2 to C10 heterocyclic group (e.g., a C2 to C10 heteroaryl group) providing a conjugated structure.

As used herein, when a definition is not otherwise provided, "spiro structure" may be a substituted or unsubstituted C3 to C30 (e.g., C5 to C30) hydrocarbon ring group, a substituted or unsubstituted C2 to C30 heterocyclic group, or a fused ring thereof, which shares one atom with the ring including $X^1$, $X^2$, or L in Chemical Formula 1. The substituted or unsubstituted C3 to C30 (e.g., C5 to C30) hydrocarbon ring group may be for example a substituted or unsubstituted C3 to C30 (e.g., C5 to C30) cycloalkyl group (e.g., a substituted or unsubstituted C3 to C20 cycloalkyl group, a substituted or unsubstituted C3 to C10 cycloalkyl group, a substituted or unsubstituted C5 to C20 cycloalkyl group, or a substituted or unsubstituted C5 to C10 cycloalkyl group); a substituted or unsubstituted C6 to C30 aryl group (e.g., a substituted or unsubstituted C6 to C20 aryl group, or a substituted or unsubstituted C6 to C10 aryl group); or a fused ring of a substituted or unsubstituted C3 to C30 (e.g., C5 to C30) cycloalkyl group (e.g., a substituted or unsubstituted C3 to C20 cycloalkyl group, a substituted or unsubstituted C3 to C10 cycloalkyl group, a substituted or unsubstituted C5 to C20 cycloalkyl group, or a substituted or unsubstituted C5 to C10 cycloalkyl group) and a substituted or unsubstituted C6 to C30 aryl group (e.g., a substituted or unsubstituted C6 to C20 aryl group, or a substituted or unsubstituted C6 to C10 aryl group), and the substituted or unsubstituted C2 to C30 heterocyclic group may be for example a substituted or unsubstituted C2 to C20 heterocycloalkyl group (e.g., a substituted or unsubstituted C2 to C10 heterocycloalkyl group) or a substituted or unsubstituted C2 to C20 heteroaryl group (e.g., a substituted or unsubstituted C2 to C10 heteroaryl group).

As used herein, when a definition is not otherwise provided, "fused ring" is a fused ring of two or more substituted or unsubstituted C5 to C30 hydrocarbon ring groups (e.g., a fluorenyl group), a fused ring of two or more substituted or unsubstituted C2 to C30 heterocyclic groups, or a fused ring of a substituted or unsubstituted C5 to C30 hydrocarbon ring group and a substituted or unsubstituted C2 to C30 heterocyclic group (e.g., a carbazolyl group). Herein, the hydrocarbon ring group and the heterocyclic group are as defined above.

As used herein, when a definition is not otherwise provided, "halogen" may be any one of F, Cl, Br, and I, and the haloalkyl group is a group in which at least one hydrogen of the alkyl group is replaced by a halogen, and may be, for example, a perfluoroalkyl group such as $-CF_3$.

As used herein, when a definition is not otherwise provided, an amine group may be a substituent represented by -NR'R" (wherein R' and R" are the same or different, and a hydrogen atom, a C1 to C20 alkyl group, a silyl group, a C1 to C20 alkylsilyl group, a C6 to C30 aryl group, or a C7 to C30 alkylarylsilyl group).

As used herein, when a definition is not otherwise provided, "combination" may include a mixture, an alloy, or a stacked structure of two or more.

As used herein, when a definition is not otherwise provided, "combination" in definition of a functional group refers to substitution in which one substituent is substituted with another substituent, fusion with each other, or a linkage to each other by a single bond or a C1 to C10 alkylene group.

It will be understood that elements and/or properties thereof may be recited herein as being "identical", "the same", or "equal" as other elements and/or properties thereof, and it will be further understood that elements and/or properties thereof recited herein as being "identical" to, "the same" as, or "equal" to other elements and/or properties

thereof may be "identical" to, "the same" as, or "equal" to or "substantially identical" to, "substantially the same" as or "substantially equal" to the other elements and/or properties thereof. Elements and/or properties thereof that are "substantially identical" to, "substantially the same" as or "substantially equal" to other elements and/or properties thereof will be understood to include elements and/or properties thereof that are identical to, the same as, or equal to the other elements and/or properties thereof within manufacturing tolerances and/or material tolerances. Elements and/or properties thereof that are identical or substantially identical to, equal to or substantially equal to, and/or the same or substantially the same as other elements and/or properties thereof may be structurally the same or substantially the same, functionally the same or substantially the same, and/or compositionally the same or substantially the same. While the term "same," "equal" or "identical" may be used in description of some example embodiments, it should be understood that some imprecisions may exist. Thus, when one element or property is referred to as being identical to, equal to, or the same as another element or property, it should be understood that the element or property is the same as another element or property within a desired manufacturing or operational tolerance range (e.g., ±10%).

It will be understood that elements and/or properties thereof described herein as being "substantially" the same, equal, and/or identical encompasses elements and/or properties thereof that have a relative difference in magnitude that is equal to or less than 10%. Further, regardless of whether elements and/or properties thereof are modified as "substantially," it will be understood that these elements and/or properties thereof should be construed as including a manufacturing or operational tolerance (e.g., ±10%) around the stated elements and/or properties thereof.

When the terms "about" or "substantially" are used in this specification in connection with a numerical value, it is intended that the associated numerical value includes a manufacturing or operational tolerance (e.g., ±10%) around the stated numerical value. Moreover, when the words "about" and "substantially" are used in connection with geometric shapes, it is intended that precision of the geometric shape is not required but that latitude for the shape is within the scope of the disclosure. Further, regardless of whether numerical values or shapes are modified as "about" or "substantially," it will be understood that these values and shapes should be construed as including a manufacturing or operational tolerance (e.g., ±10%) around the stated numerical values or shapes. When ranges are specified, the range includes all values therebetween such as increments of 0.1%.

As described herein, when an operation is described to be performed, or an effect such as a structure is described to be established "by" or "through" performing additional operations, it will be understood that the operation may be performed and/or the effect/structure may be established "based on" the additional operations, which may include performing said additional operations alone or in combination with other further additional operations.

Hereinafter, a compound according to some example embodiments is described. The compound is represented by Chemical Formula 1.

[Chemical Formula 1]

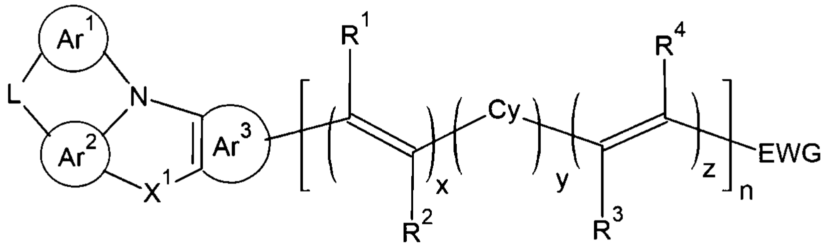

In Chemical Formula 1,

$Ar^1$, $Ar^2$, and $Ar^3$ may each independently be a substituted or unsubstituted C6 to C30 arene group, a substituted or unsubstituted C3 to C30 heteroarene group, or any combination thereof (for example, a condensed ring),

$X^1$ may be a single bond, -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-(CR^fR^g)_{n1}-$, $-CR^{ff}R^{gg}-$, $-C(R^{mm})=C(R^{nn})-$, or $-C(R^p)=N-$ (wherein $R^{a1}$, $R^{a2}$, $R^b$, $R^c$, $R^d$, $R^e$, $R^f$, $R^g$, $R^m$, $R^n$, and $R^p$ are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and $R^{bb}$, $R^{cc}$, $R^{dd}$, $R^{ee}$, $R^{ff}$, and $R^{gg}$ may each independently be $(CH_2)_x$, or a heteroatom of O, N, S, Se, or Te, wherein x in $(CH_2)_x$ is a positive integer (e.g., any positive integer, for example, a positive integer of 1 to 5, such as 2 to 3), and wherein a pair of $R^{bb}$ and $R^{cc}$, a pair of $R^{dd}$ and $R^{ee}$, or a pair of $R^{ff}$ and $R^{gg}$ may be linked to each other to form a ring structure, where such a ring structure is interchangeably referred to herein as a "first ring structure", and in $-(CR^fR^g)_{n1}-$, n1 is 1 or 2),

L may be a single bond, -O-, -S-, -Se-, -Te-, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-(CR^fR^g)_{n1}-$,

$-CR^{ff}R^{gg}-$, $-C(R^m)=C(R^n)-$, $-C(R^{mm})=C(R^{nn})-$, or$-C(R^p)=N-$ (wherein $R^{a1}$, $R^{a2}$, $R^b$, $R^c$, $R^d$, $R^e$, $R^f$, $R^g$, $R^m$, $R^n$, and $R^p$ are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and $R^{bb}$, $R^{cc}$, $R^{dd}$, $R^{ee}$, $R^{ff}$, $R^{gg}$, $R^{mm}$, and $R^{nn}$ may each independently be $(CH_2)_y$ or a heteroatom of O, N, S, Se, or Te, wherein y in $(CH_2)_y$ is a positive integer (e.g., any positive integer, for example, a positive integer of 1 to 5, such as 2 to 3), and wherein a pair of $R^{bb}$ and $R^{cc}$, a pair of $R^{dd}$ and $R^{ee}$, a pair of $R^{ff}$ and $R^{gg}$, or a pair of $R^{mm}$ and $R^{nn}$ may be linked to each other to form a ring structure, where such a ring structure is interchangeably referred to herein as a "second ring structure", and in $-(CR^fR^9)_{n1}-$, n1 is 1 or 2),

when L is $-NR^{a1}-$, $-BR^{a2}-$, $-SiR^bR^c-$, $-GeR^dR^e-$, $-(CR^fR^9)_{n1}-$, $-(C(R^m)=C(R^n))-$, or $-(C(R^p)=N))-$, L may be optionally linked to $Ar^1$ or $Ar^2$ to form a ring structure, where such a ring structure is interchangeably referred to herein as a "third ring structure",

Cy may be a substituted or unsubstituted C6 to C10 arylene group, a substituted or unsubstituted C3 to C10 heteroarylene group, a substituted or unsubstituted C3 to C10 cycloalkylene group, a substituted or unsubstituted C3 to C10 cycloalkenylene group, a substituted or unsubstituted C3 to C10 heterocycloalkylene group, a substituted or unsubstituted C3 to C10 heterocycloalkenylene group, or any combination thereof,

EWG may be $CR^aR^b$ (wherein $R^a$ and $R^b$ are each independently a cyano group or a cyano-containing group); a substituted or unsubstituted C6 to C30 hydrocarbon ring group having at least one functional group selected from C=O, C=S, C=Se, C=Te, and $CR^aR^b$ (wherein $R^a$ and $R^b$ may each independently be a cyano group or a cyano-containing group); a substituted or unsubstituted C2 to C30 heterocyclic group having at least one functional group selected from C=O, C=S, C=Se, C=Te, and $CR^aR^b$ (wherein $R^a$ and $R^b$ may each independently be a cyano group or a cyano-containing group); or any combination thereof,

$R^1$ to $R^4$ may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted C2 to C30 acyl group, a halogen, a cyano group (-CN), a cyano-containing group, a nitro group, a pentafluorosulfanyl group ($-SF_5$), a hydroxyl group, an amine group, a hydrazine group, a hydrazone group, a carboxyl group or a salt thereof, a sulfonic acid group or a salt thereof, a phosphoric acid group or a salt thereof, $-SiR^aR^bR^c$ (wherein $R^a$, $R^b$, and $R^c$ may each independently be hydrogen or a substituted or unsubstituted C1 to C10 alkyl group), or any combination thereof,

x may be an integer from 0 to 5,

y may be an integer from 1 to 5,

z may be an integer from 0 to 5, and

n may be an integer from 1 to 5.

In Chemical Formula 1, Cy may be a substituted C3 to C10 cycloalkenylene group or a substituted C3 to C10 heterocycloalkenylene group, and Cy may have an exocyclic alkylidene (for example, methylidene) as a substituent. In this case, in addition to C3 to C10 cycloalkenylene group or the C3 to C10 heterocycloalkenylene group in Cy, the exocyclic alkylidene moiety may be further linked to the $C(R^3)=C(R^4)$ group, whereby $(C(R^1)=C(R^2))_x$, $(Cy)_y$, and $(C(R^3)=C(R^4))_z$ together form a conjugated polyene structure, as exemplified by the compounds represented by Chemical Formulas 1-5, 1-6, or 1-11 in the Examples described later.

In Chemical Formula 1, the substituted C6 to C30 hydrocarbon ring or substituted C2 to C30 heterocycle in EWG may have an exocyclic alkylidene (for example, methylidene) as a substituent. In this case, the exocyclic alkylidene group may be linked to the $C(R^3)=C(R^4)$ group or to $(Cy)_y$, as exemplified by the compound represented by Chemical Formula 1-4 in the Examples described later.

The compound represented by Chemical Formula 1 may be usefully used in an optical signal processing device as a nonlinear optically active organic material. The nonlinear optically active organic material refers to a material whose optical properties, such as phase, change when light passes through the material. For example, when the nonlinear optically active organic material is applied to a Mach-Zehnder Interferometer (MZI) optical modulator, the refractive index thereof changes when an electric field is applied, and the phase of light is modulated when light passes through it.

The compound has a D-$\pi$-A structure, and includes an electron donor moiety (D) including a first ring moiety containing nitrogen (N) and L, a second ring moiety containing $X^1$ and N, and a third ring moiety of $Ar^3$; an electron acceptor moiety (A) represented by EWG; and a Cy-containing linker ($\pi$) by which the electron donor moiety and the electron acceptor moiety are linked.

In the electron donor moiety, the first ring moiety containing nitrogen (N) and L, the second ring moiety containing nitrogen (N) and $X^1$, and the third ring moiety of $Ar^3$ form a fused ring, thereby suppressing free rotation of the compound and realizing high, or increased, hyperpolarizability ($\beta$zzz) and thus a high electro-optic coefficient ($r_{33}$), thereby improving the performance of an optical modulator that includes the compound represented by Chemical Formula 1 (e.g., in an active layer of the optical modulator, in a nonlinear optically active organic material, any combination thereof, or the like) and/or an optical device including same, thereby enabling improved speed of information communication and networks including

same based on optical transmissions. Moreover, the compound exhibits an improved stability of the molecular structure to enhance thermal stability of the compound, thereby improving the reliability of the optical modulator and/or optical device including the compound (e.g., in an active layer of the optical modulator, in a nonlinear optically active organic material, any combination thereof, or the like) during fabrication and use of the optical modulator and/or optical device. In addition, since the optical modulator(s) include the compound, device manufacturing costs may be reduced.

$Ar^1$ and $Ar^2$ in the first ring moiety may each independently be a substituted or unsubstituted C6 to C30 arene group, a substituted or unsubstituted C3 to C30 heteroarene group, or a condensed ring group thereof. The condensed ring group refers to a ring in which two or more of an arene group and a heteroarene group are fused together.

The third ring moiety $Ar^3$ is a substituted or unsubstituted C6 to C30 arene group, a substituted or unsubstituted C3 to C30 heteroarene group, or any combination thereof, and the combination thereof may be a condensed ring group in which two or more of the arene groups and heteroarene groups are fused to each other, or a ring group in which two or more of the arene groups and heteroarene groups are linked by a single bond or a methylene group.

The Cy-containing linker acts as a bridge between the electron donor portion and the electron acceptor portion, and may control a wavelength range of light absorbed by controlling the conjugation length of the compound. In example embodiments, the Cy-containing linker may be a moiety having a ring structure having a conjugated structure.

In the Cy-containing linker of Chemical Formula 1, x may be an integer from 0 to 5, for example, 1 to 4, y may be an integer from 1 to 5, for example, 1 to 4, z may be an integer from 0 to 5, for example, 1 to 4, and n may be an integer from 1 to 5, for example, 1 to 4.

In the Cy-containing linker of Chemical Formula 1, when y is 2 or more, Cy may be the same or different from each other. For example, Cy may be a moiety selected from Group 3, and the Cy-containing linker may include two or more identical or different moieties selected from Group 3.

In the Cy-containing linker of Chemical Formula 1, when n is 2 or more, Cy may be the same or different from each other. For example, Cy may be a moiety selected from Group 3, and the Cy-containing linker may include two or more identical or different moieties selected from Group 3.

The compound of Chemical Formula 1 may be a compound represented by Chemical Formula 2.

[Chemical Formula 2]

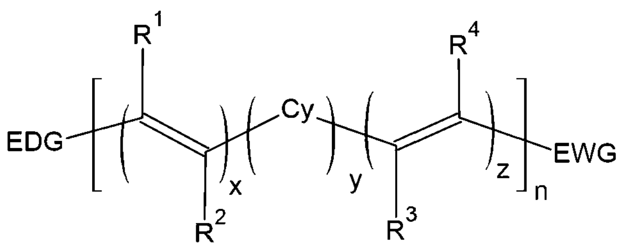

In Chemical Formula 2,

Cy, $R^1$ to $R^4$, EWG, x, y, z, and n are the same as in Chemical Formula 1, and
EDG is an electron donor moiety represented by Chemical Formula 2A:

[Chemical Formula 2A]

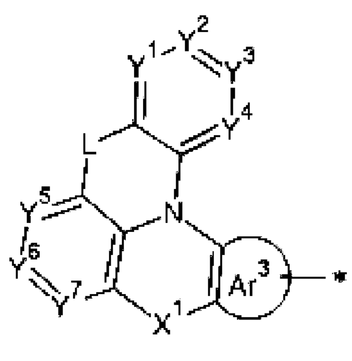

In Chemical Formula 2 and Chemical Formula 2A, $X^1$, L, Cy, $Ar^3$, $R^1$ to $R^4$, EWG, x, y, z, and n may be the same as

in Chemical Formula 1, and $Y^1$ to $Y^7$ may each independently be N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$s may be linked to each other to form a substituted or unsubstituted C6 to C30 arene group, a substituted or unsubstituted C3 to C30 heteroarene group, or a condensed ring thereof, and * in Chemical Formula 2A indicates a linking point with Chemical Formula 2 (e.g., an adjacent atom of Chemical Formula 2, an adjacent $(C(R^1)=C(R^2))_x$ or $(Cy)_y$ group of Chemical Formula 2, etc.).

In Chemical Formula 2, when EDG is an electron donor moiety represented by Chemical Formula 2A, Chemical Formula 2 may be represented by Chemical Formula 2'.

[Chemical Formula 2']

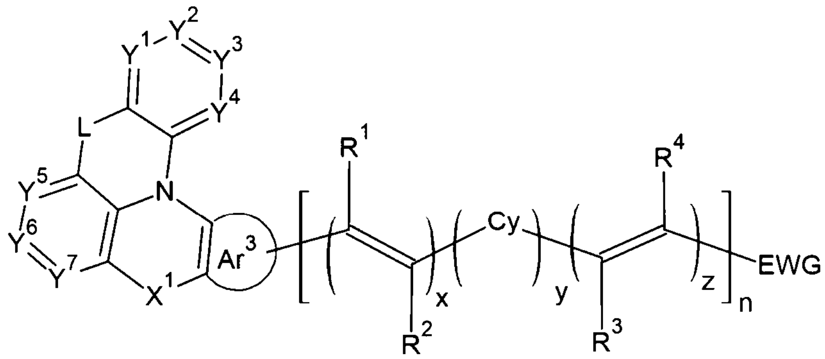

In some example embodiments, Chemical Formula 2 may be interchangeably represented, labeled, and/or referred to as "Chemical Formula 2'". As shown, Chemical Formula 2' may comprise Chemical Formula 2 and Chemical Formula 2A, such that Chemical Formula 2 and Chemical Formula 2A may collectively comprise Chemical Formula 2'. As referred to herein, a "linking point" with a chemical formula or portion thereof may be referred to interchangeably as a linking position, a bonding position, a bonding point, a linking portion, or a bonding portion linking with a chemical formula or portion thereof, any combination thereof, or the like.

In some example embodiments, $Y^1$, $Y^2$, $Y^5$, and $Y^6$ of Chemical Formula 2A and/or Chemical Formula 2' may each independently be $CR^k$, wherein $R^k$ may be an amine group (-NR'R", wherein R' and R" are the same as or different from each other and are a hydrogen atom, a C1 to C20 alkyl group or a C6 to C30 aryl group).

In some example embodiments, in Chemical Formula 2A and/or Chemical Formula 2', $Y^4$ may be N or $CR^k$ (wherein, $R^k$ may be a halogen, a cyano group, a C1 to C10 haloalkyl group, a C1 to C10 cyanoalkyl group, or an amine group). In this case, the functional groups present in $Y^4$, N present in the L or $X^1$-containing ring, and $Ar^3$ may increase intramolecular interactions, thereby inhibiting free rotation of the molecule and enhancing the hyperpolarizability.

In some example embodiments, in Chemical Formula 2A and/or Chemical Formula 2', $Y^7$ may be N or $CR^k$ (wherein $R^k$ may be a halogen, a cyano group, a C1 to C10 haloalkyl group, a C1 to C10 cyanoalkyl group, or an amine group), and $X^1$ may be -O-, -S-, -Se-, -Te-, -S(=O)-, $-S(=O)_2-$, $-NR^{a1}-$, $-BR^{a2}-$, $-SiR^bR^c-$, $-GeR^dR^e-$, - $(CR^fR^9)_{n1}-$, $-C(R^m)=C(R^n)-$, or $-C(R^p)=N-$(wherein $R^{a1}$, $R^{a2}$, $R^b$, $R^c$, $R^d$, $R^e$, $R^f$, $R^g$, $R^m$, $R^n$, and $R^p$ may each independently be a halogen, a C1 to C20 haloalkyl group, or a C1 to C20 cyanoalkyl group), and wherein n1 in $-(CR^fR^g)_{n1}-$ may be 1 or 2. In this case, $Y^7$ and $X^1$ may enhance the hyperpolarizability by increasing the intramolecular interaction and suppressing the free rotation of the molecule.

In Chemical Formula 2A and/or Chemical Formula 2', when adjacent two of $Y^1$ to $Y^7$ are $CR^k$, the adjacent two $CR^k$s may be linked to each other to form a fused ring.

In this case, the electron donor moiety EDG represented by Chemical Formula 2A in Chemical Formula 2 may be represented by Chemical Formula 2AA.

[Chemical Formula 2A]

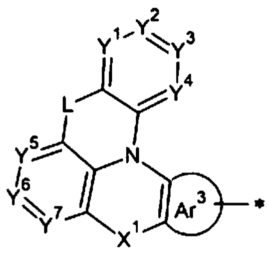

[Chemical Formula 2AA]

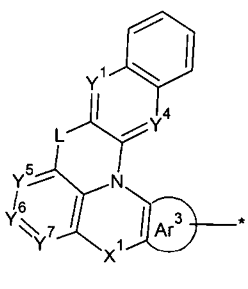

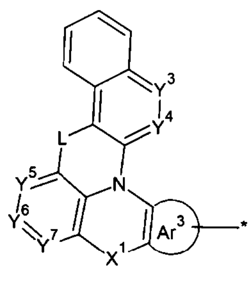

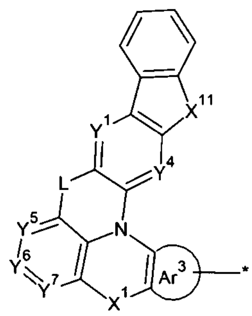

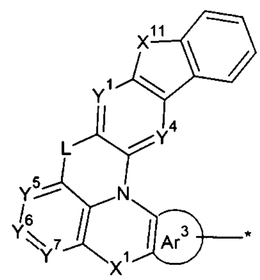

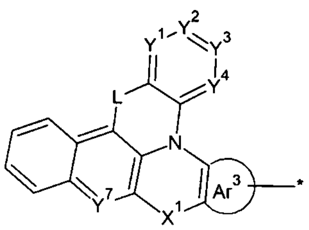

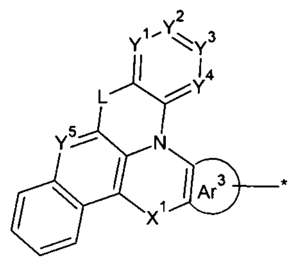

In Chemical Formula 2AA,

$X^1$, L, $Y^1$ to $Y^7$, and $Ar^3$ are the same as in Chemical Formula 2A and/or Chemical Formula 2 or 2',
$X^{11}$ is the same as $X^1$ in Chemical Formula 1, and
* indicates a linking point with Chemical Formula 2 and/or Chemical Formula 2' (e.g., an adjacent atom of Chemical Formulas 2 and/or Chemical Formula 2', an adjacent $(C(R^1)=C(R^2))_x$ or $(Cy)_y$ group of Chemical Formulas 2 and/or 2', etc.).

In Chemical Formula 2AA, one or more hydrogens of each ring may be optionally replaced with at least one substituent selected from deuterium, halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In Chemical Formula 2AA, CH of the aromatic ring may be substituted with N, and one or more (e.g., 1, 2, or 3) Ns may be included in one aromatic ring.

In $X^1$ and L of Chemical Formulas 1 and 2; $X^a$ and $X^b$ in Group 1; $X^{21}$ and $X^{23}$ of $Ar^3$ described below; $X^{11}$ of Chemical Formula 2AA; and $R^b$ and $R^c$ in Group 3, the ring structure (e.g., the "first ring structure" that may be included in $X^1$, the "second ring structure" that may be included in L, etc.) may each independently be a spiro structure or a fused ring structure. The spiro structure may be a substituted or unsubstituted C3 to C30 (e.g., C5 to C30) hydrocarbon ring group or a substituted or unsubstituted C2 to C30 heterocyclic group. The substituted or unsubstituted C3 to C30 (e.g., C5 to C30) hydrocarbon ring group may be, for example, a substituted or unsubstituted C3 to C30 (e.g., C5 to C30) cycloalkyl group (e.g., a substituted or unsubstituted C3 to C20 cycloalkyl group, a substituted or unsubstituted C3 to C10 cycloalkyl group, a substituted or unsubstituted C5 to C20 cycloalkyl group, or a substituted or unsubstituted C5 to C10 cycloalkyl group), a

substituted or unsubstituted C6 to C30 aryl group (e.g., a substituted or unsubstituted C6 to C20 aryl group or a substituted or unsubstituted C6 to C10 aryl group), or a fused ring of a substituted or unsubstituted C3 to C30 (e.g., C5 to C30) cycloalkyl group (e.g., a substituted or unsubstituted C3 to C20 cycloalkyl group, a substituted or unsubstituted C3 to C10 cycloalkyl group, a substituted or unsubstituted C5 to C20 cycloalkyl group, or a substituted or unsubstituted C5 to C10 cycloalkyl group) and a substituted or unsubstituted C6 to C30 aryl group (e.g., a substituted or unsubstituted C6 to C20 aryl group or a substituted or unsubstituted C6 to C10 aryl group). Examples of the fused ring include a fluorenyl group, an indanyl group, and the like. The substituted or unsubstituted C2 to C30 heterocyclic group may be, for example, a substituted or unsubstituted C2 to C30 heterocycloalkyl group (e.g., a substituted or unsubstituted C2 to C20 heterocycloalkyl group or a substituted or unsubstituted C2 to C10 heterocycloalkyl group).

The fused ring structure may be a fused substituted or unsubstituted C3 to C30 (e.g., C5 to C30) hydrocarbon ring group, a fused substituted or unsubstituted C2 to C30 heterocyclic group, or a fused ring thereof. The substituted or unsubstituted C3 to C30 (e.g., C5 to C30) hydrocarbon ring group may be, for example, a substituted or unsubstituted C3 to C30 (e.g., C5 to C30) cycloalkyl group (e.g., a substituted or unsubstituted C3 to C20 cycloalkyl group, a substituted or unsubstituted C3 to C10 cycloalkyl group, a substituted or unsubstituted C5 to C20 cycloalkyl group, or a substituted or unsubstituted C5 to C10 cycloalkyl group) or a substituted or unsubstituted C6 to C30 aryl group (e.g., a substituted or unsubstituted C6 to C20 aryl group or a substituted or unsubstituted C6 to C10 aryl group), the substituted or unsubstituted C2 to C30 heterocyclic group may be, for example, a substituted or unsubstituted C2 to C30 heterocycloalkyl group (e.g., a substituted or unsubstituted C2 to C20 heterocycloalkyl group or a substituted or unsubstituted C2 to C10 heterocycloalkyl group) or a substituted or unsubstituted C2 to C30 heteroaryl group (e.g., a substituted or unsubstituted C2 to C20 heteroaryl group or a substituted or unsubstituted C2 to C10 heteroaryl group).

The spiro structure may include one of a plurality of moieties represented by Group 1 (e.g., one of moieties (1) to (9) in Group 1).

[Group 1]

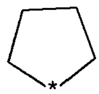
(1)

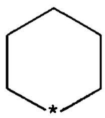
(2)

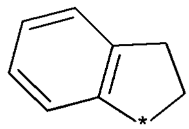
(3)

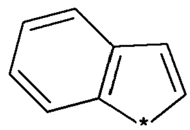
(4)

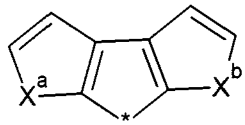
(5)

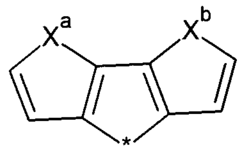
(6)

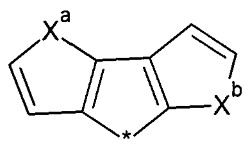
(7)

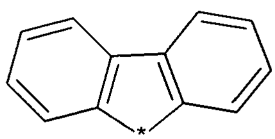
(8)

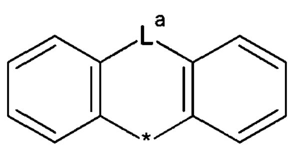
(9)

In Group 1,

$X^a$ and $X^b$ may each independently be -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, or $-GeR^{dd}R^{ee}-$ (wherein $R^{a1}$, $R^{a2}$, $R^b$, $R^c$, $R^d$, and $R^e$ are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and $R^{bb}$, $R^{cc}$, $R^{dd}$, and $R^{ee}$ may each independently be $(CH_2)_z$ or a heteroatom of O, N, S, Se, or Te, wherein z in $(CH_2)_z$ is a positive integer (e.g., a positive integer of 1 to 5, such as 2 to 3), and wherein a pair of $R^{bb}$ and $R^{cc}$ or a pair of $R^{dd}$ and

$R^{ee}$ may be linked to each other to form a fourth ring structure, also referred to herein interchangeably as a "third ring structure" or the like),

$L^a$ may be -O-, -S-, -Se-, -Te-, $-NR^{a1}-$, $-BR^{a2}-$, $-SiR^bR^c-$, $-GeR^dR^e-$, $-(CR^fR^g)_{n1}-$, $-C(R^p)=N-$, or a single bond (wherein $R^{a1}$, $R^{a2}$, $R^b$, $R^c$, $R^d$, $R^e$, $R^f$, $R^g$, and $R^p$ may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group), wherein n1 in $-(CR^fR^g)_{n1}-$ is 1 or 2, and

one or more hydrogens of each ring may be optionally replaced with at least one substituent selected from deuterium, halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group.

In Group 1, one or more CHs present in the aromatic rings of moieties (3), (4), (5), (6), and (7) may be replaced with N.

In Chemical Formula 1, $Ar^3$ may be any one of a plurality of moieties represented by Group 2 (e.g., one of moieties (1) to (9) in Group 2).

[Group 2]

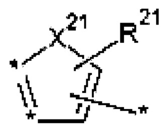

(1)

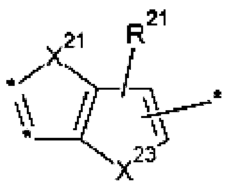

(2)

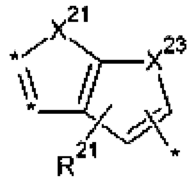

(3)

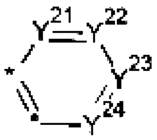

(4)

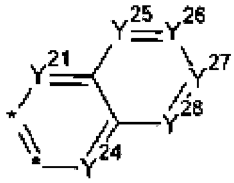

(5)

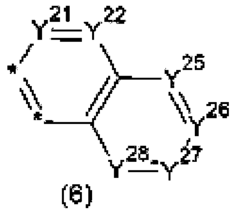

(6)

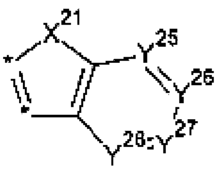

(7)

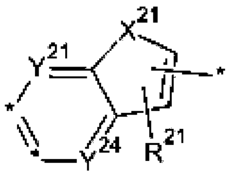

(8)

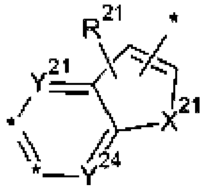

(9)

In Group 2,

$X^{21}$ and $X^{23}$ may each independently be a single bond, -O-, -S-, -Se-, -Te-, - S(=O)-, $-S(=O)_2-$, $-NR^{a1}-$ , $-BR^{a2}-$, $-SiR^bR^c-$, $-SiR^{bb}R^{cc}-$, $-GeR^dR^e-$, $-GeR^{dd}R^{ee}-$, $-CR^fR^g-$, or $-CR^{ff}R^{gg}-$ (wherein $R^{a1}$, $R^{a2}$, $R^b$, $R^c$, $R^d$, $R^e$, $R^f$, and $R^g$ may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and $R^{bb}$, $R^{cc}$, $R^{dd}$, $R^{ee}$, $R^{ff}$, and $R^{gg}$ may each independently be $(CH_2)_w$ or a heteroatom of O, N, S, Se, or Te, wherein w in $(CH_2)_w$ is a positive integer (e.g., a positive integer of 1 to 5, such as 2 to 3), and wherein a pair of $R^{bb}$ and $R^{cc}$, a pair of $R^{dd}$ and $R^{ee}$, or a pair of $R^{ff}$ and $R^{gg}$ may be linked to each other to form a fifth ring structure, also referred to herein interchangeably as a "third ring structure"),

$R^{21}$ may be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted C2 to C30 acyl group, a halogen, a cyano group (-CN), a cyano-containing group, a nitro group, a pentafluorosulfanyl group ($-SF_5$), a hydroxyl group, an amine group, a hydrazine group, a hydrazone group, a carboxyl group or a salt thereof, a sulfonic acid group or a salt thereof, a phosphoric acid group or a salt thereof, $-SiR^aR^bR^c$ (wherein $R^a$, $R^b$, and $R^c$ are each independently hydrogen or a substituted or unsubstituted C1 to C10 alkyl group), or any combination thereof,

$Y^{21}$ to $Y^{28}$ may each independently be N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$s may be linked to each other to form a substituted or unsubstituted C6 to C30 arene group, a substituted or unsubstituted C3 to C30 heteroarene group, or a condensed ring thereof, provided that, in Group 2, any one of $Y^{21}$ to $Y^{24}$ in moiety (4), any one of $Y^{25}$ to $Y^{28}$ in moiety (5), any one of $Y^{25}$ to $Y^{28}$ in moiety (6), or any one of $Y^{25}$ to $Y^{28}$ in moiety (7) may be a single bond linking group, more precisely, a linking point with an adjacent $(C(R^1)=C(R^2))_x$ or $(Cy)_y$ group of Chemical Formula 1,

*=* is a moiety fused to the $X^1$-containing ring of Chemical Formula 1, and

-* is a linking point with an adjacent $(C(R^1)=C(R^2))_x$ group of Chemical Formula 1.

In Group 2, when two or more single rings of the conjugated structure are fused as in (2), (3), and (5) to (9), the hyperpolarizability of the compound may be improved.

In Chemical Formula 1, Cy may be any one of a plurality of moieties represented by Group 3 (e.g., one of moieties (1) to (17) of Group 3).

[Group 3]

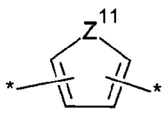
(1)

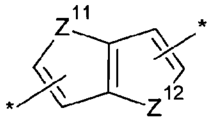
(2)

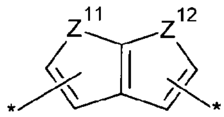
(3)

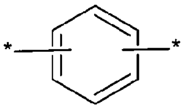
(4)

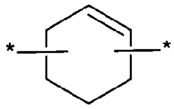
(5)

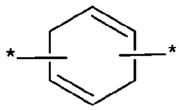
(6)

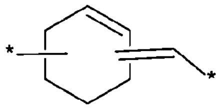
(7)

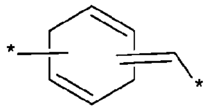
(8)

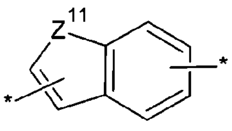
(9)

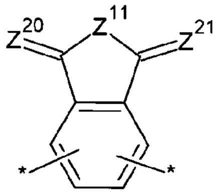
(10)

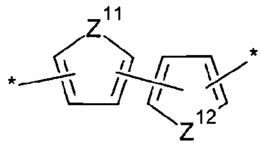
(11)

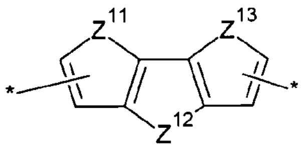
(12)

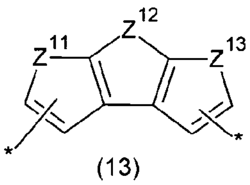
(13)

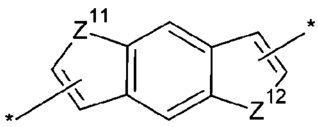
(14)

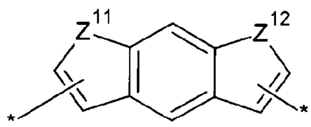
(15)

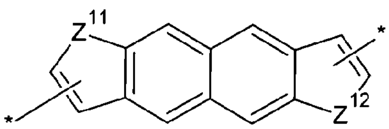
(16)

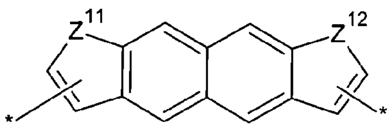
(17)

In Group 3,

$Z^{11}$, $Z^{12}$, and $Z^{13}$ may each independently be O, S, Se, Te, S(=O), $S(=O)_2$, $NR^a$, or $SiR^bR^c$ (wherein $R^a$, $R^b$, and $R^c$ may each independently be hydrogen, a C1 to C6 alkyl group, a C1 to C10 haloalkyl group, $-SiH_3$, a C1 to C10 alkylsilyl group, $-NH_2$, a C1 to C10 alkylamine group, a C6 to C12 aryl group, a C3 to C12 heteroaryl group, a halogen, a cyano group, or any combination thereof),
$Z^{20}$ and $Z^{21}$ may each independently be O, S, Se, or Te,
$R^b$ and $R^c$ may each independently be present and may be linked to form a sixth ring structure (also referred to herein interchangeably as a "third ring structure" or the like), and
* is a linking point with Chemical Formula 1.

One or more hydrogens in the ring structure of each moiety of Group 3 may be unsubstituted or substituted, and when substituted, may be replaced by, for example, a C1 to C10 alkyl group, a C1 to C10 alkoxy group, a C1 to C10 haloalkyl group, $-SiH_3$, a C1 to C10 alkylsilyl group, $-NH_2$, a C1 to C10 alkylamine group, a C6 to C12 aryl group, a C3 to C12 heteroaryl group, a halogen, or a cyano group.

In Chemical Formula 1, EWG may be a ring group represented by Chemical Formula 3.

[Chemical Formula 3]

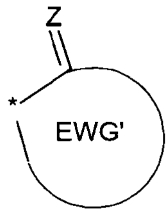

In Chemical Formula 3,

EWG' is a substituted or unsubstituted C6 to C30 (e.g., C6 to C20 or C6 to C10) aryl group, a substituted or unsubstituted C3 to C30 (e.g., C3 to C20 or C3 to C10) heteroaryl group, or a substituted or unsubstituted C3 to C30 (e.g., C3 to C20 or C3 to C10) heterocycloalkenyl group,
Z may be O, S, Se, Te, or $CR^aR^b$, wherein $R^a$ and $R^b$ are each independently a cyano group or a cyano-containing group, and
* may be a linking point with Chemical Formula 1.

In Chemical Formula 1, EWG may be a ring group represented by any one of Chemical Formula 3A to Chemical Formula 3G.

[Chemical Formula 3A]

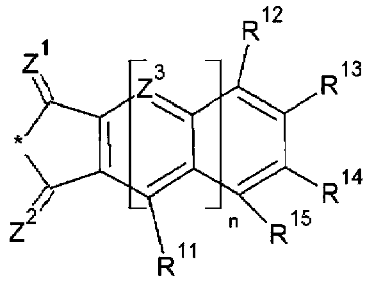

In Chemical Formula 3A,

$Z^1$ is O, S, Se, Te, or $CR^aR^b$, wherein $R^a$ and $R^b$ are each independently a cyano group or a cyano-containing group,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group,
$Z^3$ is N or $CR^c$ (wherein $R^c$ is hydrogen, deuterium, or a substituted or unsubstituted C1 to C10 alkyl group),
$R^{11}$, $R^{12}$, $R^{13}$, $R^{14}$, and $R^{15}$ may be the same or different and may each independently be hydrogen, deuterium, a substituted or unsubstituted C1 to C30 alkyl group (e.g. C1 to C30 (C1 to C20 or C1 to C10) haloalkyl group), a substituted or unsubstituted C6 to C30 aryl group, a substituted or unsubstituted C4 to C30 heteroaryl group, a halogen, a cyano group (-CN), a cyano-containing group, or any combination thereof, wherein $R^{12}$ and $R^{13}$ and $R^{14}$ and $R^{15}$ may each independently be present or be linked to each other to form a fused aromatic ring,
n is 0 or 1, and
* indicates a bonding position (e.g., a linking point with Chemical Formula 1),

[Chemical Formula 3B]

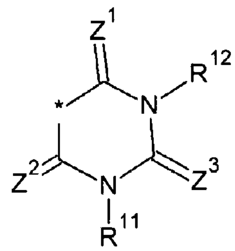

wherein, in Chemical Formula 3B,

$Z^1$ is O, S, Se, Te, or $CR^aR^b$, wherein $R^a$ and $R^b$ are each independently a cyano group or a cyano-containing group,

$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group,

$Z^3$ is O, S, Se, Te, or $C(R^a)(CN)$ (wherein $R^a$ is hydrogen, a cyano group (-CN), or a C1 to C10 alkyl group),

$R^{11}$ and $R^{12}$ are each independently hydrogen, deuterium, a substituted or unsubstituted C1 to C30 alkyl group (e.g. C1 to C30 (C1 to C20 or C1 to C10) haloalkyl group), a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and

* indicates a bonding position (e.g., a linking point with Chemical Formula 1),

[Chemical Formula 3C]

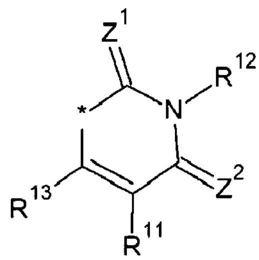

wherein, in Chemical Formula 3C,

$Z^1$ is O, S, Se, Te, or $CR^aR^b$, wherein $R^a$ and $R^b$ are each independently a cyano group or a cyano-containing group,

$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group,

$R^{11}$, $R^{12}$, and $R^{13}$ are the same or different and are each independently hydrogen, deuterium, a substituted or unsubstituted C1 to C30 alkyl group (e.g. C1 to C30 (C1 to C20 or C1 to C10) haloalkyl group), a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and

* indicates a bonding position (e.g., a linking point with Chemical Formula 1),

[Chemical Formula 3D]

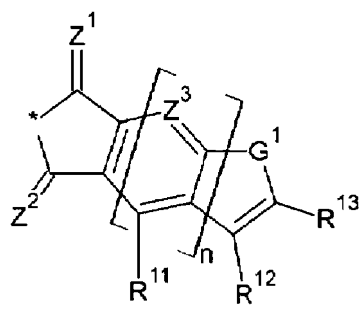

wherein, in Chemical Formula 3D,

$Z^1$ is O, S, Se, Te, or $CR^aR^b$, wherein $R^a$ and $R^b$ are each independently a cyano group or a cyano-containing group,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group,
$Z^3$ is N or $CR^c$ (wherein $R^c$ is hydrogen or a substituted or unsubstituted C1 to C10 alkyl group),
$G^1$ is O, S, Se, Te, $SiR^xR^y$, or $GeR^zR^w$, wherein $R^x$, $R^y$, $R^z$, and $R^w$ are the same or different and are each independently hydrogen, deuterium, a halogen, a substituted or unsubstituted C1 to C20 alkyl group, or a substituted or unsubstituted C6 to C20 aryl group,
$R^{11}$, $R^{12}$, and $R^{13}$ are the same or different and are each independently hydrogen, deuterium, a substituted or unsubstituted C1 to C30 alkyl group (e.g. C1 to C30 (C1 to C20 or C1 to C10) haloalkyl group), a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof, wherein $R^{12}$ and $R^{13}$ may each independently be present or be linked to each other to form a fused aromatic ring,
n is 0 or 1, and
* indicates a bonding position (e.g., a linking point with Chemical Formula 1),

[Chemical Formula 3E]

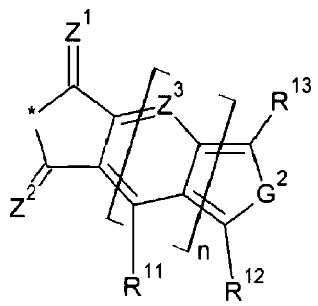

wherein, in Chemical Formula 3E,
$Z^1$ is O, S, Se, Te, or $CR^aR^b$, wherein $R^a$ and $R^b$ are each independently a cyano group or a cyano-containing group,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group,
$Z^3$ is N or $CR^c$ (wherein $R^c$ is hydrogen or a substituted or unsubstituted C1 to C10 alkyl group),
$G^2$ is O, S, Se, Te, $SiR^xR^y$, or $GeR^zR^w$, wherein $R^x$, RY, $R^z$, and $R^w$ are the same or different and are each independently hydrogen, deuterium, a halogen, a substituted or unsubstituted C1 to C20 alkyl group, or a substituted or unsubstituted C6 to C20 aryl group,
$R^{11}$, $R^{12}$, and $R^{13}$ are the same or different and are each independently hydrogen, deuterium, a substituted or unsubstituted C1 to C30 alkyl group (e.g. C1 to C30 (C1 to C20 or C1 to C10) haloalkyl group), a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof,
n is 0 or 1, and
* indicates a bonding position (e.g., a linking point with Chemical Formula 1),

[Chemical Formula 3F]

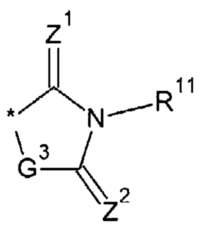

wherein, in Chemical Formula 3F,
$Z^1$ is O, S, Se, Te, or $CR^aR^b$, wherein $R^a$ and $R^b$ are each independently a cyano group or a cyano-containing group,

$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group,
$R^{11}$ is hydrogen, deuterium, a substituted or unsubstituted C1 to C30 alkyl group (e.g. C1 to C30 (C1 to C20 or C1 to C10) haloalkyl group), a substituted or unsubstituted C6 to C30 aryl group, a substituted or unsubstituted C4 to C30 heteroaryl group, a halogen, a cyano group (-CN), a cyano-containing group, or any combination thereof,
$G^3$ is O, S, Se, Te, $SiR^xR^y$, or $GeR^zR^w$, wherein $R^x$, $R^y$, $R^z$, and $R^w$ are the same or different and are each independently hydrogen, deuterium, a halogen, a substituted or unsubstituted C1 to C20 alkyl group, or a substituted or unsubstituted C6 to C20 aryl group, and
* indicates a bonding position (e.g., a linking point with Chemical Formula 1),

[Chemical Formula 3G]

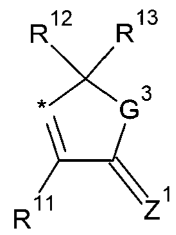

wherein, in Chemical Formula 3G,
$Z^1$ is O, S, Se, Te, or $CR^aR^b$, wherein $R^a$ and $R^b$ are each independently a cyano group or a cyano-containing group,
$R^{11}$ to $R^{13}$ are hydrogen, deuterium, a substituted or unsubstituted C1 to C30 alkyl group (e.g. C1 to C30 (C1 to C20 or C1 to C10) haloalkyl group), a substituted or unsubstituted C6 to C30 aryl group, a substituted or unsubstituted C4 to C30 heteroaryl group, a halogen, a cyano group (-CN), a cyano-containing group, or any combination thereof,
$G^3$ is O, S, Se, Te, $SiR^xR^y$, or $GeR^zR^w$, wherein $R^x$, $R^y$, $R^z$, and $R^w$ are the same or different and are each independently hydrogen, deuterium, a halogen, a substituted or unsubstituted C1 to C20 alkyl group, or a substituted or unsubstituted C6 to C20 aryl group, and
* indicates a bonding position (e.g., a linking point with Chemical Formula 1).

In Chemical Formulas 3A to 3G, the substituted or unsubstituted C1 to C30 alkyl group may be a C1 to C30 (or C1 to C20) haloalkyl group, for example a C1 to C30 (or C1 to C20) fluoroalkyl group.
Examples of the compounds represented by Chemical Formula 1 may include any one of the compounds of Group 4.

[Group 4]

[1-1] [1-2] [1-3] [1-4]

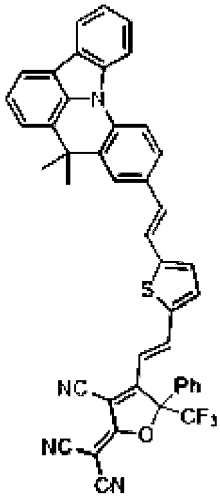

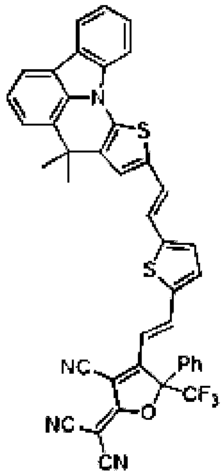

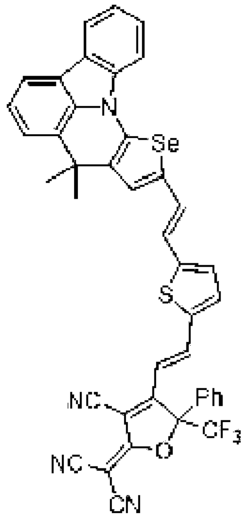

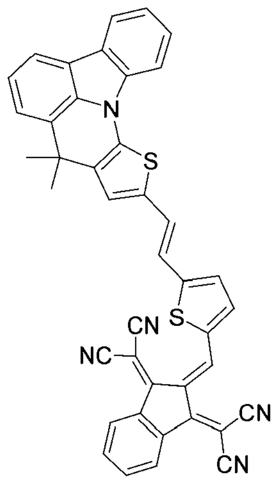

[1-5] [1-6] [1-7] [1-8]

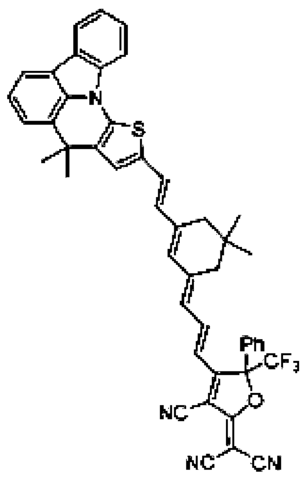
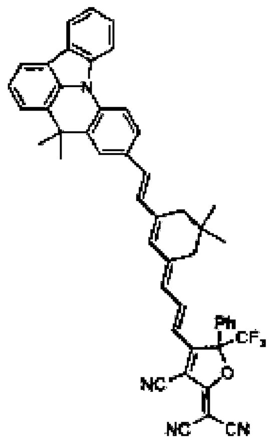
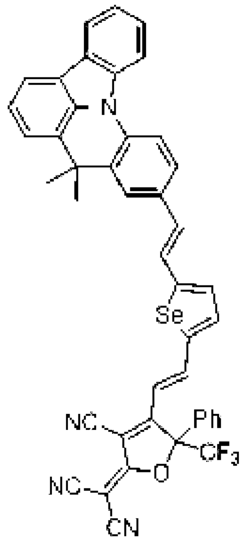
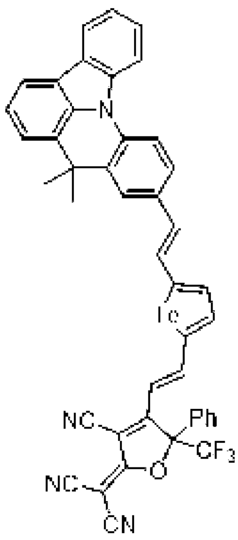

[1-9] [1-10] [1-11] [1-12]

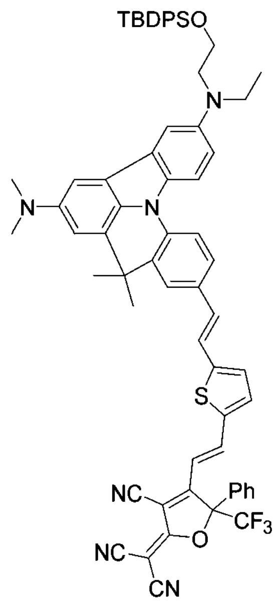
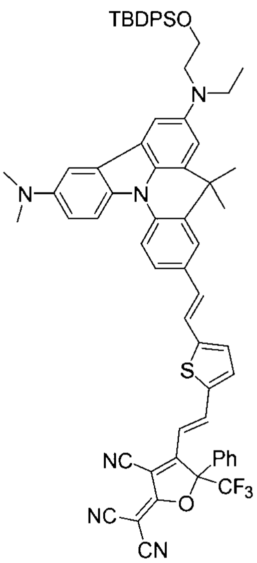
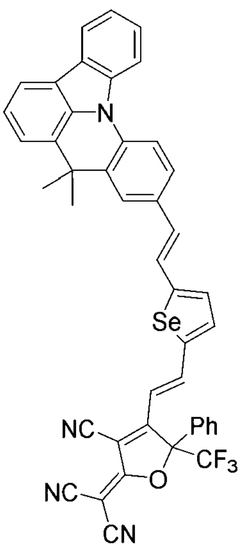
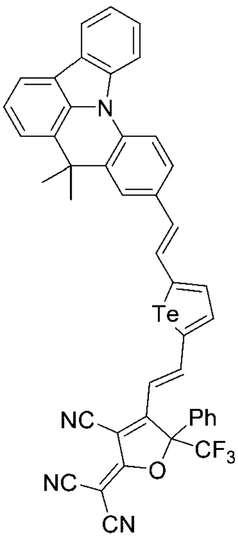

[1-13] [1-14] [1-15]

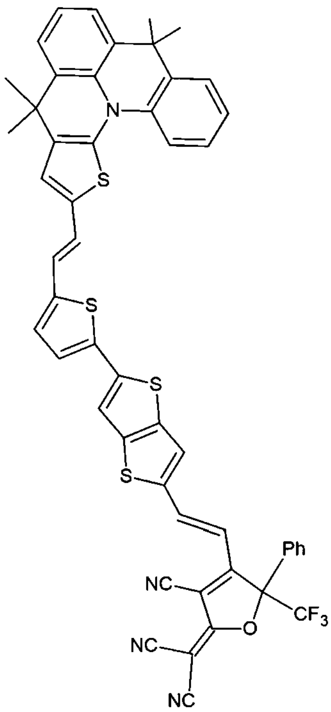

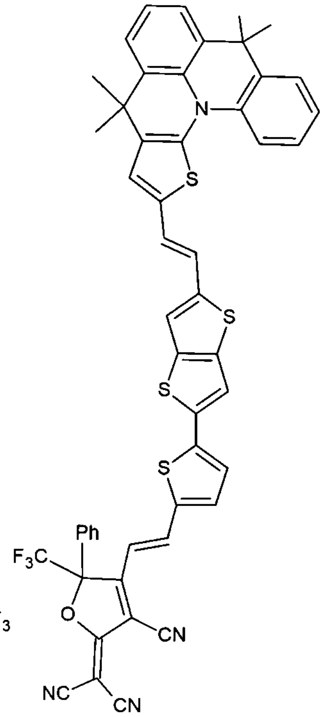

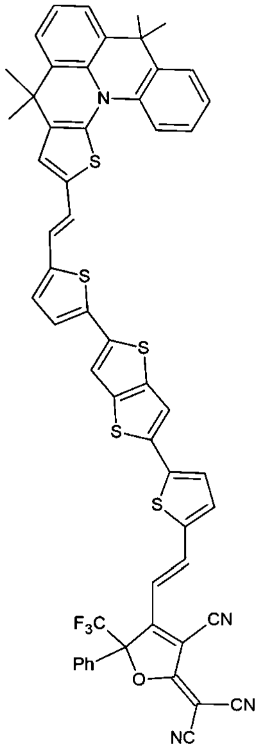

[1-16] [1-17]

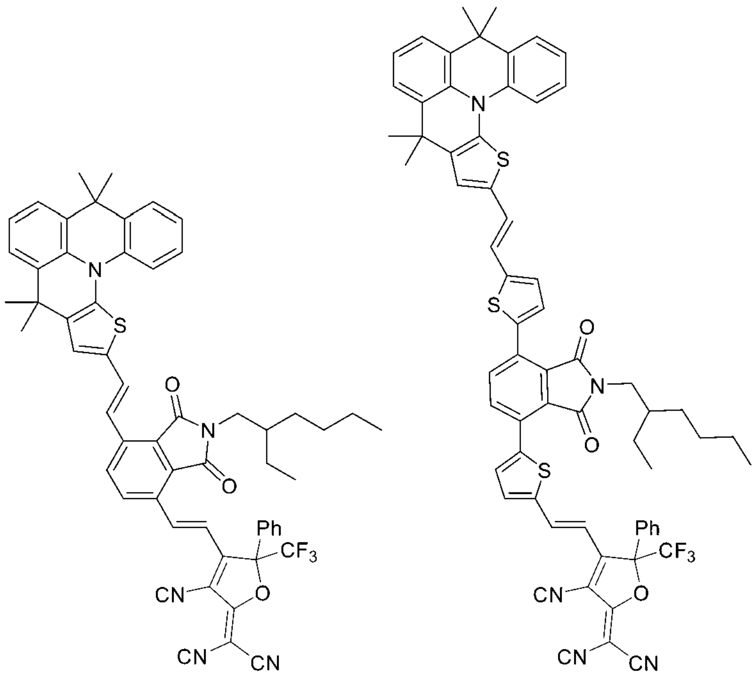

[1-18] [1-19] [1-20]

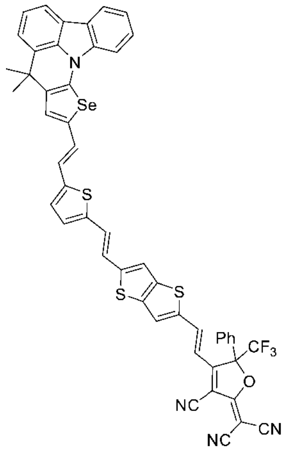

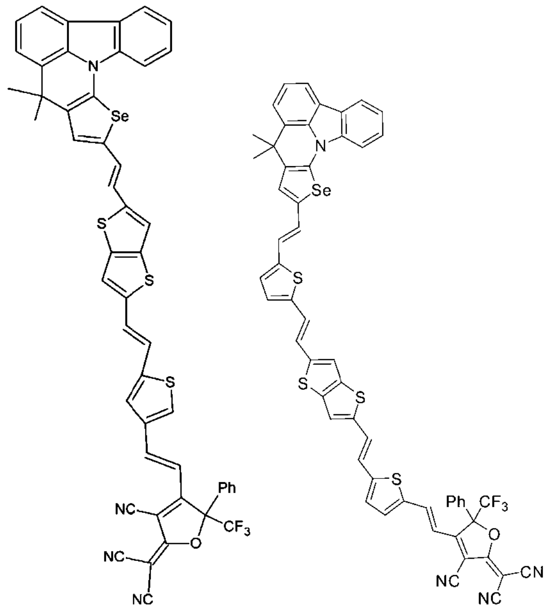

[1-21] [1-22]

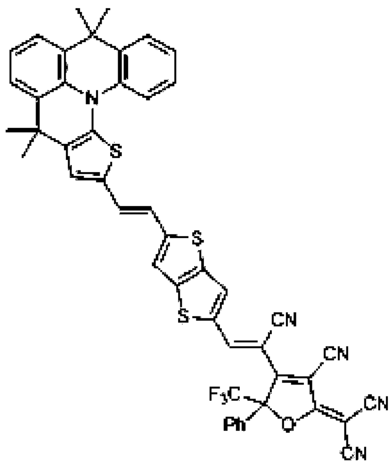

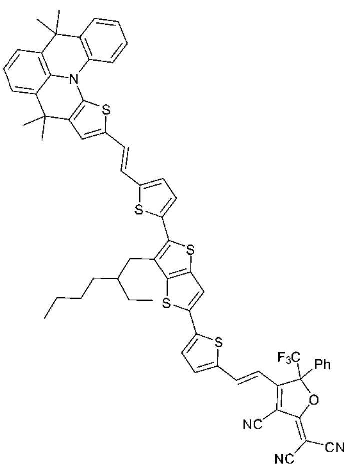

In Group 4, TBDPS is a protecting group of oxygen as a tert-butyldiphenylsilyl grpup and Ph is a pheny grpup.

The compounds represented by Chemical Formula 1 (for example, the compounds listed in Group 4) may be synthesized by known methods in the art with reference to Synthesis Examples 1 to 17 described below and are not limited to the methods described in Synthesis Examples 1 to 17.

As described above, the compound represented by Chemical Formula 1 has a structure in which the first ring moiety, the second ring moiety, and the third ring moiety are fused, thereby suppressing free rotation of the compound and realizing a high hyperpolarizability (βzzz), thereby obtaining a high, or increased, electro-optic coefficient ($r_{33}$). The electro-optic coefficient refers to the degree to which the refractive index (e.g., the refractive index of the compound or an optical modulator and/or device including same) changes by applying an electric field from the outside (e.g., externally to said compound, optical modulator, device, or the like), and a high electro-optic coefficient means that the performance of the device (e.g., optical modulator, optical device including same, etc.) can be improved based on including the compound represented by Chemical Formula 1 (e.g., in an active layer of the optical modulator, in a nonlinear optically active organic material, any combination thereof, or the like).

The relationship between the hyperpolarizability and the electro-optic coefficient is expressed by Relationship Equations 1 to 3.

[Relationship Equation 1]

$$n' = n - \frac{1}{2}n^3 r_{33} E = n + \Delta n \quad \left(\Delta n = -\frac{1}{2}n^3 r_{33} E\right)$$

In Relationship Equation 1, n' is the refractive index after applying an electric field, n is the refractive index before applying an electric field, △n is the change in the refractive index, $r_{33}$ is the electro-optic coefficient, and E is the electric field intensity.

[Relationship Equation 2]

$$r_{33}(\omega) = -\frac{2\chi_{33}^{(2)}(\omega,\omega,0)}{n_e^4}$$

In Relationship Equation 2, $r_{33}$ is the electro-optic coefficient, $\chi_{33}^{(2)}(\omega,\omega,0)$ is the second-order nonlinear susceptibility induced by the interaction of two optical waves at frequency ω with a static (zero-frequency) field, and $n_{\bar{e}}$ is the change in refractive index according to the electric field strength E.

[Relationship Equation 3]

$$\chi_{33}^{(2)}(\omega,\omega,0) = N f^{\omega} f^{\omega}\, f^{0} \beta_{zzz}(\omega,\omega,0)\langle cos^3\theta\rangle$$

In Relationship Equation 3, $\chi_{33}^{(2)}(\omega,\omega,0)$ is the second-order nonlinear susceptibility induced by the interaction of two optical waves at frequency ω with a static (zero-frequency) field, N is the molecular density of the compound, $\beta_{zzz}$ is the hyperpolarizability, and θ is the orientation angle of the molecule. In Relationship Equation 3, $cos^3\theta$ represents the degree of orientation, and this degree of orientation represents the degree of alignment of chromophores by the poling process.

The hyperpolarizability and degree of orientation play an important role in determining the electro-optic coefficients.

The compound may have a hyperpolarizability of less than or equal to about - 170, for example, less than or equal to about -200, less than or equal to about -300, less than or equal to about -400, or less than or equal to about -500. The lower limit of the hyperpolarizability is not particularly limited, but may be approximately greater than or equal to about -2500, for example, greater than or equal to about -2100, or greater than or equal to about -1500. The hyperpolarizability may be calculated by DFT(B3LYP, M062X) program.

The compound may have a dipole moment of greater than or equal to about 11 Debye, for example greater than or equal to about 14 Debye, greater than or equal to about 15 Debye, greater than or equal to about 16 Debye, or greater than or equal to about 17 Debye, and may have a dipole moment of less than or equal to about 30 Debye, less than or equal to about 29 Debye, less than or equal to about 28 Debye, less than or equal to about 27 Debye, less than or equal to about 26 Debye, or less than or equal to about 25 Debye. The dipole moment may be calculated by DFT(B3LYP, M062X) program.

The glass transition temperature of the compound may be less than or equal to about 150 °C, for example, greater than or equal to about 85 °C and less than or equal to about 150 °C or greater than or equal to about 90 °C and less than or equal to about 150 °C, greater than or equal to about 91 °C and less than or equal to about 130 °C, or greater than or equal to about 91.8 °C and less than or equal to about 110 °C. The glass transition temperature may be measured by DTA (differential thermal analysis).

The compound may be formed into films by dry processes such as the poling process, as well as inexpensive solution processes such as spin coating, inkjet printing, and spray coating.

The poling process may be a method of forming a film by heating a compound while applying an electric field.

The solution process may include a process of coating a coating composition prepared by adding the compound to a solvent, and the solvent used at this time may include ether-based solvent (e.g., tetrahydrofuran (THF), diethyl ether, 1,2-dimethoxyethane (DME), cyclopentylmethyl ether (CPME), etc.), a halogenated hydrocarbon solvent (e.g., methylene chloride, dibromomethane, chloroform, 1,2-dibromomethane, 1,2-dichloroethane, etc.), an amide-based solvent (N,N-dimethylformamide (DMF), N,N-dimethylacetamide (DMAc), N-methyl-2-pyrrolidone (NMP), etc.), a ketone-based solvent (e.g., acetone, methyl ethyl ketone, methyl isobutyl ketone, cyclopentanone, cyclohexanone, etc.), an alcohol-based solvent (e.g., methanol, ethanol, 2-propanol, n-propanol, etc.), an aliphatic hydrocarbon solvent (n-e.g., heptane, n-hexane, cyclohexane, etc.), an aromatic hydrocarbon solvent (e.g., benzene, toluene, xylene, ethylbenzene, etc.), a halogenated aromatic hydrocarbon solvent (e.g., chlorobenzene, dichlorobenzene, etc.), a glycol ether-based solvent (e.g., ethylene glycol monoethyl ether, ethylene glycol monobutyl ether, propylene glycol monomethyl ether, etc.), or a glycol-based solvent (ethylene glycol, propylene glycol, etc.). These solvents may be used alone or in combination of two or more.

In the above solution process, the compound may be included in an amount of about 1 wt% to about 20 wt%, for example, about 5 wt% to about 15 wt%, based on 100 wt% of the coating composition.

The compound has a high, or increased, electro-optic coefficient and can therefore be used as a nonlinear optical chromophore in an optical modulator to thereby enable the optical modulator to exhibit improved performance and to enable high-speed information communication via optical transmission (e.g., with reduced manufacturing costs to manufacture devices that support such communication).

Examples of the above optical modulator include an electro-optical modulator, a silicon-organic hybrid modulator, etc.

The electro-optic modulator converts an electrical signal into a light intensity signal by converting the absorbance or refractive index of light using the electro-optic effect.

The optical modulator will be described below with reference to FIGS. 1 and 2.

FIG. 1 is a schematic view showing a use state of an optical modulator in an optical communication device, such as a Mach-Zehnder interferometer according to some example embodiments, and FIG. 2 is a cross-sectional view of an optical modulator according to some example embodiments.

Referring to FIG. 1, an optical modulator 100 is provided in an optical communication device ("optical device") such as a Mach-Zehnder interferometer 2, although example embodiments are not limited thereto. The Mach-Zehnder interferometer 2 is equipped with (e.g., includes) an input section 3, a branch section 4, a first branch waveguide 5, a second branch waveguide 6, a combining section 7, and an output section 8, and an optical modulator 100 is provided in the first branch waveguide 5.

The optical modulator 100 includes an incident unit 10 (also referred to as an incident terminal, incident port, input terminal, or input port), an output unit 11 (also referred to as an output terminal or output port), an optical waveguide 12 connected from (e.g., between) the incident unit 10 to the output unit 11, and an input electrode 13 into which a modulation electrical input signal is input (e.g., from a power supply of an electronic device, for example power supply 950 of electronic device 900 as shown in FIG. 3). When light passes through the optical waveguide 12, the optical modulator 100 outputs (e.g., transmits), to the output unit 11, an optical modulation signal modulated by a modulation electrical input signal that is input (e.g., transmitted) to the input electrode 13.

Light incident on the input section 3 is branched into the first branch waveguide 5 and the second branch waveguide 6 at the branch section 4. A portion of the light branched into the first branch waveguide 5 is incident on the optical modulator 100 (e.g., at the incident unit 10 thereof) and is emitted from the optical modulator 100 as an optical modulation signal modulated by the modulation electrical input signal input to the input electrode 13. The optical modulation signal is combined with the other portion of the light propagated through the second branch waveguide 6 in the combining section 7 and is output (transmitted) from the output section 8 as a modulated light output signal with a particular (or, alternatively, predetermined) modulation intensity applied.

Referring to FIG. 2, an optical modulator 100 according to some example embodiments includes a first electrode 14, a second electrode 30 facing the first electrode 14, and an active layer 20 between (e.g., directly or indirectly between) the first electrode 14 and the second electrode 30. The first electrode 14 may be made of (e.g., may at least partially or entirely comprise) a metal such as Au, Mg, or Al; an alloy thereof; a conductive metal oxide such as zinc oxide, indium oxide, tin oxide, indium tin oxide (ITO), indium zinc oxide (IZO), or fluorine-doped tin oxide; or any combination of a metal and an oxide such as ZnO and Al or $SnO_2$ and Sb, but is not limited thereto. The second electrode 30 may be made of the same material as the first electrode 14 or a different material than the first electrode 14. In some example embodiments, the first electrode 14 may include a metal oxide such as ITO or IZO, and the second electrode 30 may include a metal such as Au, Mg, or Al. The active layer 20 includes the compound described above (e.g., a compound represented by Chemical Formula 1) as a nonlinear optically active organic material.

Although not shown in FIG. 2, a substrate may exist under the first electrode 14 (e.g., such that the first electrode 14 is between (e.g., directly or indirectly between) the active layer 20 and the substrate). The substrate may be made of (e.g., may at least partially or entirely comprise) an inorganic material such as glass; an organic material such as

polycarbonate, polymethyl methacrylate, polyethylene terephthalate, polyethylene naphthalate, polyamide, polyethersulfone, or any combination thereof; or a silicon wafer. The substrate may be omitted.

The optical modulator 100 may further include a charge blocking layer between (e.g., directly or indirectly between) the first electrode 14 and the active layer 20. The charge blocking layer may further include an inorganic oxide such as $Al_2O_3$, $ZrO_2$, $SiO_2$, $TiO_2$, $SnO_2$, $CeO_2$, MgO, NiO, CaO, GaO, ZnO, $HfO_2$, any combination thereof, or the like.

The charge blocking layer may have a thickness of about 30 nm to about 150 nm, for example, about 40 nm to about 120 nm.

The active layer 20 may further include, in addition to the compound represented by Chemical Formula 1, a matrix polymer, a photoconductive polymer, a photosensitizer, or any combination thereof.

The matrix polymer may provide good dispersion of the nonlinear optically active organic material (the compound of Chemical Formula 1), maintain orientation after poling, and offer mechanical and thermal stability. Examples of the matrix polymer may include poly(C1 to C10)alkylacrylate such as polymethylacrylate, poly(C1 to C10)alkylmethacrylate such as polymethylmethacrylate, polycarbonate (PC), polyimide (PI), polyurethane (PU), epoxy-based polymers, polysiloxane-based polymers, polystyrene (PS), polyvinyl alcohol (PVA), and polyvinyl chloride (PVC), but are not limited thereto.

The photoconductive polymer may include, for example, a carbazole unit or a triphenylamine unit, but is not limited thereto. The photoconductive polymer may include, for example, polyvinylcarbazole (PVK), polysiloxane carbazole, polyparaphenylenevinylene, polyaniline, polypyrrole, polyacetylene, polythiophene, polyalkylthiophene, carbazole-substituted polysiloxane (PSX-Cz), poly(p-phenylene terephthalate) carbazole (PPT-CZ), poly(meth)acrylate triphenylamine (TATPD), a derivative thereof, a mixture thereof, or a copolymer thereof.

The photosensitizer can be excited by a light source of a specific wavelength, for example, visible light, to generate electrons and holes. The photosensitizer may include, for example, C60 fullerene, PCBM (phenyl-C61-butyric acid methyl ester), TNF (2,4,7-trinitrofluorenone), TNFDM (2,4,7-trinitro-9-fluorenylidene-malononitrile), or any combination thereof.

A thickness of the active layer 20 may be in the range of about 100 nm to 1500 nm. The active layer 20 may be formed as a single layer or multiple layers of two or more layers.

In addition to the Mach-Zehnder interferometer device, the optical modulator may also be used in various additional optical devices. The additional optical device may be a 3D imaging system, a 3D camera, a light laser detection and ranging (LiDAR) device, a phased array antenna, or the like.

FIG. 3 is a schematic view of an electronic device 900 according to some example embodiments.

Referring to FIG. 3, an electronic device 900 may include a processor 920 (e.g., a central processing unit or CPU), a memory 930 (e.g., a solid-state drive (SSD) storage device), an additional device 940, and a power supply 950 electrically connected to each other through a bus 910. The additional device 940 may include an optical device that includes an optical modulator 100. The optical device may be an optical device including an optical modulator 100 according to any of the example embodiments, for example, a Mach-Zehnder interferometer 2 as described above. The memory 930 (and/or a memory of the additional device 940), which may be a non-transitory computer readable medium (e.g., an SSD storage device), may store a program of instructions. The processor 920, for example a CPU, may execute the stored program of instructions to perform one or more functions. The power supply 950 may include a rechargeable battery, such as a lithium-ion battery.

The processor 920 (and/or a processor of the additional device 940) may be configured to adjustably control a voltage of electrical power (e.g., electrical power supplied from the power supply 950) applied to one or more elements of an optical device and/or optical modulator of the additional device 940. For example, the processor 920 may be configured to adjustably control a supply of a modulation electrical input signal to the input electrode 13 of an optical modulator 100 (e.g., of a Mach-Zehnder interferometer 2 as shown in FIG. 1), to control an optical modulation signal that is output (transmitted) by the optical modulator 100 (e.g., transmitted to an output unit 11). The additional device 940 may include a communication interface (e.g., an optical communication interface) configured to transmit the optical modulation signal to an external device to implement optical transmissions and thus communications based on same.

The processor 920 may be configured to generate an output (e.g., cause a modulated electrical input signal to be transmitted to the input electrode 13 of the optical modulator 100) based on processing a program of instructions stored at the memory 930. In some example embodiments, the additional device 940 may be electrically connected to the power supply 950 independently of a bus 910, and a processor included in additional device 940 and/or the power supply 950 may be configured to adjustably control the application of a modulation electrical input signal to the optical modulator 100 (e.g., input electrode 13 thereof) and/or optical device including same (e.g., interferometer 2) based on electrical power supplied from the power supply 950. In some example embodiments, one or more of the bus 910, the processor 920, the memory 930, and/or the power supply 950 may be omitted.

The electronic device 900 and/or any portion thereof (e.g., processor 920, memory 930, additional device 940, power supply 950, etc.) may include processing circuitry such as a hardware including logic circuits; a hardware/software combination such as processor-implemented software; or any combination thereof. For example, the processing circuitry

may be a central processing unit (CPU), an arithmetic logic unit (ALU), a digital signal processor, a microcomputer, a field programmable gate array (FPGA), System-on-Chip (SoC), a programmable logic unit, a microprocessor, an application-specific integrated circuit (ASIC), or the like. As an example, the processing circuitry may include a non-transitory computer readable storage device. The processor 920 (e.g., a central processing unit or CPU) may, for example, control an operation of the additional device 940 (e.g., an optical modulator 100 thereof, an optical device including same, etc.), for example based on executing an instruction program stored at the memory 930 (e.g., a solid-state drive (SSD) storage device).

Hereinafter, some example embodiments are illustrated in more detail with reference to examples. However, the following examples are for illustrative purposes only and are not intended to limit the scope.

Synthesis Example 1: Synthesis of Compound Represented by Chemical Formula 1-1

[Chemical Formula 1-1]

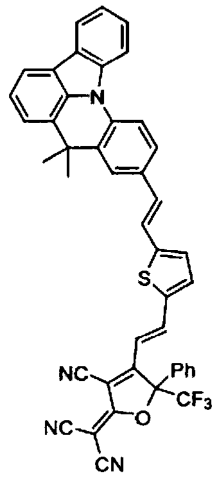

(1) Synthesis of Compound 1-1b

2.0 g (6.5 mmol) of Compound 1-1a and 3.5 g (7.9 mmol) of triphenyl(2-thienylmethyl)phosphonium bromide (CAS No. 23259-98-5) in 40 ml of tetrahydrofuran (THF) was used to prepare a suspension, and 0.6 g (60 wt%, 15.6 mmol) of NaH was added thereto. After stirring the mixture at room temperature for one day, a reaction temperature was increased to 40 °C, and the stirring was additionally performed for several more hours. Then, purification through silica gel column chromatography (by using hexane and ethyl acetate as eluents) was performed to obtain 2.4 g (6.0 mmol) of Compound 1-1b (yield: 92.5 %).

[Compound 1-1a]

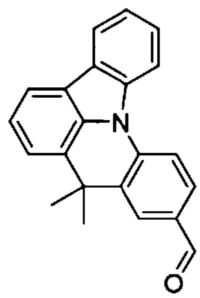

[Compound 1-1b]

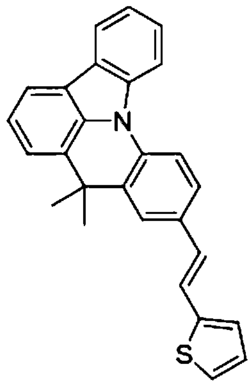

(2) Synthesis of Compound 1-1c

2.2 g (5.6 mmol) of Compound 1-1b with 300 ml of chloroform was used to prepare a solution, and 7.7 ml (23.3 mmol) of a reagent for a Vilsmeier-Haack reaction, which was pre-prepared with DMF and $POCl_3$, was added thereto at 0 °C. When the addition was completed, after increasing the temperature to room temperature, the mixture was stirred. Then, purification through silica gel column chromatography (by using hexane and ethyl acetate as eluents) was performed to obtain 1.6 g (3.8 mmol) of Compound 1-1c (yield: 69.1 %).

[Compound 1-1c]

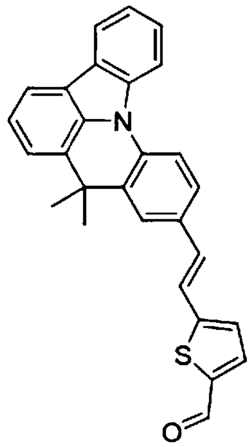

(3) Synthesis of Compound Represented by Chemical Formula 1-1

1.6 g (3.8 mmol) of Compound 1-1c and 1.1 g (3.4 mmol) of 2-[3-cyano-4-methyl-5-phenyl-5-(trifluoromethyl) furan-2(5H)-ylidene]malononitrile (CAS No. 436097-14-2) were used with a mixed solvent of 17 ml of toluene and 17 ml of ethanol to prepare a suspension and then, stirred at 55 °C for one day. Then, purification through silica gel column chromatography (by using hexane, chloroform, and acetone as eluents) was performed to obtain 2.1 g (2.9 mmol) of a compound represented by Chemical Formula 1-1 (yield: 97.8 %).

$^1H$ NMR (500 MHz, $CD_2Cl_2$): $\delta$ 1.77 (s, 6H), 6.75 (d, 1H), 7.16 (d, 1H), 7.25 (d, 2H), 7.35-7.40 (m, 3H), 7.50-7.61 (m, 8H), 7.74 (d, 1H), 7.77 (d, 1H), 7.91 (d, 1H), 8.12 (d, 1H), 8.15 (t, 2H).

**Synthesis Example 2: Synthesis of Compound Represented by Chemical Formula 1-2**

[Chemical Formula 1-2]

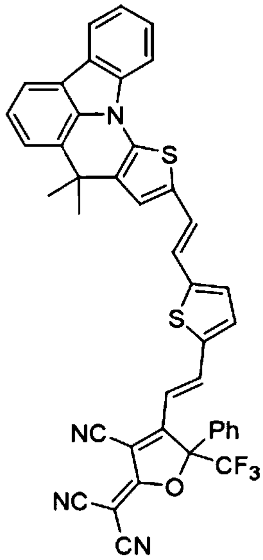

Compound 1-2b (yield: 99.9 %) was synthesized in the same manner as in the step (1) of Synthesis Example 1 except that Compound 1-2a (CAS No. 3055740-49-0) was used instead of Compound 1-1a.

[Compound 1-2a]

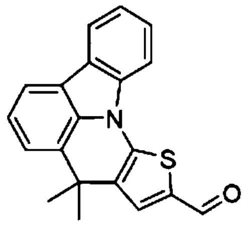

[Compound 1-2b]

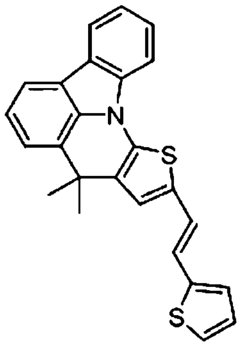

Compound 1-2c (yield: 99.9 %) was synthesized in the same manner as in the step (2) of Synthesis Example 1 except that Compound 1-2b was used instead of Compound 1-1b.

[Compound 1-2c]

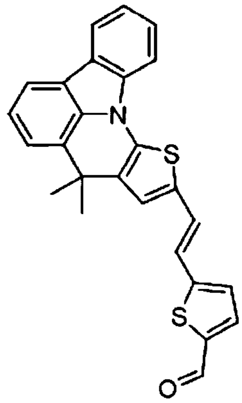

A compound represented by Chemical Formula 1-2 (yield: 67.5 %) was synthesized in the same manner as in the step (3) of Synthesis Example 1 except that Compound 1-2c was used instead of Compound 1-1c.

The $^{1}H$ NMR of one of the two stereoisomers is as follows.

$^{1}H$ NMR (500 MHz, $CD_2Cl_2$): δ 1.65 (s, 6H), 6.06 (d, 1H), 7.06 (d, 1H), 7.31-7.84 (m, 13H), 7.80 (d, 1H), 7.83 (d, 1H), 8.01-8.04 (m, 2H).

The $^{1}H$ NMR of the other of the two stereoisomers is as follows.

$^{1}H$ NMR (500 MHz, $CD_2Cl_2$): δ 1.70 (s, 6H), 6.67 (d, 1H), 6.94 (d, 1H), 7.07 (d, 1H), 7.23 (s, 1H), 7.30-7.54 (m, 11H), 7.56 (d, 1H), 7.58 (d, 1H), 7.59 (d, 1H), 7.69 (d, 1H).

Synthesis Example 3: Synthesis of Compound Represented by Chemical Formula 1-3

[Chemical Formula 1-3]

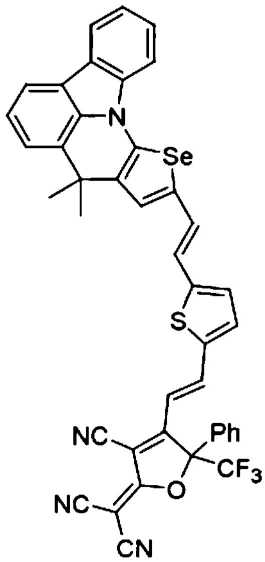

Compound 1-3b (yield: 93.3 %) was synthesized in the same manner as in the step (1) of Synthesis Example 1 except that 1-3a (CAS No. 2661611-43-2) was used instead of Compound 1-1a.

[Compound 1-3a]

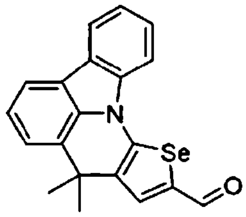

[Compound 1-3b]

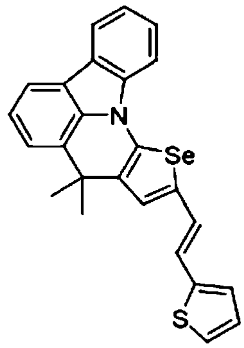

Compound 1-3c (yield: 99.9%) was synthesized in the same manner as in the step (2) of Synthesis Example 1 except that Compound 1-3b was used instead of Compound 1-1b.

[Compound 1-3c]

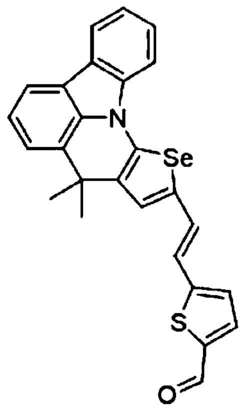

A compound represented by Chemical Formula 1-3 (yield: 55.1%) was synthesized in the same manner as in the step (3) of Synthesis Example 1 except that Compound 1-3c was used instead of Compound 1-1c. The $^1$H NMR of one of the two stereoisomers is as follows.

$^1$H NMR (500 MHz, $CD_2Cl_2$): δ 1.66 (s, 6H), 5.97 (d, 1H), 7.06 (d, 1H), 7.32 (d, 1H), 7.36-7.53 (m, 10H), 7.66 (s, 1H), 7.75 (s, 1H), 7.80 (dd, 2H), 8.04-8.05 (m, 2H).

The $^1$H NMR of the other of the two stereoisomers is as follows.

$^1$H NMR (500 MHz, $CD_2Cl_2$): δ 1.68 (s, 6H), 6.66 (d, 1H), 6.79 (d, 1H), 7.04 (d, 1H), 7.13-7.45 (m, 7H), 7.50-7.58 (m, 5H), 7.66-7.70 (m, 2H), 8.36 (d, 1H), 8.08 (d, 1H).

Synthesis Example 4: Synthesis of Compound Represented by Chemical Formula 1-4

[Chemical Formula 1-4]

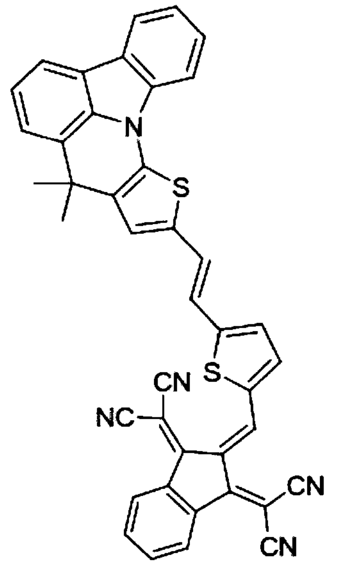

A compound represented by Chemical Formula 1-4 (yield: 54.6%) was synthesized in the same manner as in the step (3) of Synthesis Example 1 except that Compound 1-2c was used instead of Compound 1-1c, 1,3-bis(dicyanomethylidene)indan (CAS No. 38172-19-9) was used instead of the 2-[3-cyano-4-methyl-5-phenyl-5-(trifluoromethyl) furan-2(5H)-ylidene]malononitrile (CAS No. 436097-14-2), the solvent was changed to acetic anhydride, and the reaction temperature was changed to 90 °C.

$^1$H NMR (500 MHz, $CD_2Cl_2$): δ 1.73 (s, 6H), 6.93 (d, 1H), 7.12 (d, 1H), 7.32 (d, 1H), 7.39-7.43 (m, 4H), 7.47 (d, 1H), 7.59 (m, 2H), 7.80 (m, 2H), 7.85 (d, 1H), 7.89 (d, 1H), 8.10 (d, 1H), 8.55-8.57 (m, 2H).

Synthesis Example 5: Synthesis of Compound Represented by Chemical Formula 1-5

[Chemical Formula 1-5]

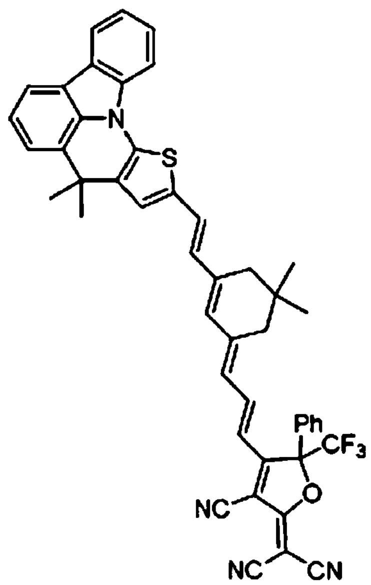

(1) Synthesis of Compound 1-5b

3.0 g (9.5 mmol) of Compound 1-2a according to Synthesis Example 2, 1.5 ml (10.4 mmol) of isophorone (CAS No. 78-59-1), and 30 ml of EtOH were used to prepare a solution, and 4.2 mL (11.3 mmol) of 21 wt% EtONa (EtOH solution) was added thereto and then, stirred at 85 °C for 2 hours. Then, purification through silica gel column chromatography (by using hexane and acetone as eluents) was performed to prepare 3.8 g (8.8 mmol) of Compound 1-5b (yield: 93.2%).

[Compound 1-5b]

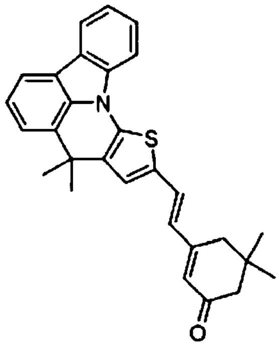

(2) Synthesis of Compound 1-5c

Subsequently, 0.33 g (60 wt%, 8.2 mmol) of NaH and 60 mL of THF were used to prepare a suspension and then, cooled to 0 °C, and 1.1 ml (7.5 mmol) of diethyl(cyanomethyl)phosphonate was slowly added in a dropwise fashion thereto. After stirring the mixture for 10 minutes, 3.0 g (6.9 mmol) of Compound 1-5b was dissolved in 3 ml of THF and then, added thereto in a dropwise fashion. After completing the dropwise addition, the mixture was stirred for 18 hours by increasing the temperature to 70 °C. Then, purification through silica gel column chromatography (by using dichloromethane as a solvent) was performed to obtain 2.7 g (5.9 mmol) of Compound 1-5c (yield: 85.5%).

[Compound 1-5c]

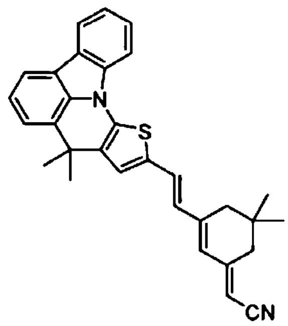

(3) Synthesis of Compound 1-5d

Subsequently, 1.0 g (2.2 mmol) of Compound 1-5c and 50 mL of toluene were used to prepare a solution, which was cooled to -78 °C, and 3.3 mL (3.3 mmol) of diisobutylaluminum hydride (1.0 M in a THF solution) was added thereto in a dropwise fashion. After stirring for 2 hours, the temperature was increased to room temperature for 1 hour. Then, purification through silica gel column chromatography (by using hexane and dichloromethane as eluents) was performed to obtain 0.9 g (1.9 mmol) of Compound 1-5d (yield: 88.8%).

[Compound 1-5d]

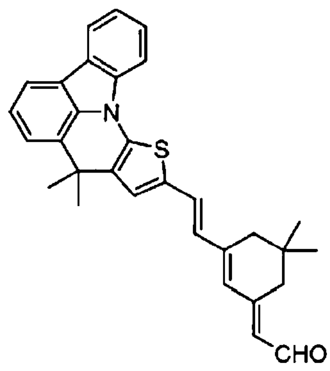

(4) Synthesis of Compound Represented by Chemical Formula 1-5

Subsequently, 0.5 g (1.0 mmol) of Compound 1-5d, 0.4 g (1.2 mmol) of 2-[3-cyano-4-methyl-5-phenyl-5-(trifluoromethyl)furan-2(5H)-ylidene]malononitrile (CAS No. 436097-14-2), and 25 ml of EtOH were used to prepare a suspension, which was stirred for one day by increasing the temperature to 55 °C. Then, purification through silica gel column chromatography (by using hexane, dichloromethane, and MeOH as eluents) was performed to obtain 0.4 g (0.5 mmol) of a compound represented by Chemical Formula 1-5 (yield: 50.8%).

$^1H$ NMR (500 MHz, $CD_2Cl_2$): $\delta$ 0.97 (s, 3H), 1.18 (s, 3H), 1.70 (s, 6H), 2.25 (ABq, 2H), 2.36 (s, 2H), 6.37-6.43 (m, 3H), 6.71 (d, 1H), 7.15 (d, 1H), 7.21 (s, 1H), 7.35-7.41 (m, 2H), 7.47 (d, 1H), 7.53-7.61 (m, 6H), 7.85 (d, 1H), 7.89-7.98 (m, 2H), 8.11 (d, 1H).

**Synthesis Example 6: Synthesis of Compound Represented by Chemical Formula 1-6**

[Chemical Formula 1-6]

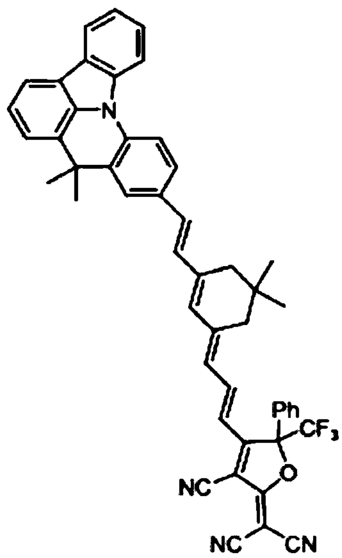

Compound 1-6b was synthesized (yield: 57.7 %) in the same manner as in the step (1) of Synthesis Example 5 except that Compound 1-1a of Synthesis Example 1 was used instead of Compound 1-2a.

[Compound 1-6b]

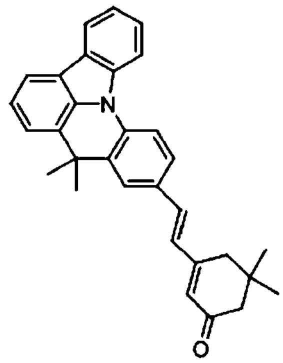

Compound 1-6b was used in the same manner as in the step (2) of Synthesis Example 5 to synthesize Compound 1-6c (yield: 64.6 %).

[Compound 1-6c]

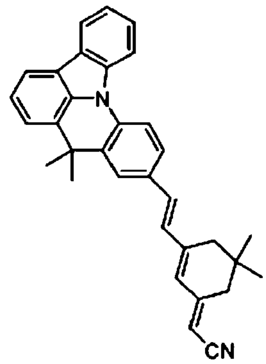

Compound 1-6c was used in the same manner as in the step (3) of Synthesis Example 5 to synthesize Compound 1-6d (yield: 39.2%).

[Compound 1-6d]

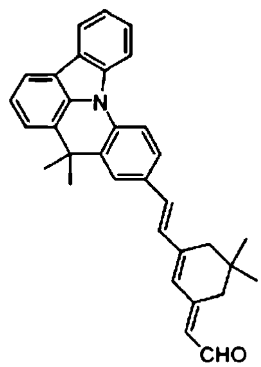

Compound 1-6d was used in the same manner as in the step (4) of Synthesis Example 5 to synthesize a compound represented by Chemical Formula 1-6 (yield: 74.2 %).

$^1H$ NMR (500 MHz, $CD_2Cl_2$): δ 0.97 (s, 3H), 1.04 (s, 3H), 1.76 (s, 6H), 2.26 (ABq, 2H), 2.45 (s, 2H), 6.40 (d, 1H), 6.46 (d, 2H), 6.49 (s, 1H), 7.00 (s, 2H), 7.36-7.40 (m, 2H), 7.50-7.59 (m, 8H), 7.75 (s, 1H), 7.90 (d, 2H), 8.10 (d, 1H), 8.16 (t, 2H).

**Synthesis Example 7: Synthesis of Compound Represented by Chemical Formula 1-7**

[Chemical Formula 1-7]

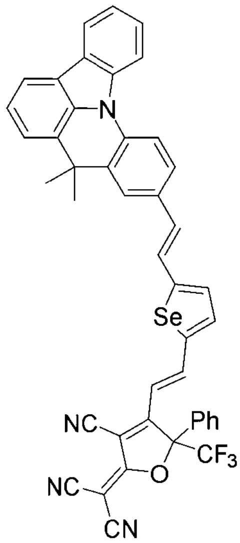

A compound represented by Chemical Formula 1-7 was obtained in the same manner as in Synthesis Example 1, except that triphenyl(2-thienylmethyl)phosphonium bromide (CAS No.: 23259-98-5) in the step (1) of Synthesis Example 1 was replaced with triphenyl(2-selenophenylmethyl)phosphonium bromide (CAS 60466-52-6).

**Synthesis Example 8: Synthesis of Compound Represented by Chemical Formula 1-8**

[Chemical Formula 1-8]

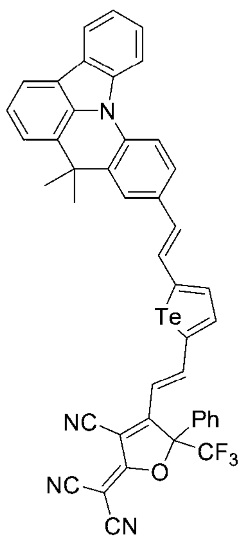

A compound represented by Chemical Formula 1-8 can be obtained in the same manner as in Synthesis Example 1, except that triphenyl(2-thienylmethyl)phosphonium bromide (CAS No.: 23259-98-5) in the step (1) of Synthesis Example 1 is replaced with triphenyl(2-tellurophenylmethyl)phosphonium bromide.

**Synthesis Example** 9: **Synthesis of Compound Represented by Chemical** Formula 1-9

[Chemical Formula 1-9]

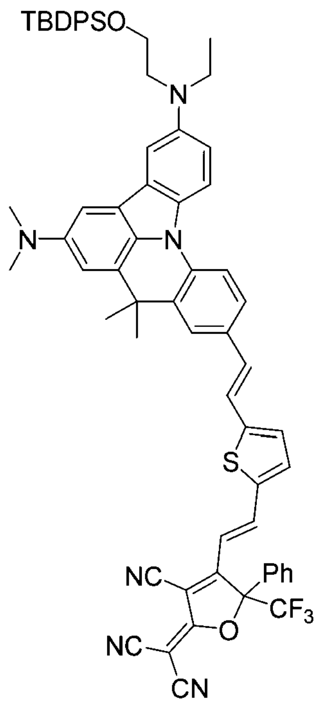

A compound represented by Chemical Formula 1-9 can be obtained in the same manner as in Synthesis Example 1, except that Compound 1-9a (6-((2-((tert-butyldiphenylsilyl)oxy)ethyl)(ethyl)amino)-3-(dimethylamino)-8,8-dimethyl-8H-indolo[3,2,1-de]acridine-10-carbaldehyde) is used instead of Compound 1-1a in the step (1) of Synthesis Example 1.

[Compound 1-9a]

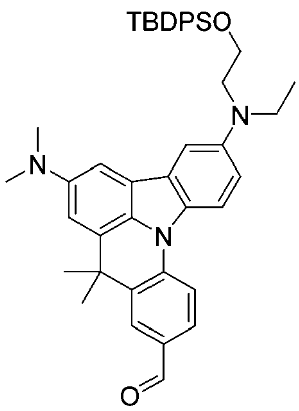

**Synthesis Example 10: Synthesis of Compound Represented** by **Chemical Formula 1-10**

[Chemical Formula 1-10]

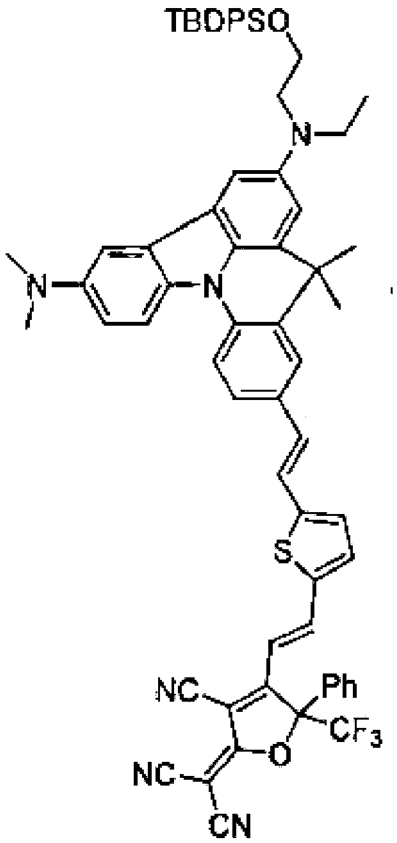

A compound represented by Chemical Formula 1-10 can be obtained in the same manner as in Synthesis Example 1, except that Compound 1-10a (3-((2-((tert-butyldiphenylsilyl)oxy)ethyl)(ethyl)amino)-6-(dimethylamino)-8,8-dimethyl-8H-indolo[3,2,1-de]acridine-10-carbaldehyde) is used instead of Compound 1-1a in the step (1) of Synthesis Example 1.

[Compound 1-10a]

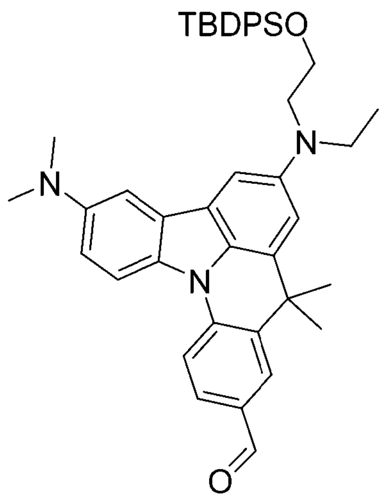

**Synthesis Example 11: Synthesis of Compound Represented by Chemical Formula 1-11**

A compound represented by Chemical Formula 1-11 can be obtained in the same manner as in Synthesis Example 5, except that Compound 1-11a (2-(2,5-dimethoxyphenyl)-4,4-dimethyl-4H-thieno[3',2':5,6]pyrido[3,2,1-jk]carbazole) is used instead of Compound 1-2a in the step (1) of Synthesis Example 5.

[Chemical Formula 1-11]

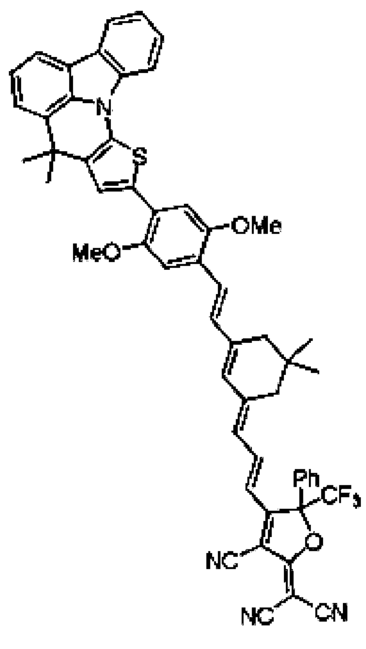

[Compound 1-11a]

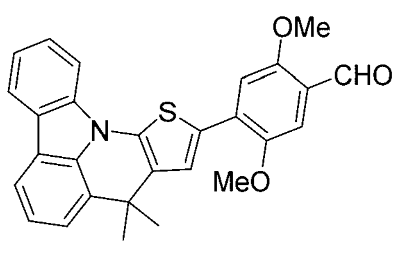

**Synthesis Example 12: Synthesis of Compound Represented by Chemical Formula 1-12**

[Chemical Formula 1-12]

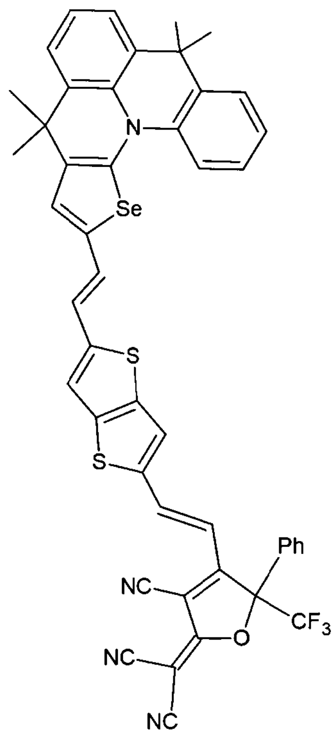

A compound represented by Chemical Formula 1-12 can be obtained in the same manner as in Synthesis Example 2, except that Compound 1-12a (4,4,8,8-tetramethyl-4H,8H-seleno[3',2':5,6]pyrido[3,2,1-de]acridine-2-carbaldehyde) is used instead of Compound 1-2a in Synthesis Example 2 and triphenyl(thieno[3,2-b]thien-2-ylmethyl)Phosphonium bromide (CAS No.: 1639046-22-2) is used instead of triphenyl(2-thienylmethyl)phosphonium bromide (CAS No.: 23259-98-5).

[Compound 1-12a]

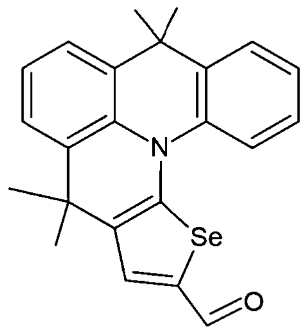

**Synthesis Example 13: Synthesis of Compound Represented by Chemical Formula 1-13**

[Chemical Formula 1-13]

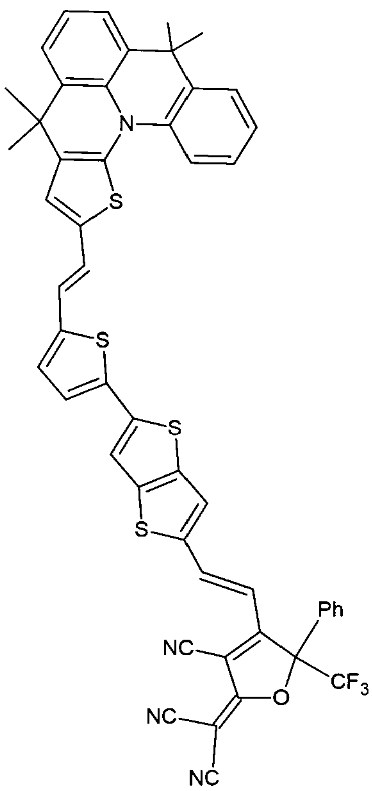

The compound represented by Chemical Formula 1-13 can be synthesized by the following method. Compound 1-13b is synthesized in the same manner as the step (1) of Synthesis Example 1, except that Compound 1-13a (4,4,8,8-tetramethyl-4H,8H-thieno[3',2':5,6]pyrido[3,2,1-de]acridine-2-carbaldehyde) is used instead of Compound 1-1a in Synthesis Example 1. Br is introduced into Compound 1-13b to obtain Compound 1-13c. Compound 1-13d is obtained by reacting Compound 1-13c with thieno[3,2-b]thiophene-2-boronic acid pinacol ester (CAS No.: 1004784-50-2). Compound 1-13e is obtained from Compound 1-13d in the same manner as the step (2) of Synthesis Example 1. A compound represented by Chemical Formula 1-13 can be obtained by the same method as the step (3) of Synthesis Example 1, except that Compound 1-13e is used instead of Compound 1-1c.

[Compound 1-13a]

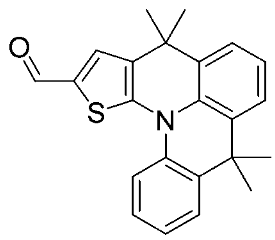

[Compound 1-13b]

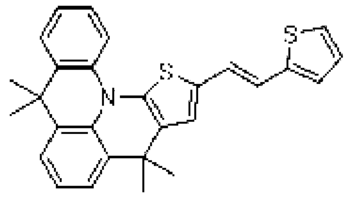

[Compound 1-13c]

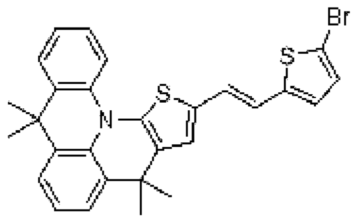

[Compound 1-13d]

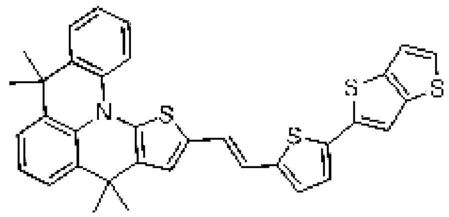

[Compound 1-13e]

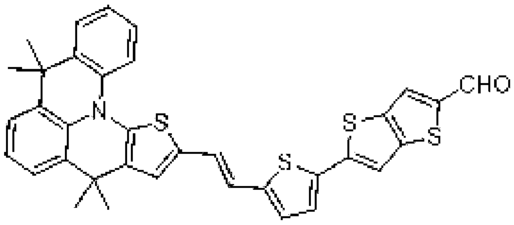

**Synthesis Example 14: Synthesis of Compound Represented by Chemical Formula 1-14**

[Chemical Formula 1-14]

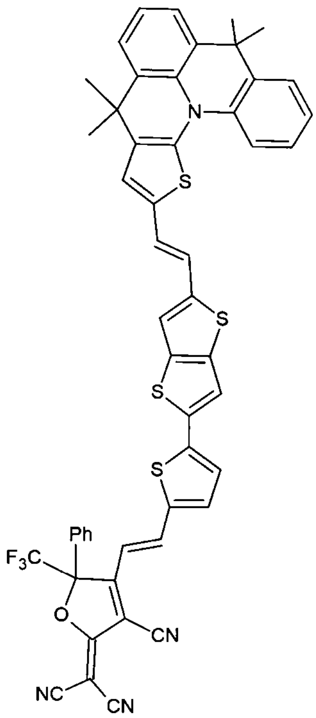

The compound represented by Chemical Formula 1-14 can be synthesized by the following method. Compound 1-14b is synthesized in the same manner as the step (1) of Synthesis Example 1, except that Compound 1-13a (4,4,8,8-tetramethyl-4H,8H-thieno[3',2':5,6]pyrido[3,2,1-de]acridine-2-carbaldehyde) is used instead of Compound 1-1a in Synthesis Example 1 and triphenyl(thieno[3,2-b]thien-2-ylmethyl)phosphonium bromide (CAS No.: 1639046-22-2) is used instead of triphenyl(2-thienylmethyl)phosphonium bromide (CAS No.: 23259-98-5) in Synthesis Example 1. Br is introduced into Compound 1-14b to obtain Compound 1-14c. Compound 1-14d is obtained by Suzuki coupling reaction with Compound 1-14c and thiophene-2-boronic acid pinacol ester (CAS No.: 193978-23-3). Compound 1-14e is obtained from Compound 1-14d by the same method (Vilsmeier-Haack reaction) as the step (2) of Synthesis Example 1. A compound represented by Chemical Formula 1-14 can be obtained by the same method as the step (3) of Synthesis Example 1, except that Compound 1-14e is used instead of Compound 1-1c.

[Compound 1-14b]

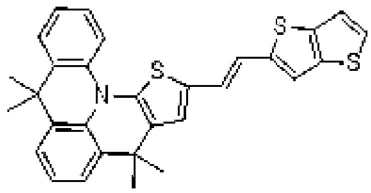

[Compound 1-14c]

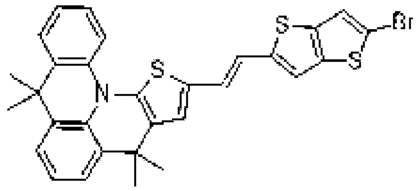

[Compound 1-14d]

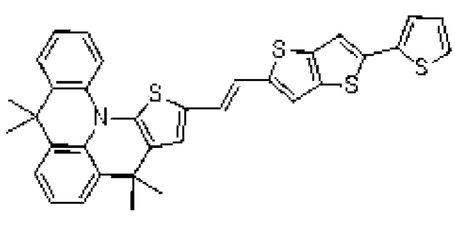

[Compound 1-14e]

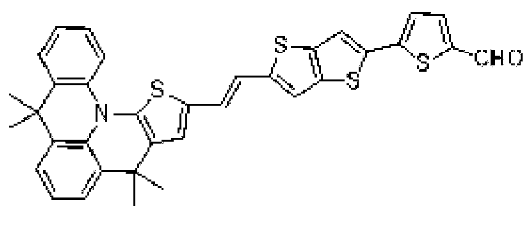

**Synthesis Example 15: Synthesis of Compound Represented by Chemical Formula 1-15**

[Chemical Formula 1-15]

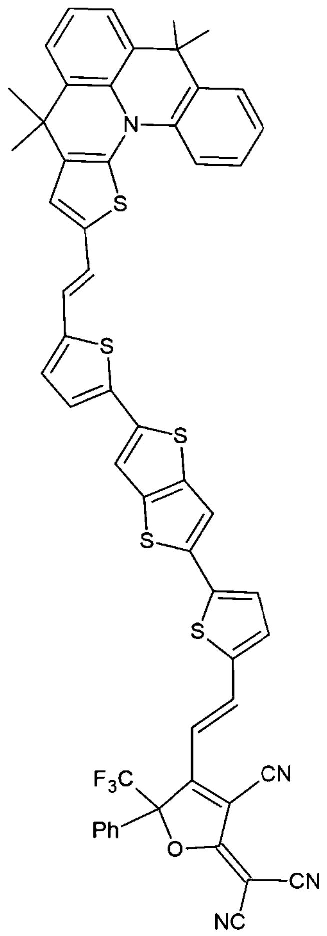

The compound represented by Chemical Formula 1-15 can be synthesized by the following method. Compound 1-13b is synthesized in the same manner as the step (1) of Synthesis Example 1, except that Compound 1-13a (4,4,8,8-tetramethyl-4H,8H-thieno[3',2':5,6]pyrido[3,2,1-de]acridine-2-carbaldehyde) is used instead of Compound 1-1a in Synthesis Example 1. Br is introduced into Compound 1-13b to obtain Compound 1-13c. Compound 1-13d is obtained by Suzuki coupling reaction with Compound 1-13c and thieno[3,2-b]thiophene-2-boronic acid pinacol ester (CAS No.: 1004784-50-2). Br is introduced into Compound 1-13d to obtain Compound 1-15e. Compound 1-15f is obtained by Suzuki coupling reaction with Compound 1-15e and thiophene-2-boronic acid pinacol ester (CAS No.: 193978-23-3). Compound 1-15g is obtained from Compound 1-15f by the same method (Vilsmeier-Haack reaction) as the step (2) of Synthesis Example 1. A compound represented by Chemical Formula 1-15 can be obtained by the same method as the step (3) of Synthesis Example 1, except that Compound 1-15g is used instead of Compound 1-1c.

[Compound 1-15e]

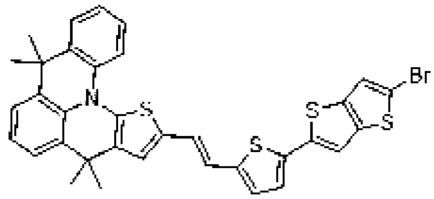

[Compound 1-15f]

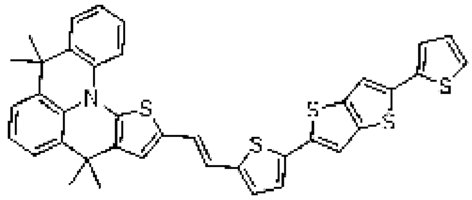

[Compound 1-15g]

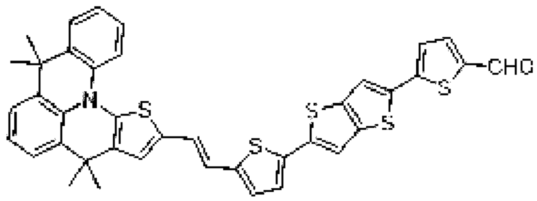

**Synthesis Example 16: Synthesis of Compound Represented by Chemical Formula 1-16**

[Chemical Formula 1-16]

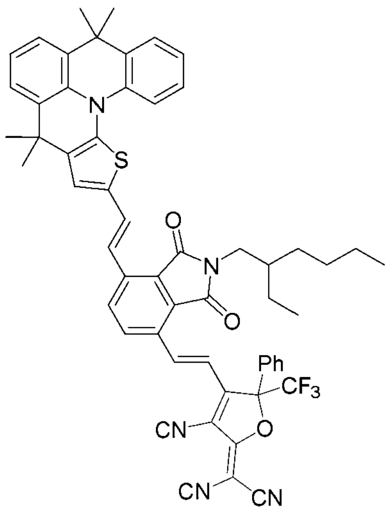

The compound represented by Chemical Formula 1-16 can be synthesized by the following method. Compound 1-16b is synthesized in the same manner as the step (1) of Synthesis Example 1, except that Compound 1-13a (4,4,8,8-tetramethyl-4H,8H-thieno[3',2':5,6]pyrido[3,2,1-de]acridine-2-carbaldehyde) is used instead of Compound 1-1a in Synthesis Example 1, and Compound 1-16a (4-((bromotriphenyl-l5-phosphaneyl)methyl)-2-(2-ethylhexyl)isoindoline-1,3-dione) is used instead of triphenyl(2-thienylmethyl)phosphonium bromide (CAS No.: 23259-98-5) in Synthesis Example 1. Compound 1-16c is obtained from Compound 1-16b by the same method (Vilsmeier-Haack reaction) as the step (2) of Synthesis Example 1. A compound represented by Chemical Formula 1-16 can be obtained by the same method as the step (3) of Synthesis Example 1, except that Compound 1-16c is used instead of Compound 1-1c.

[Compound 1-16a] [Compound 1-16b] [Compound 1-16c]

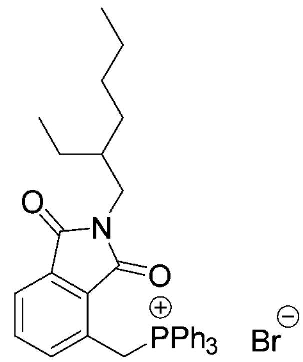
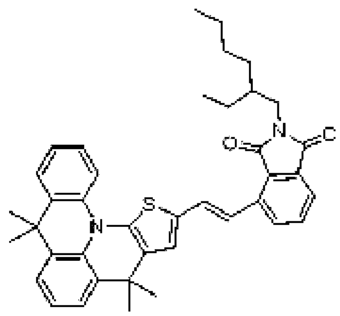
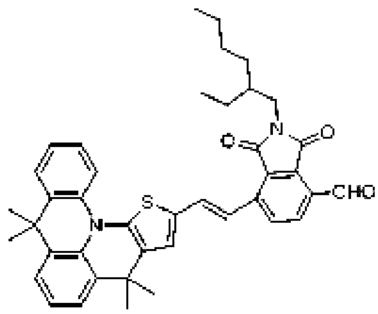

**Synthesis Example 17: Synthesis of Compound Represented by Chemical Formula 1-17**

[Chemical Formula 1-17]

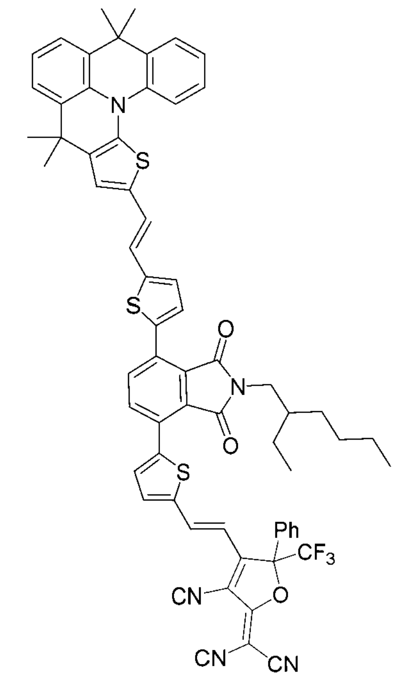

The compound represented by Chemical Formula 1-17 can be synthesized by the following method. Compound 1-17b is synthesized in the same manner as the step (1) of Synthesis Example 1, except that Compound 1-13a (4,4,8,8-tetramethyl-4H,8H-thieno[3',2':5,6]pyrido[3,2,1-de]acridine-2-carbaldehyde) is used instead of Compound 1-1a in Synthesis Example 1, and Compound 1-17a (((5-(2-(2-ethylhexyl)-1,3-dioxo-7-(thiophen-2-yl)isoindolin-4-yl)thiophen-2-yl)methyl)triphenylphosphoniumbromide) is used instead of triphenyl(2-thienylmethyl)phosphonium bromide (CAS No.: 23259-98-5) in Synthesis Example 1. Compound 1-17c is obtained from Compound 1-17b by the same method (Vilsmeier-Haack reaction) as the step (2) of Synthesis Example 1. A compound represented by Chemical Formula 1-17 can be obtained by the same method as the step (3) of Synthesis Example 1, except that Compound 1-17c is used instead of Compound 1-1c.

[Compound 1-17a] [Compound 1-17b]

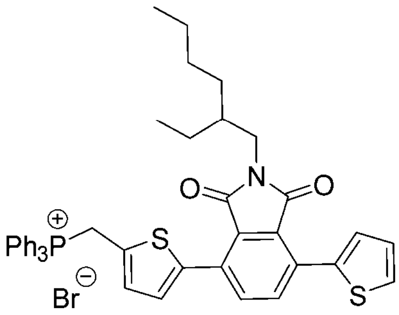

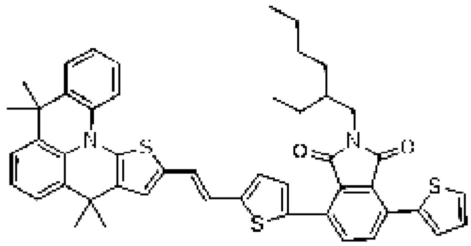

[Compound 1-17c]

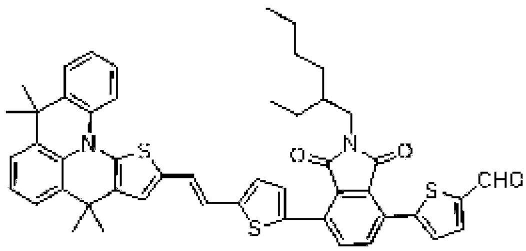

In Comparative Synthesis Examples 1 to 6, the compounds represented by Chemical Formulas 1-1C to 1-6C were synthesized according to conventional method in the present field.

[Chemical Formula 1-1C] [Chemical Formula 1-2C] [Chemical Formula 1-3C]

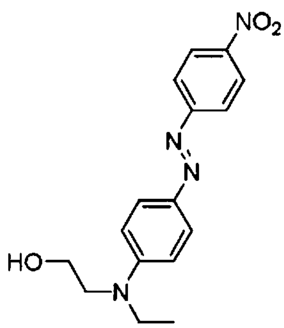

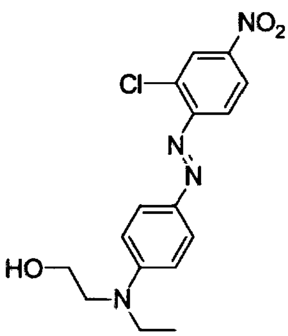

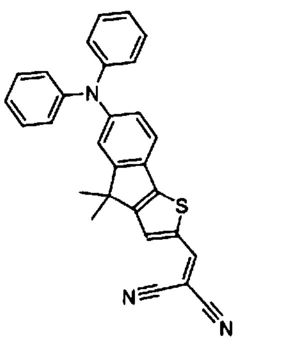

[Chemical Formula 1-4C] [Chemical Formula 1-5C] [Chemical Formula 1-6C]

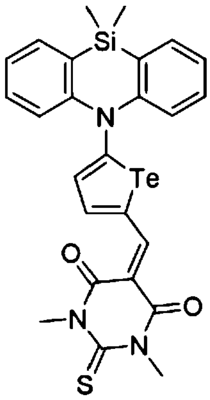
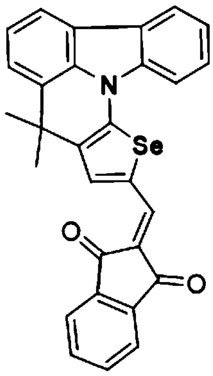
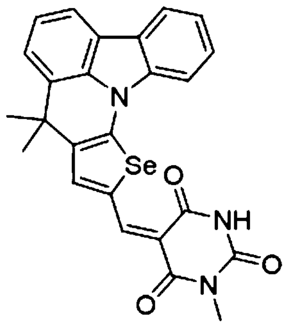

**Examples 1A to 17A and Comparative Examples 1A to 6A: Manufacturing of Evaluation Devices**

Each compound according to Synthesis Examples 1 to 17 and Comparative Synthesis Examples 1 to 6 and a polymethylmethacrylate (PMMA) matrix polymer were mixed in a cyclehexanone solvent, preparing coating compositions. The amounts of the compounds used in the coating compositions is listed in Table 3. After forming a first electrode (ITO, 100 nm) and an $Al_2O_3$ charge blocking layer (40 nm) on a glass substrate using vacuum evaporation, the coating compositions were respectively spin-coated on the $Al_2O_3$ charge blocking layer to form a 200 nm-thick active layer, and then, silver was deposited thereon to form a 100 nm-thick second electrode, manufacturing evaluation devices (optical modulators for evaluation) according to Examples 1A to 17A and Comparative Examples 1A to 6A.

**Examples 1B to 17B and Comparative Examples 1B to 6B:**

Each evaluation device was manufactured in the same manner as in Examples 1A to 17A and Comparative Examples 1A to 6A except that PC ( polycarbonate) was used instead of the polymethylmethacrylate (PMMA) as the matrix polymer.

**Evaluation 1: Measurement of Dipole Moment, Hyperpolarizability, and Glass Transition Temperature**

Dipole moments of the compounds represented by Chemical Formulas 1-1 to 1-17 according to Synthesis Examples 1 to 17 and the compounds represented by Chemical Formulas 1-1C to 1-6C according to Comparative Synthesis Examples 1 to 6 were respectively calculated by using DFT (B3LYP, M062X), and the results are shown in Table 1.

In addition, hyperpolarizability of the compounds represented by Chemical Formulas 1-1 to 1-17 according to Synthesis Examples 1 to 17 and the compounds represented by Chemical Formulas 1-1C to 1-6C according to Comparative Synthesis Examples 1 to 6 were respectively calculated by using DFT (B3LYP, M062X), and the results are shown in Table 1.

(Table 1)

| Synthesis Example | Chemical Formula | Dipole moment (Debye) | Hyperpolari zability β (0,0,0) |
|---|---|---|---|
| Synthesis Example 1 | Chemical Formula 1-1 | 19.8 | -729.1 |
| Synthesis Example 2 | Chemical Formula 1-2 | 21.2 | -1017.0 |
| Synthesis Example 3 | Chemical Formula 1-3 | 18.7 | -536.4 |
| Synthesis Example 4 | Chemical Formula 1-4 | 11.16 | -699.64 |
| Synthesis Example 5 | Chemical Formula 1-5 | 20.0 | -1307.6 |
| Synthesis Example 6 | Chemical Formula 1-6 | 17.6 | -925.3 |
| Synthesis Example 7 | Chemical Formula 1-7 | 20.2 | -794.7 |
| Synthesis Example 8 | Chemical Formula 1-8 | 20.6 | -853.3 |

(continued)

| Synthesis Example | Chemical Formula | Dipole moment (Debye) | Hyperpolari zability β (0,0,0) |
|---|---|---|---|
| Synthesis Example 9 | Chemical Formula 1-9 | 24.6 | -1404.0 |
| Synthesis Example 10 | Chemical Formula 1-10 | 24.6 | -1337.7 |
| Synthesis Example 11 | Chemical Formula 1-11 | 19.2 | -2022.8 |
| Synthesis Example 12 | Chemical Formula 1-12 | 17.1 | -718.6 |
| Synthesis Example 13 | Chemical Formula 1-13 | 17.7 | -861.6 |
| Synthesis Example 14 | Chemical Formula 1-14 | 18.0 | -1121.4 |
| Synthesis Example 15 | Chemical Formula 1-15 | 19.3 | -1436.6 |
| Synthesis Example 16 | Chemical Formula 1-16 | 14.5 | -412.6 |
| Synthesis Example 17 | Chemical Formula 1-17 | 16.0 | -474.9 |
| Comparative Synthesis Example 1 | Chemical Formula 1-1C | 9.11 | -142.0 |
| Comparative Synthesis Example 2 | Chemical Formula 1-2C | 11.8 | -151.0 |
| Comparative Synthesis Example 3 | Chemical Formula 1-3C | 10.8 | -206.2 |
| Comparative Synthesis Example 4 | Chemical Formula 1-4C | 10.2 | -35.4 |
| Comparative Synthesis Example 5 | Chemical Formula 1-5C | 2.6 | -111.4 |
| Comparative Synthesis Example 6 | Chemical Formula 1-6C | 5.4 | -75.9 |

Referring to Table 1, the compounds according to Synthesis Examples 1 to 17 were confirmed to exhibit a larger or equivalent dipole moment and a higher absolute value of hyperpolarizability than those of Comparative Synthesis Examples 1 to 6. Accordingly, the compounds according to Synthesis Examples 1 to 17 were expected to provide a high, or improved, electro-optic coefficient and to thereby enable improved performance of an optical modulator including such compounds (e.g., in an active layer, nonlinear optically active organic material, or any combination thereof) to enable improved performance of optical transmission-based communications using such an optical modulator and/or device including same.

Glass transition temperatures of the compounds according to Synthesis Examples 1 to 17 and Comparative Synthesis Examples 1 to 6 were respectively measured through DTA (differential thermal analysis) and decomposition temperatures thereof were respectively measured by TGA (thermogravimetric analysis), and among them, the results of the compounds of Synthesis Examples 1, 2, 3, 5, and 6 and Comparative Synthesis Example 2 are shown in Table 2.

(Table 2)

| | Glass transition temperature $T_g$ (°C) | Decomposition temperature $T_d$ (°C) |
|---|---|---|
| Synthesis Example 1 | 108.2 | 320.3 |
| Synthesis Example 2 | 94.7 | 307.6 |
| Synthesis Example 3 | 91.8 | 300.7 |
| Synthesis Example 5 | 101.1 | 297.9 |
| Synthesis Example 6 | 93.9 | 302.0 |
| Comparative Synthesis Example 2 | 91.3 | 280.8 |

Referring to Table 2, the compounds of Synthesis Example 1, 2, 3, 5, and 6 exhibited a higher glass transition temperature and a higher decomposition temperature than that of Comparative Synthesis Example 2, which confirmed excellent, or improved, thermal stability.

**Evaluation 2: Measurement of Electro-optic Coefficients**

The evaluation devices according to Examples 1A to 17A and Comparative Examples 1A to 6A and Examples 1B

to 17B and Comparative Examples 1B to 6B, after applying a poling field thereto, were subjected to poling at 85 °C to 150 °C for 40 minutes and rapidly quenched to 25 °C, and then, the applied voltage was lowered to 0 V.

Each electro-optic coefficient ($r_{33}$) of each of the evaluation devices according to Examples 1A to 17A and Comparative Examples 1A to 6A and Examples 1B to 17B and Comparative Examples 1B to 6B was measured and calculated according to Relationship Equation 1. The results of the evaluation devices according to Examples 1A and 1B, Example 2B, Example 3B, Examples 5A and 6B, and Comparative Examples 1A and 2A are shown in Table 3.

(Table 3)

| | Polymer | Solvent | Concentration 1 [wt%] | Concentration 2 [wt%] | Temperature [°C] | Voltage [V] | $r_{33}$ [pm/V] |
|---|---|---|---|---|---|---|---|
| Example 1A | PMMA | Cyclopentanone | 10 | 10 | 85 | 70 | 60.0 |
| Example 1B | PC | Chlorobenzene | 10 | 5 | 150 | 50 | 69.4 |
| Example 2B | PC | Dibromomethane | 20 | 10 | 150 | 140 | 226.0 |
| Example 3B | PC | Dibromomethane | 30 | 10 | 150 | 170 | 343 |
| Example 5A | PMMA | Cyclopentanone | 10 | 20 | 100 | 120 | 10.6 |
| Example 6B | PC | Chlorobenzene | 10 | 5 | 150 | 50 | 17.0 |
| Comparative Example 1A | PMMA | Cyclopentanone | 10 | 20 | 85 | 100 | 1 |
| Comparative Example 2A | PMMA | Cyclopentanone | 10 | 20 | 85 | 100 | 3 |

In Table 3, "Concentration 1" refers to concentrations (wt%) of each compound relative to the total amount of the matrix polymer and each compound, and "Concentration 2" refers to the concentration (wt%) of the matrix polymer and compound relative to the total amount of the coating composition.

Referring to Table 3, the electro-optic coefficients ($r_{33}$) of the evaluation devices according to Examples 1A, 1B, 2B, 3B, 5A, and 6B exhibited an excellent, or significantly improved, electro-optic coefficient ($r_{33}$), compared to those of Comparative Examples 1A and 2A, thereby indicating that the evaluation devices according to Examples 1A, 1B, 2B, 3B, 5A, and 6B may enable improved performance of optical transmission-based communications.

While the inventive concepts have been described in connection with what is presently considered to be practical example embodiments, it is to be understood that the inventive concepts are not limited to such example embodiments. On the contrary, the scope of the inventive concepts is intended to cover various modifications and equivalent arrangements included within the scope of the appended claims.

**<Description of symbols>**

| | | | |
|---|---|---|---|
| 100: | optical modulator | 2: | Mach-Zehnder interferometer |
| 3: | input section | 4: | branch section |
| 5: | first branch waveguide | 6: | second branch waveguide |
| 7: | combining section | 8: | output section |
| 14: | first electrode | 30: | second electrode |
| 20: | active layer | | |

**Claims**

1. A compound represented by Chemical Formula 1:

[Chemical Formula 1]

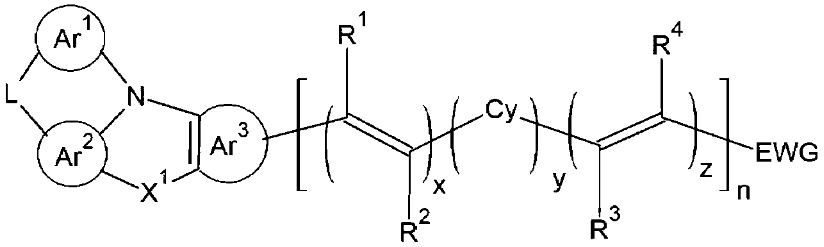

wherein, in Chemical Formula 1,

$Ar^1$, $Ar^2$, and $Ar^3$ are each independently a substituted or unsubstituted C6 to C30 arene group, a substituted or unsubstituted C3 to C30 heteroarene group, or any combination thereof,
$X^1$ is a single bond, -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-(CR^fR^g)_{n1}$, $-CR^{ff}R^{gg}-$,$-C(R^{mm})=C(R^{nn})-$, or $-C(R^p)=N-$, wherein

$R^{a1}$, $R^{a2}$, $R^b$, $R^c$, $R^d$, $R^e$, $R^f$, $R^g$, $R^m$, $R^n$, and $R^p$ are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and
$R^{bb}$, $R^{cc}$, $R^{dd}$, $R^{ee}$, $R^{ff}$, and $R^{gg}$ are each independently $(CH_2)_x$ or a heteroatom of O, N, S, Se, or Te, wherein x in $(CH_2)_x$ is a positive integer, and wherein a pair of $R^{bb}$ and $R^{cc}$, a pair of $R^{dd}$ and $R^{ee}$, or a pair of $R^{ff}$ and $R^{gg}$ are linked to each other to form a first ring structure, and
in $-(CR^fR^g)_{n1}-$, n1 is 1 or 2,

L is a single bond, -O-, -S-, -Se-, -Te-, $-NR^{a1}-$, $-BR^{a2}-$, $-SiR^bR^c-$, $-SiR^{bb}R^{cc}-$, $-GeR^dR^e-$, $-GeR^{dd}R^{ee}-$, $-(CR^fR^g)_{n1}-$, $-CR^{ff}R^{gg}-$, $-C(R^m)=C(R^n)-$, or $-C(R^{mm})=C(R^{nn})-$, or $-C(R^p)=N-$, wherein

$R^{a1}$, $R^{a2}$, $R^b$, $R^c$, $R^d$, $R^e$, $R^f$, $R^g$, $R^m$, $R^n$, and $R^p$ are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group,
$R^{bb}$, $R^{cc}$, $R^{dd}$, $R^{ee}$, $R^{ff}$, $R^{gg}$ $R^{mm}$, and $R^{nn}$ are each independently $(CH_2)_y$ or a heteroatom of O, N, S, Se, or Te, wherein y in $(CH_2)_y$ is a positive integer, and wherein a pair of $R^{bb}$ and $R^{cc}$, a pair of $R^{dd}$ and $R^{ee}$, a pair of $R^{ff}$ and $R^{gg}$, or a pair of $R^{mm}$ and $R^{nn}$ are linked to each other to form a second ring structure, and
in $-(CR^fR^g)_{n1}-$, n1 is 1 or 2,
wherein, when L is $-NR^{a1}-$, $-BR^{a2}-$, $-SiR^bR^c-$, $-GeR^dR^e-$, $-(CR^fR^g)_{n1}-$, $-(C(R^m)=C(R^n))-$, or $-(C(R^p)=N))-$, L is optionally linked to Ar' or $Ar^2$ to form a third ring structure,

Cy is a substituted or unsubstituted C6 to C10 arylene group, a substituted or unsubstituted C3 to C10 heteroarylene group, a substituted or unsubstituted C3 to C10 cycloalkylene group, a substituted or unsubstituted C3 to C10 cycloalkenylene group, a substituted or unsubstituted C3 to C10 heterocycloalkylene group, a substituted or unsubstituted C3 to C10 heterocycloalkenylene group, or any combination thereof,
EWG is $CR^aR^b$, wherein $R^a$ and $R^b$ are each independently a cyano group or a cyano-containing group; a substituted or unsubstituted C6 to C30 hydrocarbon ring group having at least one functional group selected from C=O, C=S, C=Se, C=Te, and $CR^aR^b$, wherein $R^a$ and $R^b$ are each independently a cyano group or a cyano-containing group; a substituted or unsubstituted C2 to C30 heterocyclic group having at least one functional group selected from C=O, C=S, C=Se, C=Te, and $CR^aR^b$, wherein $R^a$ and $R^b$ are each independently a cyano group or a cyano-containing group; or any combination thereof,
$R^1$ to $R^4$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted C2 to C30 acyl group, a halogen, a cyano group (-CN), a cyano-containing group, a nitro group, a pentafluorosulfanyl group ($-SF_5$), a hydroxyl group, an amine group, a hydrazine group, a hydrazone group, a carboxyl group or a salt thereof, a sulfonic acid group or a salt thereof, a phosphoric acid group or a salt thereof, $-SiR^aR^bR^c$, wherein $R^a$, $R^b$, and $R^c$ are each independently hydrogen or a substituted or unsubstituted C1 to C10 alkyl group, or any combination thereof,

x is an integer from 0 to 5,
y is an integer from 1 to 5,
z is an integer from 0 to 5, and
n is an integer from 1 to 5.

**2.** The compound of claim 1, wherein the compound represented by Chemical Formula 1 is represented by Chemical Formula 2:

[Chemical Formula 2]

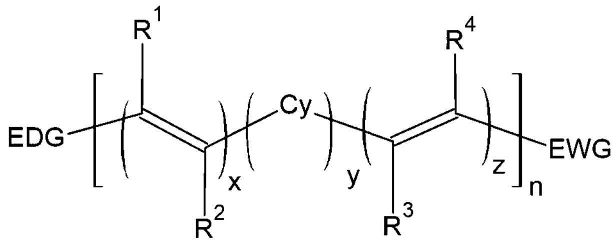

wherein, in Chemical Formula 2, EDG is an electron donor moiety represented by Chemical Formula 2A:

[Chemical Formula 2A]

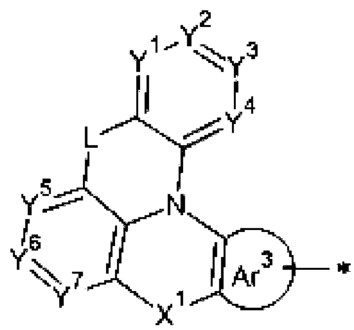

wherein, in Chemical Formula 2 and Chemical Formula 2A,

$X^1$, L, Cy, $Ar^3$, $R^1$ to $R^4$, EWG, x, y, z, and n are the same as in Chemical Formula 1,
$Y^1$ to $Y^7$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$s are linked to each other to form a substituted or unsubstituted C6 to C30 arene group, a substituted or unsubstituted C3 to C30 heteroarene group, or a condensed ring thereof, and
* indicates a linking point with Chemical Formula 2.

**3.** The compound of claim 2, wherein, in Chemical Formula 2,

$Y^4$ is N or $CR^k$, wherein $R^k$ is a halogen, a cyano group, a C1 to C10 haloalkyl group, a C1 to C10 cyanoalkyl group, or an amine group, or
$Y^7$ is N or $CR^k$, wherein $R^k$ is a halogen, a cyano group, a C1 to C10 haloalkyl group, a C1 to C10 cyanoalkyl group, or an amine group, and $X^1$ is -O-, -S-, -Se-, -Te-, -S(=O)-, $-S(=O)_2-$, $-NR^{a1}-$, $-BR^{a2}-$, $-SiR^bR^c-$, $-GeR^dR^e-$, $-(CR^fR^g)_{n1}-$, $-C(R^m)=C(R^n)-$, or $-C(R^p)=N-$, wherein $R^{a1}$, $R^{a2}$, $R^b$, $R^c$, $R^d$, $R^e$, $R^f$, $R^g$, $R^m$, $R^n$ and $R^p$ are each independently halogen, a C1 to C20 haloalkyl group, or a C1 to C20 cyanoalkyl group, and wherein n1 in $-(CR^fR^g)_{n1}-$ is 1 or 2.

**4.** The compound of claim 2, wherein

EDG in Chemical Formula 2 is represented by Chemical Formula 2AA:

[Chemical Formula 2AA]

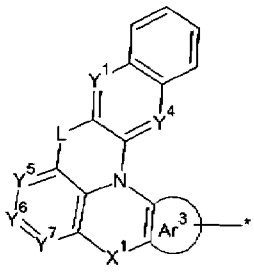
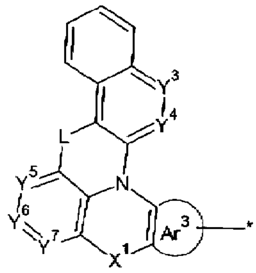
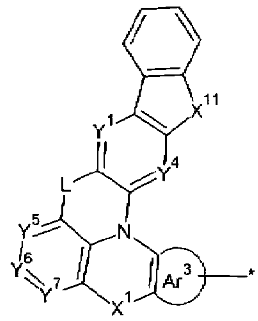
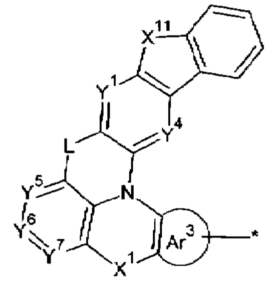
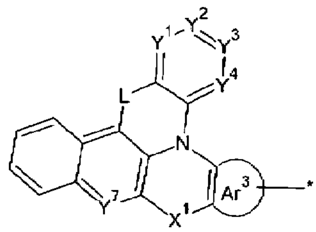
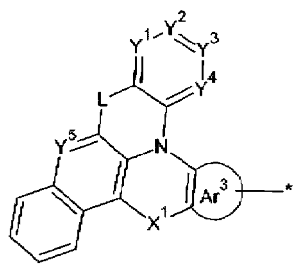

wherein, in Chemical Formula 2AA,
$X^1$, L, $Y^1$ to $Y^7$, and $Ar^3$ are the same as in Chemical Formula 2A,
$X^{11}$ is the same as $X^1$ in Chemical Formula 1, and
* indicates a linking point with an adjacent $(C(R^1)=C(R^2))_x$ or $(Cy)_y$ group of Chemical Formula 2.

**5.** The compound of claim 1, wherein

in Chemical Formula 1, the first ring structure and the second ring structure are each independently a spiro structure or a fused ring structure,
wherein the spiro structure preferably includes one of a plurality of moieties represented by Group 1:

[Group 1]

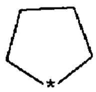

(1)

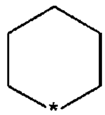

(2)

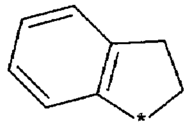

(3)

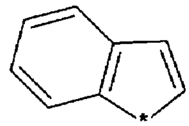

(4)

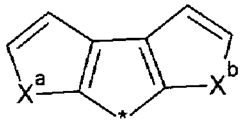

(5)

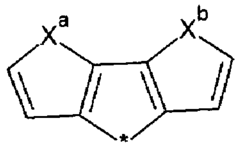

(6)

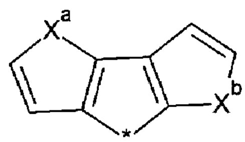

(7)

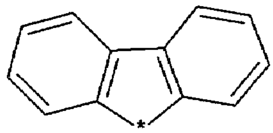

(8)

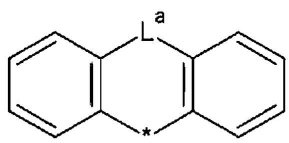

(9)

wherein, in Group 1,

$X^a$ and $X^b$ are each independently -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$, or $-GeR^{dd}R^{ee}-$, wherein $R^{a1}$, $R^{a2}$, $R^b$, $R^c$, $R^d$ and $R^e$ are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and $R^{bb}$, $R^{cc}$, $R^{dd}$, and $R^{ee}$ are each independently $(CH_2)_z$, or a heteroatom of O, N, S, Se, or Te, wherein z in $(CH_2)_z$ is a positive integer, and wherein a pair of $R^{bb}$ and $R^{cc}$ or a pair of $R^{dd}$ and $R^{ee}$ are linked to each other to form a fourth ring structure,

$L^a$ is -O-, -S-, -Se-, -Te-, $-NR^{a1}-$, $-BR^{a2}-$, $-SiR^bR^c-$, $-GeR^dR^e-$, $-(CR^fR^g)_{n1}-$, $-(C(R^p)=N))-$, or a single bond, wherein $R^{a1}$, $R^{a2}$, $R^b$, $R^c$, $R^d$, $R^e$, $R^f$, $R^g$, and $R^p$ are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and wherein n1 in - $(CR^fR^g)_{n1}$- is 1 or 2,

one or more hydrogens of each ring are optionally replaced with at least one substituent selected from deuterium, halogen, a substituted or unsubstituted C1 to C20 alkyl group, a substituted or unsubstituted C1 to C20 alkoxy group, a substituted or unsubstituted C6 to C20 aryl group, and a substituted or unsubstituted C6 to C20 aryloxy group and

CH present in the aromatic rings of moieties (3), (4), (5), (6) and (7) is optionally replaced with N.

**6.** The compound of claim 1, wherein

in Chemical Formula 1, $Ar^3$ is any one of a plurality of moieties represented by Group 2:

[Group 2]

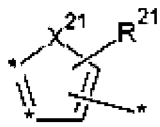
(1)
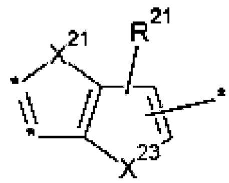
(2)
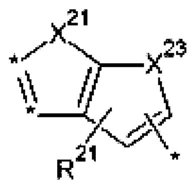
(3)

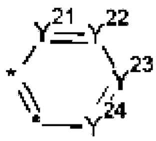
(4)
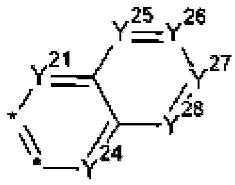
(5)
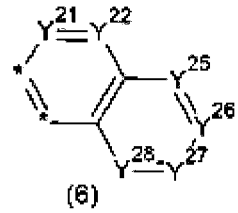
(6)

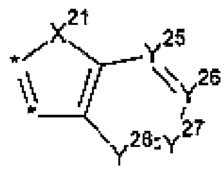
(7)
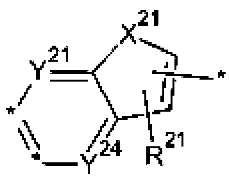
(8)
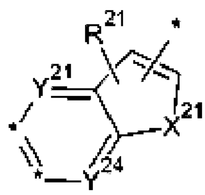
(9)

wherein, in Group 2,

$X^{21}$ and $X^{23}$ are each independently a single bond, -O-, -S-, -Se-, -Te-, -S(=O)-, $-S(=O)_2-$, $-NR^{a1}-$, $-BR^{a2}-$, $-SiR^bR^c-$, $-SiR^{bb}R^{cc}-$, $-GeR^dR^e-$ , $-GeR^{dd}R^{ee}-$, $-CR^fR^g-$, or $-CR^{ff}R^{gg}-$, wherein $R^{a1}$, $R^{a2}$, $R^b$, $R^c$, $R^d$ , $R^e$, $R^f$, and $R^g$ are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C6 to C20 aryloxy group, and $R^{bb}$, $R^{cc}$, $R^{dd}$, $R^{ee}$, $R^{ff}$, and $R^{gg}$ are each independently $(CH_2)_w$ or a heteroatom of O, N, S, Se, or Te, wherein w in $(CH_2)_w$ is a positive integer, and wherein a pair of $R^{bb}$ and $R^{cc}$, a pair of $R^{dd}$ and $R^{ee}$, or a pair of $R^{ff}$ and $R^{gg}$ are linked to each other to form a fifth ring structure,

$R^{21}$ is hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C3 to C30 heteroaryl group, a substituted or unsubstituted C2 to C30 acyl group, a halogen, a cyano group (-CN), a cyano-containing group, a nitro group, a pentafluorosulfanyl group ($-SF_5$), a hydroxyl group, an amine group, a hydrazine group, a hydrazone group, a carboxyl group or a salt thereof, a sulfonic acid group or a salt thereof, a phosphoric acid group or a salt thereof, $-SiR^aR^bR^c$, wherein $R^a$, $R^b$, and $R^c$ are each independently hydrogen or a substituted or unsubstituted C1 to C10 alkyl group, or any combination thereof,

$Y^{21}$ to $Y^{28}$ are each independently N or $CR^k$, wherein $R^k$ is hydrogen, deuterium, a halogen, a cyano group, a nitro group, a hydroxyl group, an amine group, a substituted or unsubstituted C1 to C10 alkyl group, or a substituted or unsubstituted C1 to C10 alkoxy group, or adjacent $R^k$s are optionally linked to each other to form a substituted or unsubstituted C6 to C30 arene group, a substituted or unsubstituted C3 to C30 heteroarene group, or a condensed ring thereof, provided that, in Group 2, any one of $Y^{21}$ to $Y^{24}$ in moiety (4), any one of $Y^{25}$ to $Y^{28}$ in moiety (5), any one of $Y^{25}$ to $Y^{28}$ in moiety (6), or any one of $Y^{25}$ to $Y^{28}$ in moiety (7) is a group linked with an adjacent $(C(R^1)=C(R^2))_x$ or $(Cy)_y$ group of Chemical Formula 1,

*=* is a moiety fused to an $X^1$-containing ring of Chemical Formula 1, and

-* is a linking point with an adjacent $(C(R^1)=C(R^2))_x$ or $(Cy)_y$ group of Chemical Formula 1.

**7.** The compound of claim 1, wherein

in Chemical Formula 1, Cy is any one of a plurality of moieties represented by Group 3:

[Group 3]

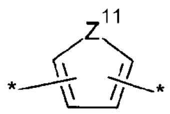
(1)
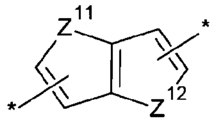
(2)
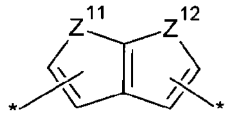
(3)

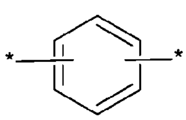
(4)
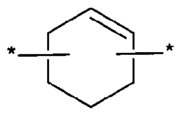
(5)
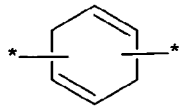
(6)
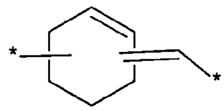
(7)
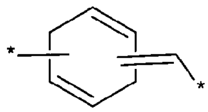
(8)

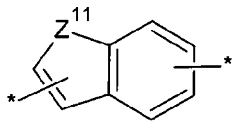
(9)
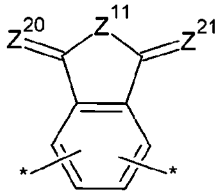
(10)
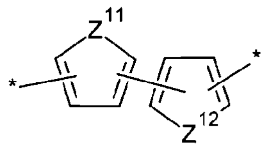
(11)
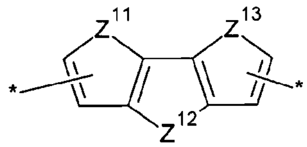
(12)

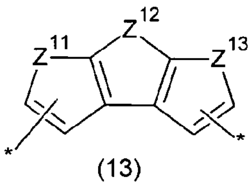
(13)
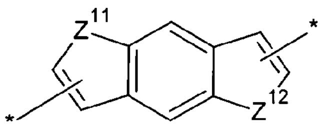
(14)
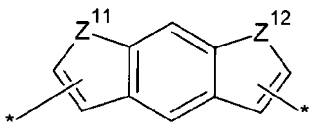
(15)

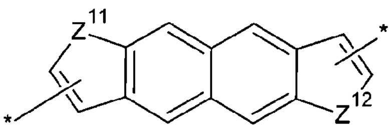
(16)
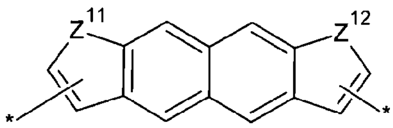
(17)

wherein, in Group 3,

$Z^{11}$, $Z^{12}$, and $Z^{13}$ are each independently O, S, Se, Te, S(=O), $S(=O)_2$, $NR^a$, or $SiR^bR^c$, wherein $R^a$, $R^b$, and $R^c$ are each independently hydrogen, a C1 to C10 alkyl group, a C1 to C10 haloalkyl group, $-SiH_3$, a C1 to C10 alkylsilyl group, $-NH_2$, a C1 to C10 alkylamine group, a C6 to C12 aryl group, a C3 to C12 heteroaryl group, a halogen, a cyano group, or any combination thereof,
$Z^{20}$ and $Z^{21}$ are each independently O, S, Se, or Te,
$R^b$ and $R^c$ are each independently present or are linked to form a sixth ring structure, and
* is a linking point with Chemical Formula 1.

**8.** The compound of claim 1, wherein

in Chemical Formula 1, EWG is a ring group represented by Chemical Formula 3:

[Chemical Formula 3]

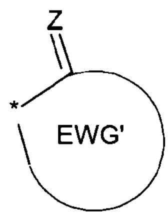

wherein, in Chemical Formula 3,
EWG' is a substituted or unsubstituted C6 to C30 aryl group, a substituted or unsubstituted C3 to C30 heteroaryl group, or a substituted or unsubstituted C3 to C30 heterocycloalkenyl group,
Z is O, S, Se, Te, or $CR^aR^b$, wherein $R^a$ and $R^b$ are each independently a cyano group or a cyano-containing group, and
* is a linking point with Chemical Formula 1.

**9.** The compound of claim 1, wherein

in Chemical Formula 1, EWG is a ring group represented by any one of Chemical Formula 3A to Chemical Formula 3G:

[Chemical Formula 3A]

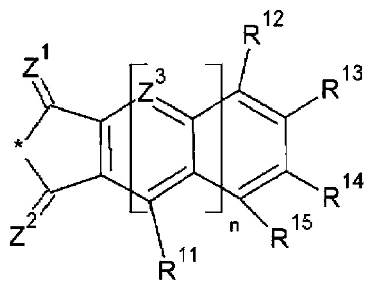

wherein, in Chemical Formula 3A,

$Z^1$ is O, S, Se, Te, or $CR^aR^b$, wherein $R^a$ and $R^b$ are each independently a cyano group or a cyano-containing group,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group,
$Z^3$ is N or $CR^c$, wherein $R^c$ is hydrogen, deuterium, or a substituted or unsubstituted C1 to C10 alkyl group,
$R^{11}$, $R^{12}$, $R^{13}$, $R^{14}$, and $R^{15}$ are the same or different and are each independently hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof, wherein $R^{12}$ and $R^{13}$ and $R^{14}$ and $R^{15}$ are each independently present or are linked to each other to form a fused aromatic ring,
n is 0 or 1, and
* indicates a linking point with Chemical Formula 1,

[Chemical Formula 3B]

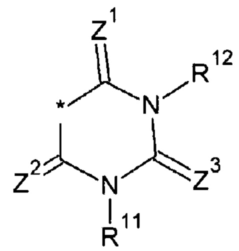

wherein, in Chemical Formula 3B,

$Z^1$ is O, S, Se, Te, or $CR^aR^b$, wherein $R^a$ and $R^b$ are each independently a cyano group or a cyano-containing group,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group,
$Z^3$ is O, S, Se, Te, or $C(R^a)(CN)$, wherein $R^a$ is hydrogen, a cyano group (-CN), or a C1 to C10 alkyl group,
$R^{11}$ and $R^{12}$ are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* indicates a linking point with Chemical Formula 1,

[Chemical Formula 3C]

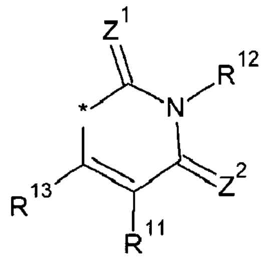

wherein, in Chemical Formula 3C,

$Z^1$ is O, S, Se, Te, or $CR^aR^b$, wherein $R^a$ and $R^b$ are each independently a cyano group or a cyano-containing group,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group,
$R^{11}$, $R^{12}$, and $R^{13}$ are the same or different and are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* indicates a linking point with Chemical Formula 1,

[Chemical Formula 3D]

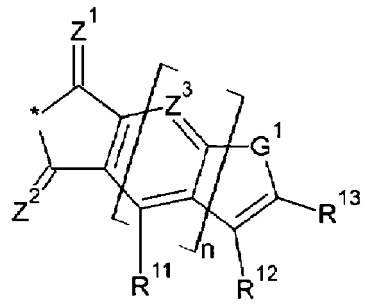

wherein, in Chemical Formula 3D,

$Z^1$ is O, S, Se, Te, or $CR^aR^b$, wherein $R^a$ and $R^b$ are each independently a cyano group or a cyano-containing group,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group,
$Z^3$ is N or $CR^c$, wherein $R^c$ is hydrogen or a substituted or unsubstituted C1 to C10 alkyl group,
$G^1$ is O, S, Se, Te, $SiR^xR^y$, or $GeR^zR^w$, wherein $R^x$, $R^y$, $R^z$, and $R^w$ are the same or different and are each independently hydrogen, deuterium, a halogen, a substituted or unsubstituted C1 to C20 alkyl group, or a substituted or unsubstituted C6 to C20 aryl group,
$R^{11}$, $R^{12}$, and $R^{13}$ are the same or different and are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof, wherein $R^{12}$ and $R^{13}$ are each independently present or linked to each other to form a fused aromatic ring,
n is 0 or 1, and
* indicates a linking point with Chemical Formula 1,

[Chemical Formula 3E]

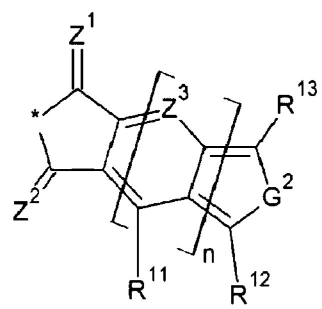

wherein, in Chemical Formula 3E,

$Z^1$ is O, S, Se, Te, or $CR^aR^b$, wherein $R^a$ and $R^b$ are each independently a cyano group or a cyano-containing group,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group,
$Z^3$ is N or $CR^c$, wherein $R^c$ is hydrogen or a substituted or unsubstituted C1 to C10 alkyl group,
$G^2$ is O, S, Se, Te, $SiR^xR^y$, or $GeR^zR^w$, wherein $R^x$, $R^y$, $R^z$, and $R^w$ are the same or different and are each independently hydrogen, deuterium, a halogen, a substituted or unsubstituted C1 to C20 alkyl group, or a substituted or unsubstituted C6 to C20 aryl group,
$R^{11}$, $R^{12}$ and $R^{13}$ are the same or different and are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a

cyano group, a cyano-containing group, or any combination thereof,
n is 0 or 1, and
* indicates a linking point with Chemical Formula 1,

[Chemical Formula 3F]

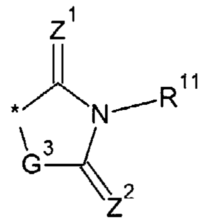

wherein, in Chemical Formula 3F,

$Z^1$ is O, S, Se, Te, or $CR^aR^b$, wherein $R^a$ and $R^b$ are each independently a cyano group or a cyano-containing group,
$Z^2$ is O, S, Se, Te, or $CR^aR^b$, wherein $R^a$ and $R^b$ are each independently hydrogen, a substituted or unsubstituted C1 to C10 alkyl group, a cyano group, or a cyano-containing group,
$R^{11}$ is hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof,
$G^3$ is O, S, Se, Te, $SiR^xR^y$, or $GeR^zR^w$, wherein $R^x$, $R^y$, $R^z$, and $R^w$ are the same or different and are each independently hydrogen, deuterium, a halogen, a substituted or unsubstituted C1 to C20 alkyl group, or a substituted or unsubstituted C6 to C20 aryl group, and
* indicates a linking point with Chemical Formula 1,

[Chemical Formula 3G]

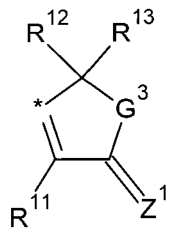

wherein, in Chemical Formula 3G,

$Z^1$ is O, S, Se, Te, or $CR^aR^b$, wherein $R^a$ and $R^b$ are each independently a cyano group or a cyano-containing group,
$R^{11}$ to $R^{13}$ are hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof,
$G^3$ is O, S, Se, Te, $SiR^xR^y$, or $GeR^zR^w$, wherein $R^x$, $R^y$, $R^z$, and $R^w$ are the same or different and are each independently hydrogen, deuterium, a halogen, a substituted or unsubstituted C1 to C20 alkyl group, or a substituted or unsubstituted C6 to C20 aryl group, and
* indicates a linking point with Chemical Formula 1.

**10.** The compound of one of claims 1 to 9 having one of the following:

a hyperpolarizability of less than or equal to about -170,
a dipole moment of greater than or equal to about 15 Debye, and
a glass transition temperature of less than or equal to about 150 °C.

**11.** A nonlinear optically active organic material, comprising the compound of one of claims 1 to 10.

**12.** An optical modulator, comprising the compound of one of claims 1 to 10.

**13.** The optical modulator of claim 12 further comprising:

a first electrode and a second electrode facing each other; and
an active layer between the first electrode and the second electrode, wherein the active layer includes the compound.

**14.** The optical modulator of claim 13, wherein

the optical modulator further includes a charge blocking layer between the first electrode and the active layer, or the active layer further includes a photoconductive polymer, a photosensitizer, or any combination thereof.

**15.** An optical device, comprising:

an input section;
a branch section;
a waveguide;
a combining section; and
an output section,
wherein the waveguide includes the optical modulator according to one of claims 12 to 14.

FIG. 1

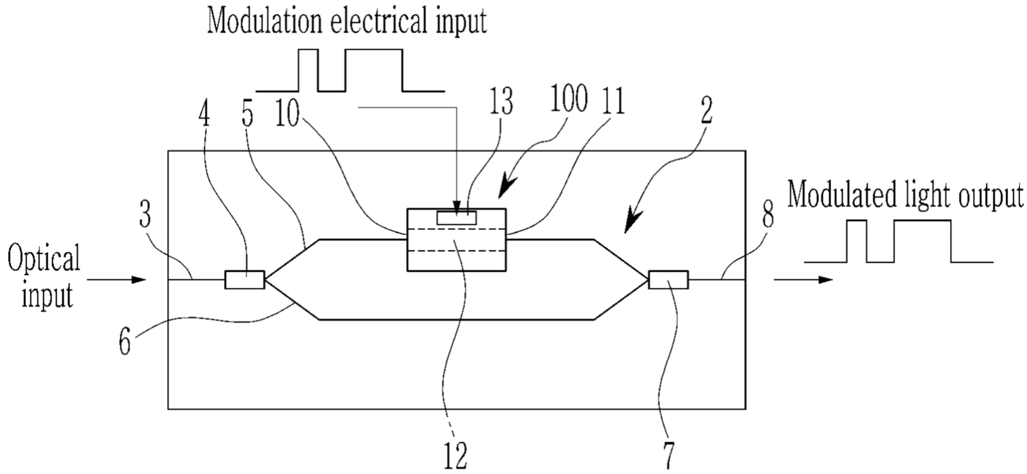
Modulation electrical input
4
5
10
13
100
11
2
3
Modulated light output
8
Optical
input
6
12
7

FIG. 2

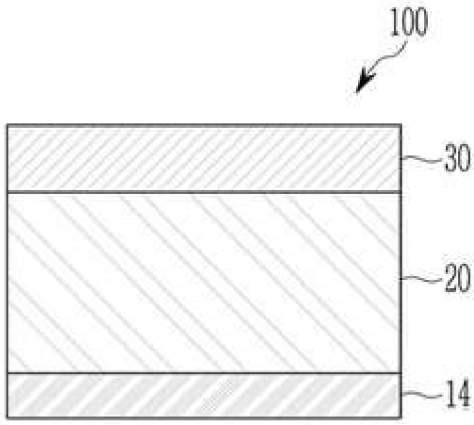

FIG. 3

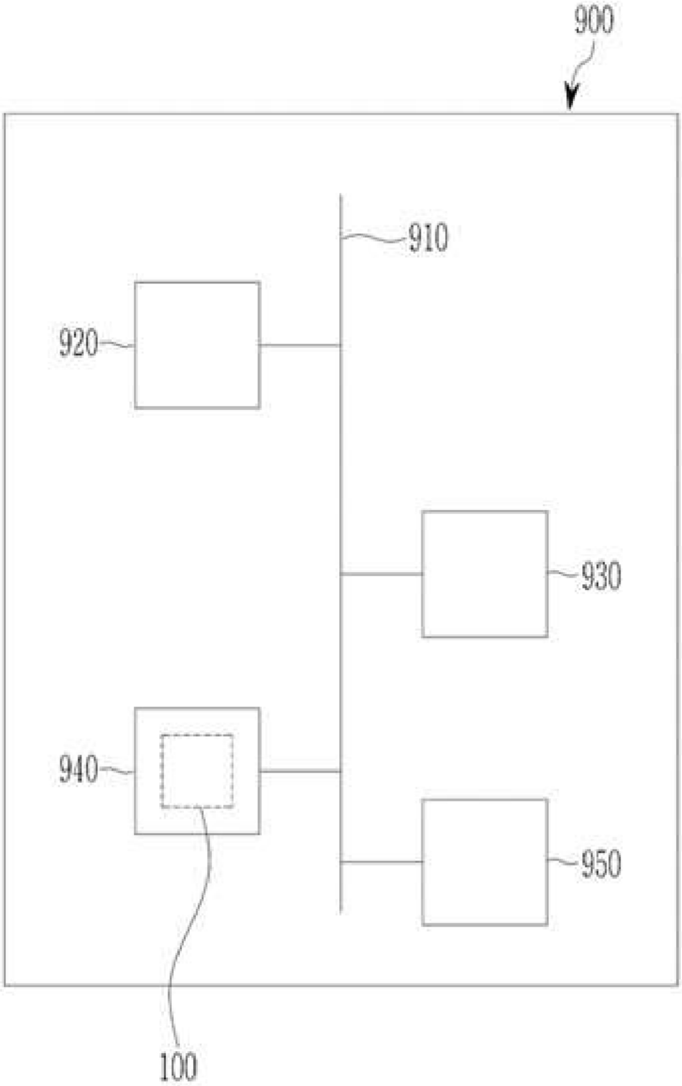

Europäisches Patentamt
European Patent Office
Office européen des brevets

# EUROPEAN SEARCH REPORT

Application Number
EP 25 22 3031

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/155868 A1 (JUNG HYEJIN [KR] ET AL) 9 May 2024 (2024-05-09)<br>* claims 1, 13, 14; compounds 4, 29, 30 *<br>----- | 1-15 | INV.<br>C07D471/06<br>C07D495/16<br>C07D517/16<br>H10K50/00 |
| A | WO 2023/247338 A1 (MERCK PATENT GMBH [DE]) 28 December 2023 (2023-12-28)<br>* page 66 - page 98; claims 1, 14; compounds 1-282 *<br>----- | 1-15 | |
| A | EP 4 458 829 A1 (SAMSUNG ELECTRONICS CO LTD [KR]) 6 November 2024 (2024-11-06)<br>* page 27, line 40; claims 1, 9 *<br>----- | 1-15 | |
| A | CAO WEI ET AL: "New D-[pi]-A organic dyes containing a tert-butyl-capped indolo[3,2,1-jk]carbazole donor with bithiophene unit as [pi]-linker for dye-sensitized solar cells",<br>RSC ADVANCES,<br>vol. 5, no. 42,<br>1 January 2015 (2015-01-01), pages 32967-32975, XP093388568,<br>GB<br>ISSN: 2046-2069, DOI: 10.1039/c5ra02720a<br>* page 32968; figure 1; compounds CL10, CL11 *<br>-----<br>-/-- | 1-15 | TECHNICAL FIELDS SEARCHED (IPC)<br>C07D<br>H10K |

The present search report has been drawn up for all claims

| Place of search | Date of completion of the search | Examiner |
|---|---|---|
| Munich | 17 April 2026 | Moriggi, J |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 25 22 3031

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DO KWANGSOEK ET AL: "Efficient planar organic semiconductors containing fused triphenylamine for solution processed small molecule organic solar cells", SOLAR ENERGY MATERIALS AND SOLAR CELLS ELSEVIER SCIENCE PUBLISHERS, AMSTERDAM., NL, vol. 115, 20 April 2013 (2013-04-20), pages 52-57, XP028553530, ISSN: 0927-0248, DOI: 10.1016/J.SOLMAT.2013.03.020 * page 53; compound 1 * ----- | 1-15 | |
| A | US 2022/093875 A1 (SUZAKI YUJI [JP]) 24 March 2022 (2022-03-24) * at least compounds 13-32; claims 1, 18 * ----- | 1-15 | |
| X | US 2023/192719 A1 (MIYASHITA HIROKAZU [JP] ET AL) 22 June 2023 (2023-06-22) * at least compounds A4, A6-A16; pages 10, 11; claims 1 , 6 * ----- | 1-3,6-8, 11-15 | TECHNICAL FIELDS SEARCHED (IPC) |

The present search report has been drawn up for all claims

2

| Place of search | Date of completion of the search | Examiner |
|---|---|---|
| Munich | 17 April 2026 | Moriggi, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

Europäisches Patentamt
European Patent Office
Office européen des brevets

Application Number
EP 25 22 3031

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [X] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 25 22 3031

The Search Division considers that the present European patent application does not comply with the requirements of unity of invention and relates to several inventions or groups of inventions, namely:

1. claims: 1-15

The compounds according to Chemical Formula 1 of current claim 1 and the optical devices containing them

1.1. claims: 1-15(partially)

The compounds according to Chemical Formula 1 of current claim 1 wherein both x and z equal 0, and the optical devices containing them

1.2. claims: 1-15(partially)

The compounds according to Chemical Formula 1 of current claim 1 wherein at least one of x and z is 1, 2, 3, 4, or 5, and the optical devices containing them

---

Please note that all inventions mentioned under item 1, although not necessarily linked by a common inventive concept, could be searched without effort justifying an additional fee.

## ANNEX TO THE EUROPEAN SEARCH REPORT ON EUROPEAN PATENT APPLICATION NO.

EP 25 22 3031

This annex lists the patent family members relating to the patent documents cited in the above-mentioned European search report. The members are as contained in the European Patent Office EDP file on
The European Patent Office is in no way liable for these particulars which are merely given for the purpose of information.

17-04-2026

| Patent document cited in search report | Publication date | Patent family member(s) | Publication date |
|---|---|---|---|
| US 2024155868 A1 | 09-05-2024 | CN 117800988 A | 02-04-2024 |
| | | KR 20240046839 A | 11-04-2024 |
| | | US 2024155868 A1 | 09-05-2024 |
| WO 2023247338 A1 | 28-12-2023 | CN 119325755 A | 17-01-2025 |
| | | EP 4541156 A1 | 23-04-2025 |
| | | KR 20250025717 A | 24-02-2025 |
| | | WO 2023247338 A1 | 28-12-2023 |
| EP 4458829 A1 | 06-11-2024 | CN 118852198 A | 29-10-2024 |
| | | EP 4458829 A1 | 06-11-2024 |
| | | KR 20240159308 A | 05-11-2024 |
| | | US 2024381765 A1 | 14-11-2024 |
| US 2022093875 A1 | 24-03-2022 | CN 114203936 A | 18-03-2022 |
| | | KR 20220038200 A | 28-03-2022 |
| | | US 2022093875 A1 | 24-03-2022 |
| US 2023192719 A1 | 22-06-2023 | JP 7802517 B2 | 20-01-2026 |
| | | JP 2023090310 A | 29-06-2023 |
| | | US 2023192719 A1 | 22-06-2023 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *CHEMICAL ABSTRACTS*, 436097-14-2
- *CHEMICAL ABSTRACTS*, 23259-98-5
- *CHEMICAL ABSTRACTS*, 60466-52-6
- *CHEMICAL ABSTRACTS*, 1639046-22-2
- *CHEMICAL ABSTRACTS*, 1004784-50-2
- *CHEMICAL ABSTRACTS*, 193978-23-3